# EUROPEAN PATENT SPECIFICATION

(11) **EP 3 715 352 B1**
(45) Date of publication and mention of the grant of the patent: **30.07.2025**
(21) Application number: 20165175.9
(22) Date of filing: 24.03.2020
(51) Int. Cl.: C07F 15/00, C09K 11/06, H10K 85/30

(54) **ORGANOMETALLIC COMPOUND, ORGANIC LIGHT-EMITTING DEVICE INCLUDING THE SAME, AND ELECTRONIC APPARATUS INCLUDING THE ORGANIC LIGHT-EMITTING DEVICE**
ORGANOMETALLISCHE VERBINDUNG, ORGANISCHE LICHTEMITTIERENDE VORRICHTUNG DAMIT UND ELEKTRONISCHE VORRICHTUNG MIT DER ORGANISCHEN LICHTEMITTIERENDEN VORRICHTUNG
COMPOSÉ ORGANOMÉTALLIQUE, DISPOSITIF ÉLECTROLUMINESCENT ORGANIQUE LE COMPRENANT ET APPAREIL ÉLECTRONIQUE COMPRENANT LE DISPOSITIF ÉLECTROLUMINESCENT ORGANIQUE

(30) Priority: 29.03.2019 KR 20190037213
(43) Date of publication of application: 30.09.2020
(73) Proprietor: Samsung Electronics Co., Ltd., Suwon-si, Gyeonggi-do 16677 (KR)
(72) Inventor: LEE, Jiyoun, Suwon-si, Gyeonggi-do 16678 (KR); KIM, Soyeon, Suwon-si, Gyeonggi-do 16678 (KR); CHO, Yongsuk, Suwon-si, Gyeonggi-do 16678 (KR); CHOI, Jongwon, Suwon-si, Gyeonggi-do 16678 (KR); KRAVCHUK, Dmitry, Suwon-si, Gyeonggi-do 16678 (KR); LEE, Banglin, Suwon-si, Gyeonggi-do 16678 (KR); KOO, Hyun, Suwon-si, Gyeonggi-do 16678 (KR); LEE, Sunghun, Suwon-si, Gyeonggi-do 16678 (KR); CHO, Yuri, Suwon-si, Gyeonggi-do 16678 (KR)
(74) Representative: Elkington and Fife LLP

(56) References cited:
- EP-A1- 1 418 217
- EP-A1- 2 182 002
- WO-A1-2010/031738
- WO-A1-2019/221487
- US-A1- 2007 015 005
- US-A1- 2010 289 406
- Y J CHO ET AL: "Novel compounds for electronic material and organic electronic device using the same", EP2182002 A1, 1 January 2010 (2010-01-01), pages 1 - 4, XP055694565

## Description

### FIELD OF THE INVENTION

One or more embodiments relate to an organometallic compound, an organic light-emitting device including the organometallic compound, and an electronic apparatus including the organic light-emitting device.

### BACKGROUND OF THE INVENTION

Organic light-emitting devices are self-emission devices, which have better characteristics in terms of a viewing angle, response time, brightness, driving voltage, and response speed, and produce full-color images.

In an example, an organic light-emitting device includes an anode, a cathode, and an organic layer between the anode and the cathode, wherein the organic layer includes an emission layer. A hole transport region may be between the anode and the emission layer, and an electron transport region may be between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons transit from an excited state to a ground state, thereby generating light.

EP 2 182 002 A1 discloses compounds for electronic materials, and organic electronic devices and organic solar cells comprising the same.

WO 2010/031738 A1 discloses electroluminescent metal complexes with azapyrenes, a process for their preparation, electronic devices comprising the metal complexes and their use in electronic devices.

US 2007/0015005 A1 discloses an organic electroluminescent device including an anode on a substrate, an organic luminescent layer on the anode, and a cathode on the organic luminescent layer.

EP 1 418 217 A1 discloses an organic luminescent device, wherein the light emitting layer includes an organic platinum group element compound composed of a phenanthridine derivative and a platinum group element.

US 2010/0289406 A1 discloses compounds containing a 2-azatriphenylene core having an additional aromatic group, and their use in organic light-emitting devices.

### SUMMARY OF THE INVENTION

Aspects of the present disclosure provide an organometallic compound, an organic light-emitting device including the organometallic compound, and an electronic apparatus including the organic light-emitting device.

Additional aspects will be set forth in part in the description which follows and, in part, will be apparent from the description, or may be learned by practice of the presented embodiments.

An aspect of the present disclosure provides an organometallic compound in accordance with claim 1.

Another embodiment of the present disclosure provides an organic light-emitting device in accordance with claim 11.

Another embodiment of the present disclosure provides an electronic apparatus including the above-described organic light-emitting device.

In one or more embodiments, the organometallic compound may be in the emission layer of the organic layer, and the organometallic compound in the emission layer may serve as a dopant.

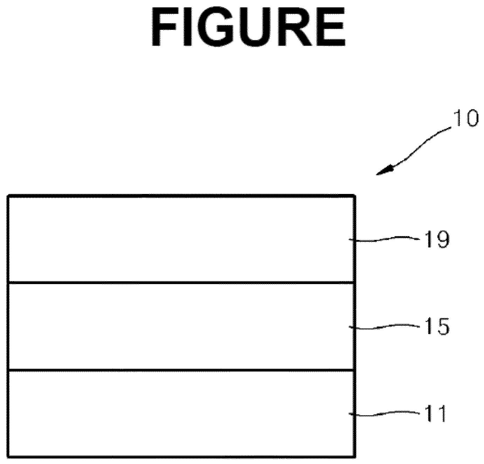

### BRIEF DESCRIPTION OF THE DRAWING

These and/or other aspects will become apparent and more readily appreciated from the following description of the embodiments, taken in conjunction with the accompanying drawing in which:
FIGURE is a schematic cross-sectional view of an organic light-emitting device according to an embodiment.

### DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

It will be understood that when an element is referred to as being "on" another element, it can be directly on the other element or intervening elements may be present therebetween In contrast, when an element is referred to as being "directly on" another element, there are no intervening elements present

It will be understood that, although the terms "first," "second," "third" etc. may be used herein to describe various elements, components, regions, layers and/or sections, these elements, components, regions, layers and/or sections should not be limited by these terms These terms are only used to distinguish one element, component, region, layer or section from another element, component, region, layer or section Thus, "a first element," "component," "region," "layer" or "section" discussed below could be termed a second element, component, region, layer or section without departing from the teachings herein.

The terminology used herein is for the purpose of describing particular embodiments only and is not intended to be limiting. As used herein, "a," "an," "the," and "at least one" do not denote a limitation of quantity, and are intended to cover both the singular and plural, unless the context clearly indicates otherwise. For example, "an element" has the same meaning as "at least one element," unless the context clearly indicates otherwise.

"Or" means "and/or." As used herein, the term "and/or" includes any and all combinations of one or more of the associated listed items It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top," may be used herein to describe one element's relationship to another element as illustrated in the Figures It will be understood that relative terms are intended to encompass different orientations of the device in addition to the orientation depicted in the Figures For example, if the device in one of the figures is turned over, elements described as being on the "lower" side of other elements would then be oriented on "upper" sides of the other elements The exemplary term "lower," can therefore, encompasses both an orientation of "lower" and "upper," depending on the particular orientation of the figure Similarly, if the device in one of the figures is turned over, elements described as "below" or "beneath" other elements would then be oriented "above" the other elements The exemplary terms "below" or "beneath" can, therefore, encompass both an orientation of above and below.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10% or 5% of the stated value.

Unless otherwise defined, all terms (including technical and scientific terms) used herein have the same meaning as commonly understood by one of ordinary skill in the art to which this disclosure belongs It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments As such, variations from the shapes of the illustrations as a result, for example, of manufacturing techniques and/or tolerances, are to be expected Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features Moreover, sharp angles that are illustrated may be rounded Thus, the regions illustrated in the figures are schematic in nature and their shapes are not intended to illustrate the precise shape of a region and are not intended to limit the scope of the present claims.

An aspect of the present disclosure provides an organometallic compound represented by Formula 1:

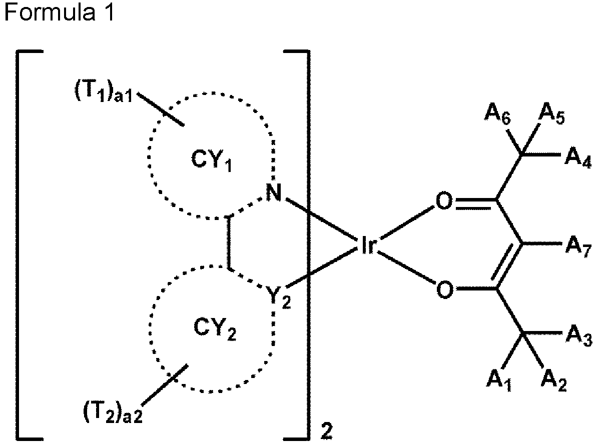

In Formula 1, Y₂ is C.
In Formula 1, ring CY₁ is a C₅-C₃₀ polycyclic group in which three or more (for example, three, four, five, or six) monocyclic groups are condensed to one another, wherein the group represented by 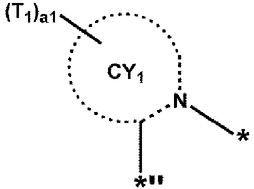 in Formula 1 is one of the groups represented by Formulae CY1-1, and CY1-7 to CY1-9: 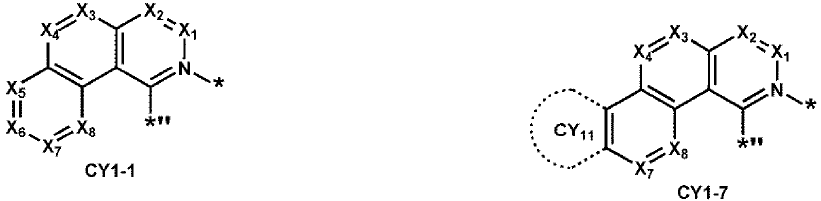 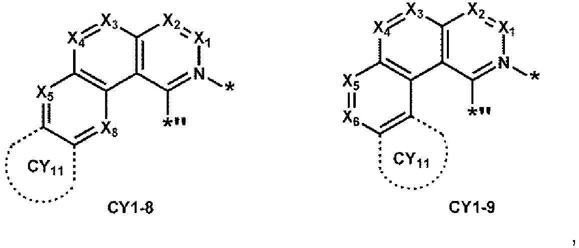 * is a binding site to iridium (Ir) in Formula 1, and *" is a binding site to the ring CY₂ in Formula 1,
X₁ to X₈ are each independently C or N, wherein the case where all of X₁ to X₈ are N is excluded,
ring CY₁₁ is a cyclopentane group, a cyclopentene group, a cyclohexane group, a cyclohexene group, an adamantane group, a norbornane group, a norbornene group, a benzene group, a pyridine group, a pyrimidine group, a pyrazine group, a pyridazine group, a naphthalene group, a quinoline group, an isoquinoline group, or a quinoxaline group.

For example, the monocyclic group may include: a benzene group; and at least one of a pyridine group, a pyrimidine group, a pyrazine group, and a pyridazine group.

In one or more embodiments, the monocyclic group may include: a benzene group; and at least one of a pyridine group, a pyrimidine group, a pyrazine group, and a pyridazine group, and optionally, further include a cyclopentane group, a cyclopentene group, a cyclohexane group, a cyclohexene group, an adamantane group, a norbornane group, a norbornene group, or any combination thereof.

In one or more embodiments, in Formula 1, ring CY₁ may include one or two nitrogens.

In Formula 1, ring CY₂ is a C₅-C₃₀ carbocyclic group or a C₁-C₃₀ heterocyclic group.

For example, in Formula 1, ring CY₂ may be a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazol group, a benzopyrazole group, a benzimidazole group, a benzoxazole group, a benzothiazole group, a benzoxadiazole group, a benzothiadiazol group, a 5,6,7,8-tetrahydroisoquinoline group, a 5,6,7,8-tetrahydroquinoline group, a phenoxazine group, a phenothiazine group, a dihydrophenazine group, a dihydroacridine group, an azaphenoxazine group, an azaphenothiazine group, an azadihydrophenazine group, or an azadihydroacridine group.

In one or more embodiments, in Formula 1, ring CY₂ may be a benzene group, a naphthalene group, a 1,2,3,4-tetrahydronaphthalene group, a thiophene group, a furan group, a pyrrole group, a cyclopentadiene group, a silole group, a benzothiophene group, a benzofuran group, an indole group, an indene group, a benzosilole group, a dibenzothiophene group, a dibenzofuran group, a carbazole group, a fluorene group, or a dibenzosilole group.

In one or more embodiments, in Formula 1, ring CY₂ may be a benzene group, a dibenzothiophene group, a dibenzofuran group, a carbazole group, a fluorene group, or a dibenzosilole group.

In Formula 1, T₁ and A₁ to A₇ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or - P(Q₈)(Q₉), and

T₂ is deuterium, -Cl, -Br, -I, -SF₅, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or - P(Q₈)(Q₉. Here, Q₁ to Q₉ will be understood with reference to the descriptions thereof in the specification.

For example, in Formula 1, T₁ and A₁ to A₇ may each independently be:
hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, C₁-C₂₀ alkyl group, a C₂-C₂₀ alkenyl group, a C₁-C₂₀ alkoxy group, or C₁-C₂₀ alkylthio group;
a C₁-C₂₀ alkyl group, a C₂-C₂₀ alkenyl group, a C₁-C₂₀ alkoxy group, or a C₁-C₂₀ alkylthio group, each substituted with deuterium, -F, -Cl, -Br, -I, -CD₃, - CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cyclooctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a biphenyl group, a terphenyl group, a naphthyl group, a pyridinyl group, a pyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, or an azadibenzothiophenyl group, each unsubstituted or substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a deuterated C₂-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cyclooctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, or any combination thereof; or
-N(Q₁)(Q₂), -Si(Q₃)(Q₄)(Q₅), -Ge(Q₃)(Q₄)(Q₅), -B(Q₆)(Q₇), - P(=O)(Q₈)(Q₉), or -P(Q₈)(Q₉); and
Q₁ to Q₉ may each independently be:
   deuterium, -F, -CH₃, -CD₃, -CD₂H, -CDH₂, -CH₂CH₃, -CH₂CD₃, - CH₂CD₂H, -CH₂CDH₂, -CHDCH₃, -CHDCD₂H, -CHDCDH₂, -CHDCD₃, - CD₂CD₃, -CD₂CD₂H, -CD₂CDH₂, -CF₃, -CF₂H, -CFH₂, -CH₂CF₃, -CH₂CF₂H, - CH₂CFH₂, -CHFCH₃, -CHFCF₂H, -CHFCFH₂, -CHFCF₃, -CF₂CF₃, -CF₂CF₂H, or -CF₂CFH₂; or
   an n-propyl group, an iso-propyl group, an n-butyl group, a sec-butyl group, an isobutyl group, a tert-butyl group, an n-pentyl group, a tert-pentyl group, a neopentyl group, an isopentyl group, a sec-pentyl group, 3-pentyl group, a sec-isopentyl group, a phenyl group, a biphenyl group, or a naphthyl group, each unsubstituted or substituted with deuterium, -F, C₁-C₁₀ alkyl group, a phenyl group, or any combination thereof.

In one or more embodiments, in Formula 1, T₁ and A₁ to A₇ may each independently be hydrogen, deuterium, -F, -CH₃, -CD₃, -CD₂H, -CDH₂, -CF₃, -CF₂H, - CFH₂, a C₂-C₁₀ alkenyl group, a C₁-C₁₀ alkoxy group, a C₁-C₁₀ alkylthio group, one of groups represented by Formulae 9-1 to 9-39, one of groups represented by Formulae 9-1 to 9-39 in which at least one hydrogen is substituted with deuterium, one of groups represented by Formulae 9-1 to 9-39 in which at least one hydrogen is substituted with -F, one of groups represented by Formulae 9-201 to 9-237, one of groups represented by Formulae 9-201 to 9-237 in which at least one hydrogen is substituted with deuterium, one of groups represented by Formulae 9-201 to 9-237 in which at least hydrogen is substituted with -F, one of groups represented by Formulae 10-1 to 10-129, one of groups represented by Formulae 10-1 to 10-129 in which at least one hydrogen is substituted with deuterium, one of groups represented by Formulae 10-1 to 10-129 in which at least one hydrogen is substituted with -F, one of groups represented by Formulae 10-201 to 10-350, one of groups represented by Formulae 10-201 to 10-350 in which at least one hydrogen is substituted with deuterium, one of groups represented by Formulae 10-201 to 10-350 in which at least one hydrogen is substituted with -F, -Si(Q₃)(Q₄)(Q₅), or -Ge(Q₃)(Q₄)(Q₅) (wherein Q₃ to Q₅ will be understood with reference to the descriptions thereof in the specification).

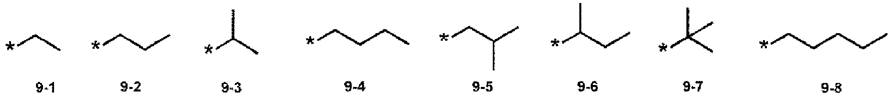

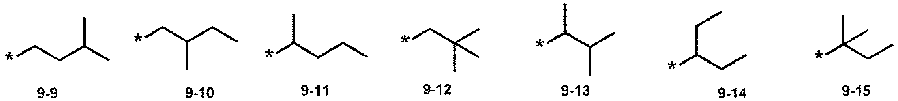

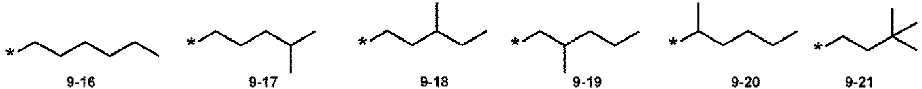

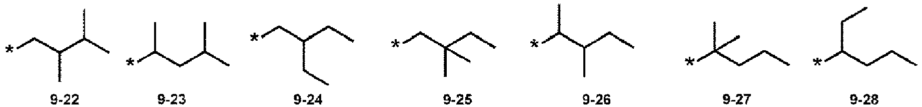

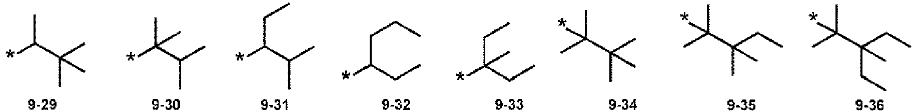

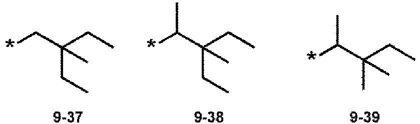

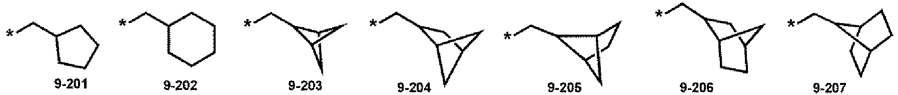

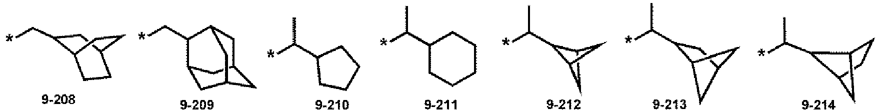

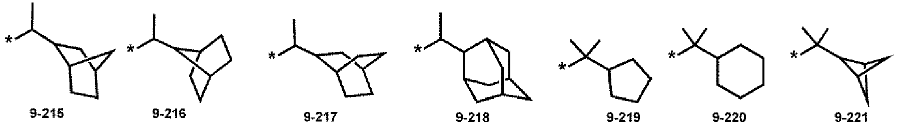

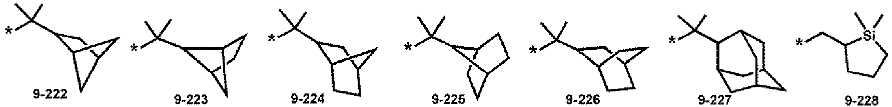

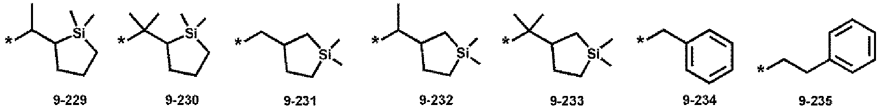

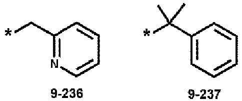

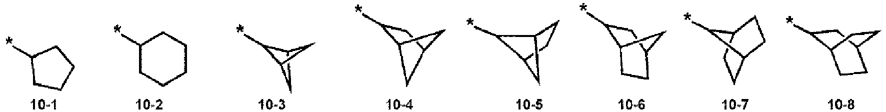

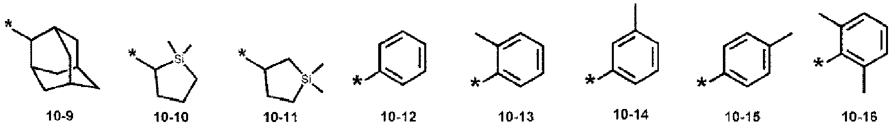

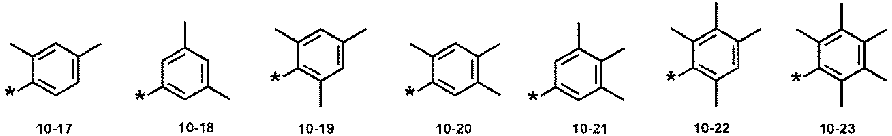

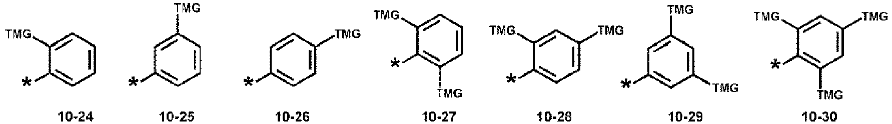

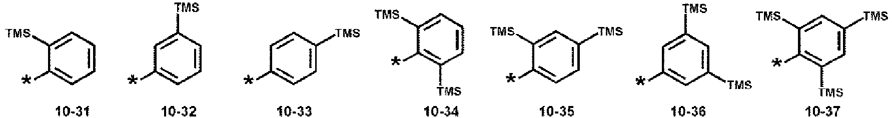

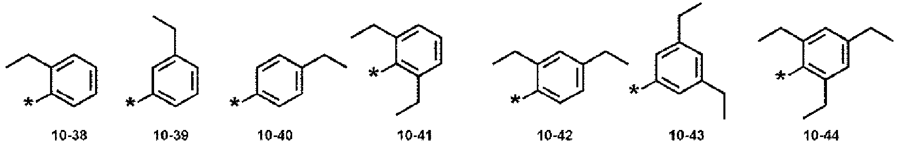

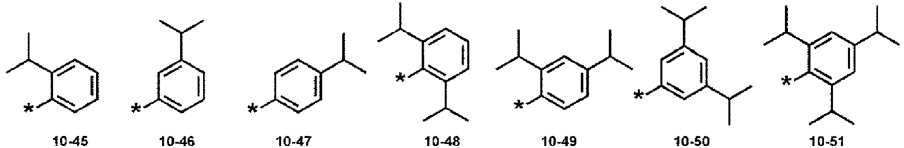

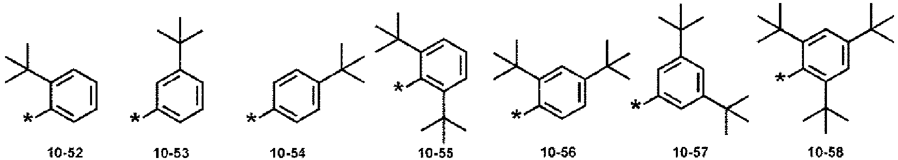

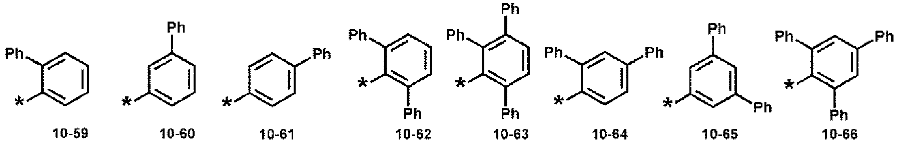

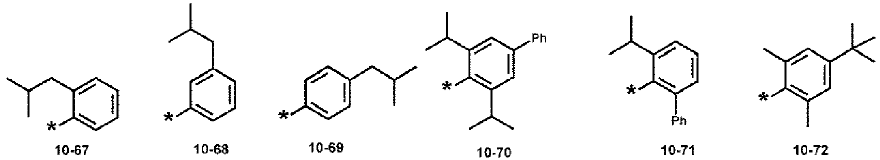

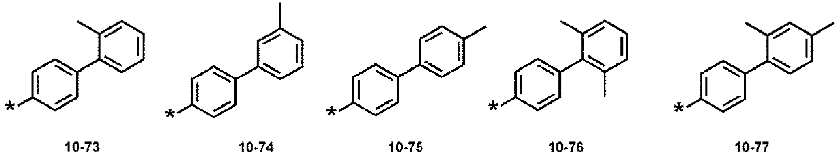

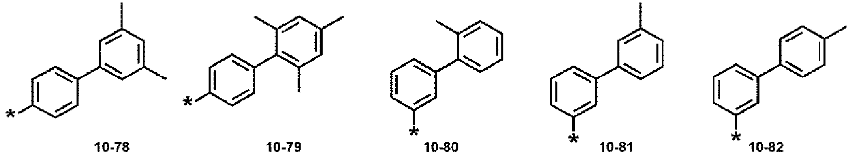

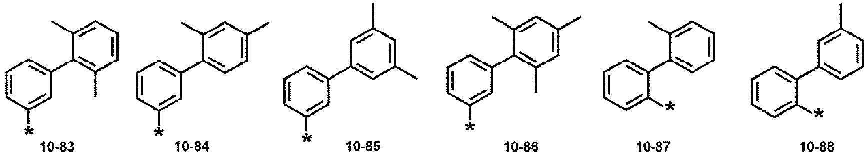

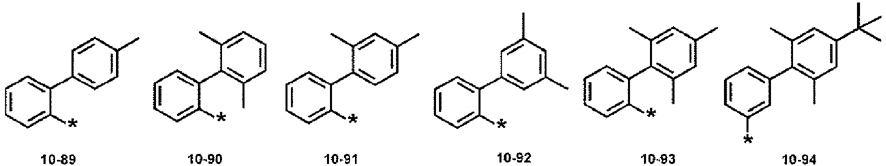

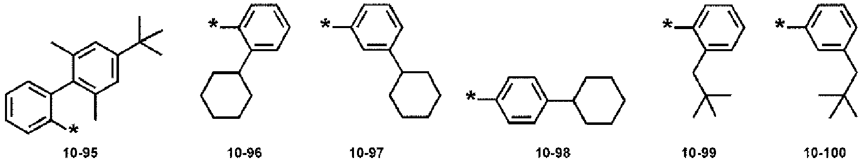

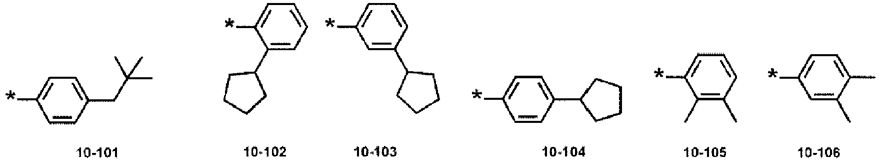

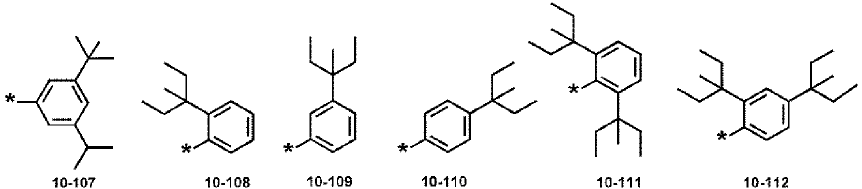

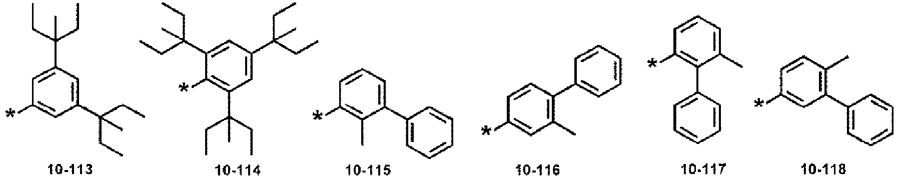

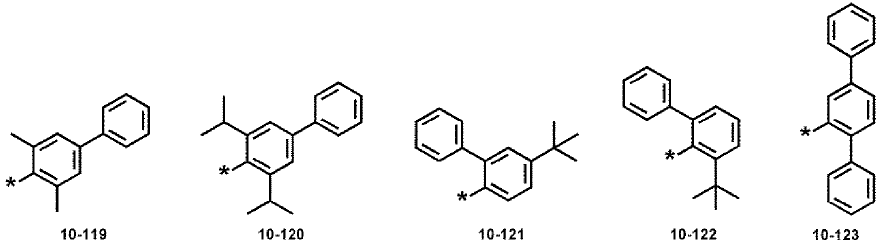

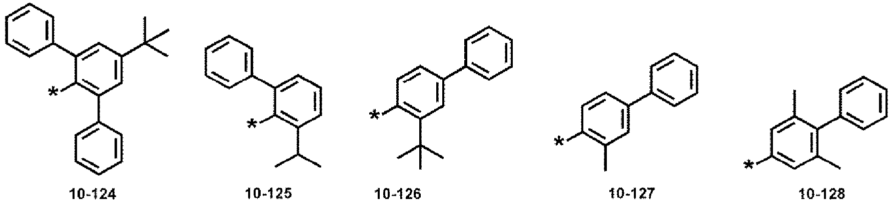

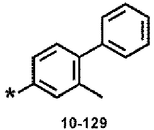

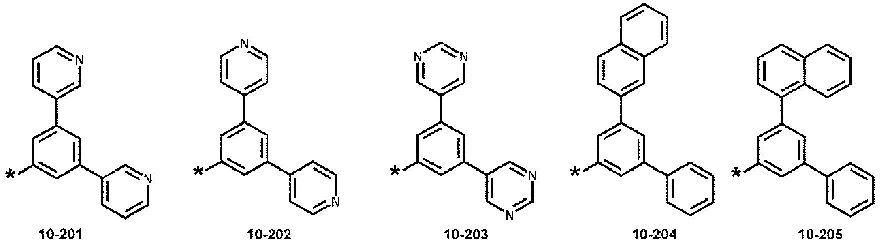

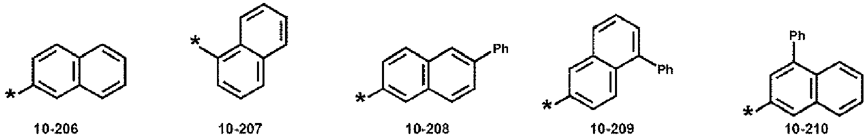

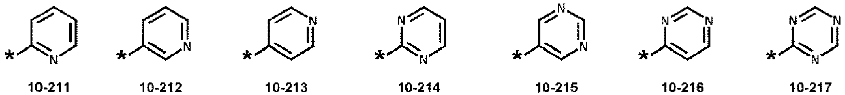

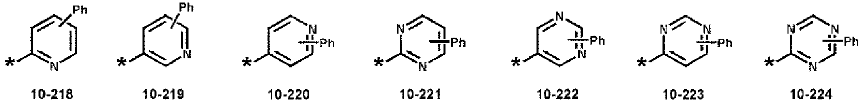

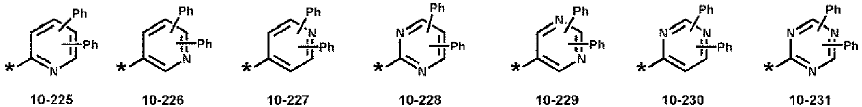

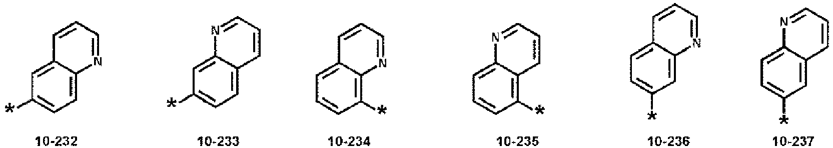

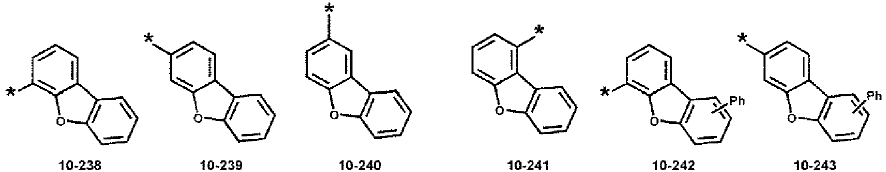

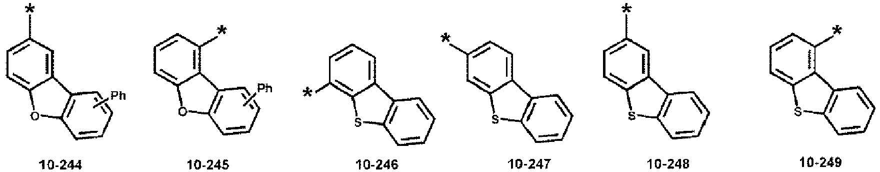

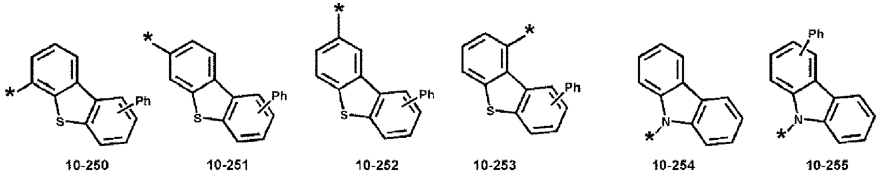

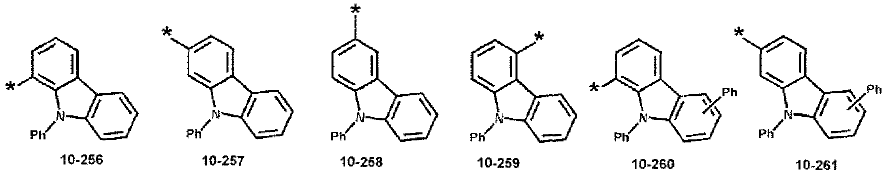

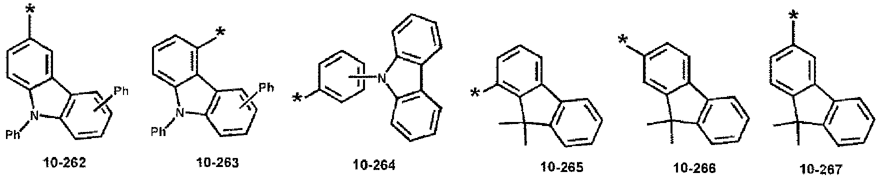

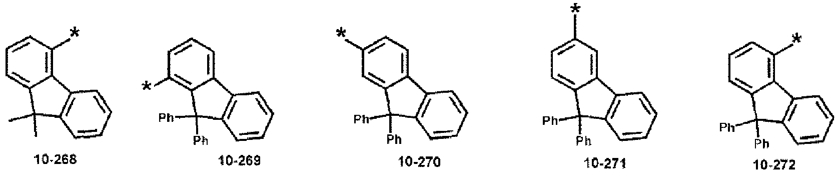

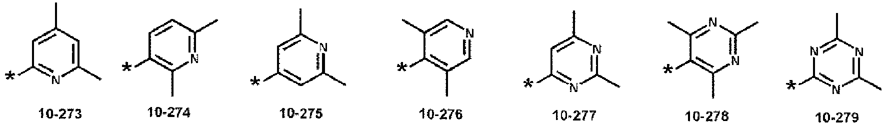

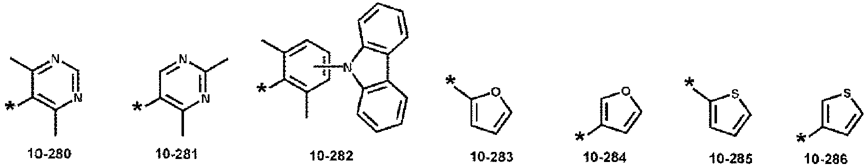

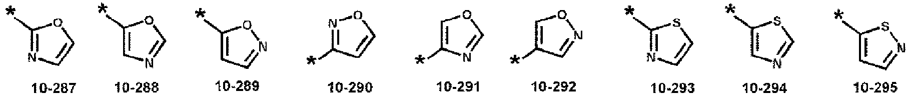

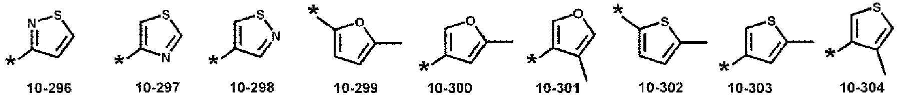

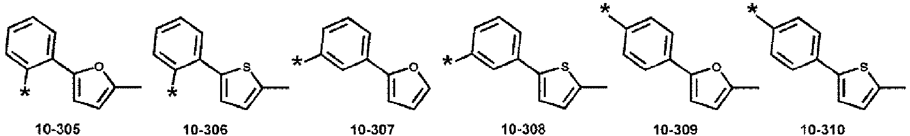

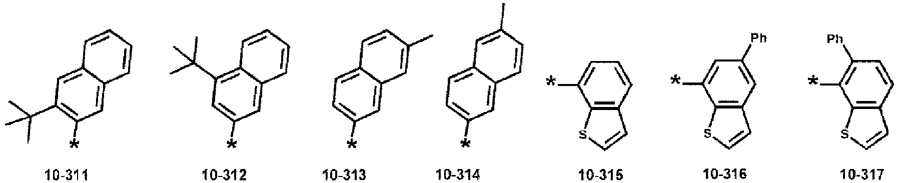

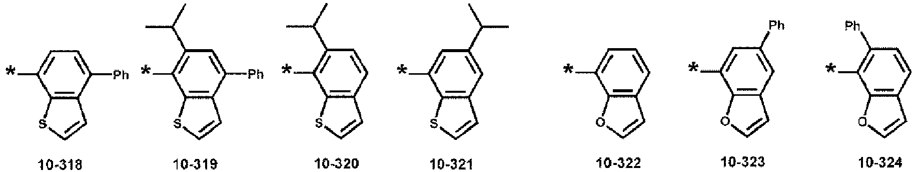

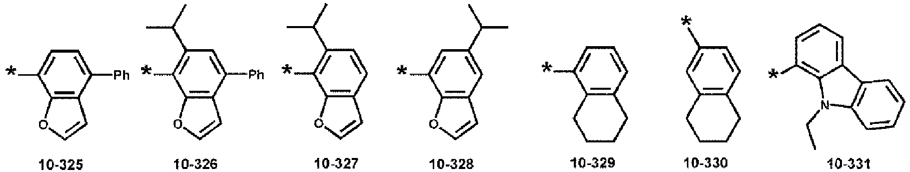

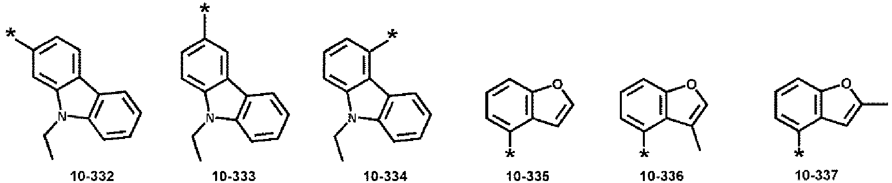

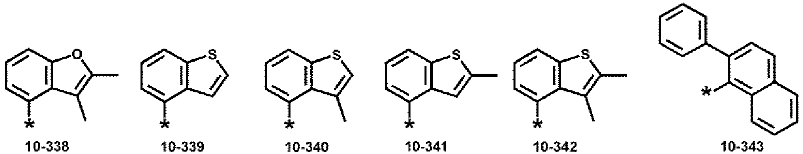

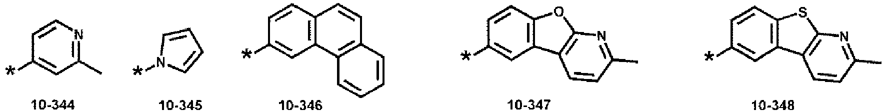

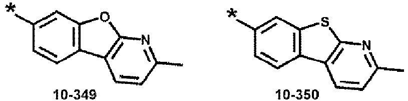

In Formulae 9-1 to 9-39, 9-201 to 9-237, 10-1 to 10-129 and 10-201 to 10-350, * indicates a binding site to an adjacent atom, Ph indicates a phenyl group, TMS indicates a trimethylsilyl group, and TMG indicates a trimethylgermyl group.

The groups represented by Formulae 9-1 to 9-39 in which at least one hydrogen is substituted with deuterium, and the groups represented by Formulae 9-201 to 9-237 in which at least one hydrogen is substituted with deuterium may be, for example, groups represented by Formulae 9-501 to 9-514 and 9-601 to 9-636.

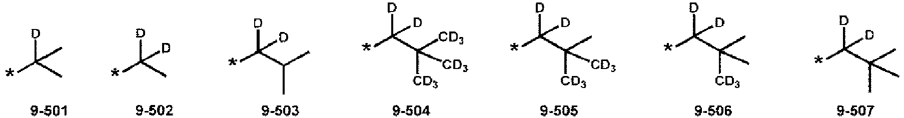

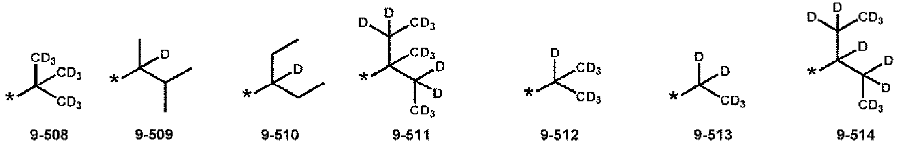

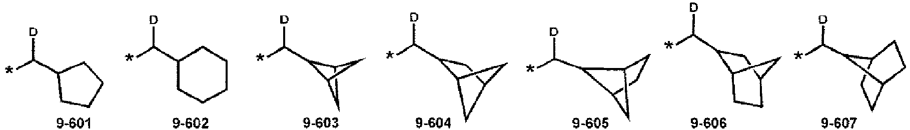

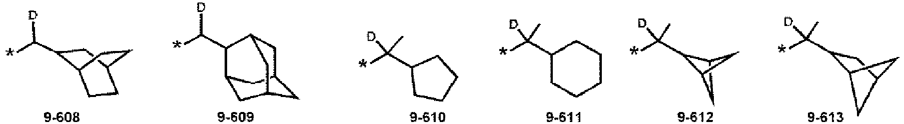

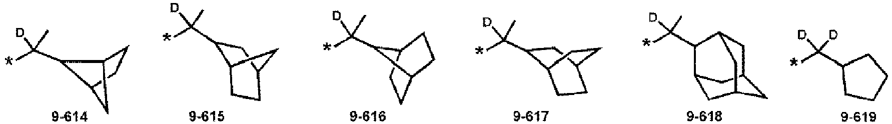

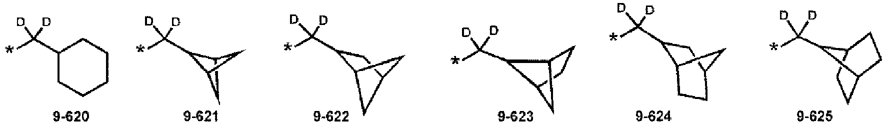

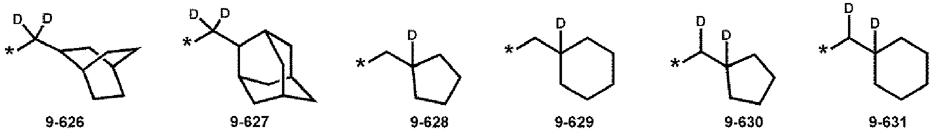

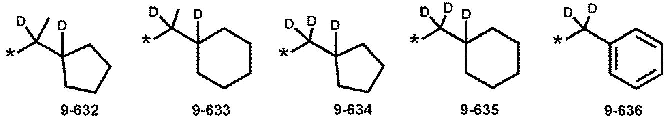

The groups represented by Formulae 9-1 to 9-39 in which at least one hydrogen is substituted with -F, and the group represented by Formulae 9-201 to 9-236 in which at least one hydrogen is substituted with -F may be, for example, groups represented by Formulae 9-701 to 9-710.

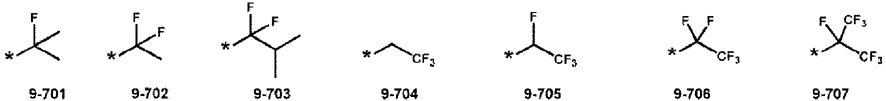

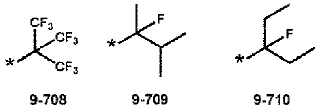

The group represented by Formulae 10-1 to 10-129 in which at least one hydrogen is substituted with deuterium, and the groups represented by Formulae 10-201 to 10-350 in which at least one hydrogen is substituted with deuterium may be, for examples, groups represented by Formulae 10-501 to 10-553.

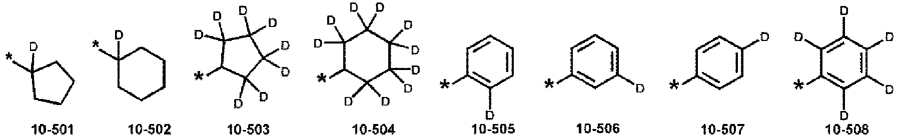

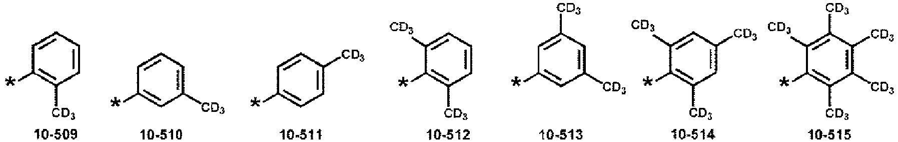

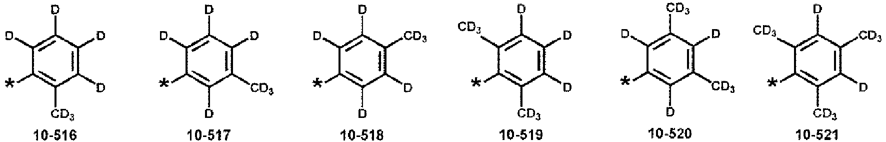

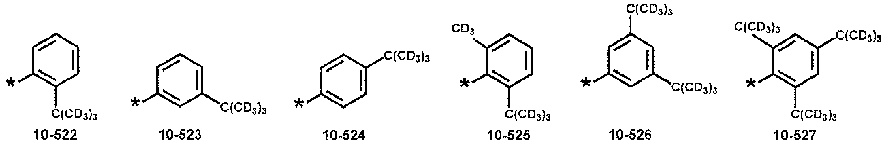

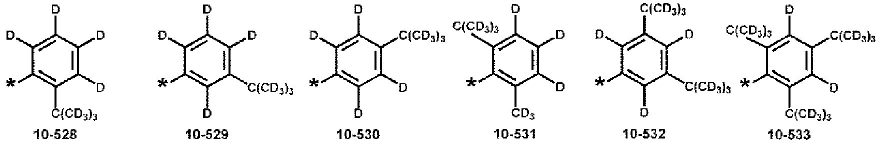

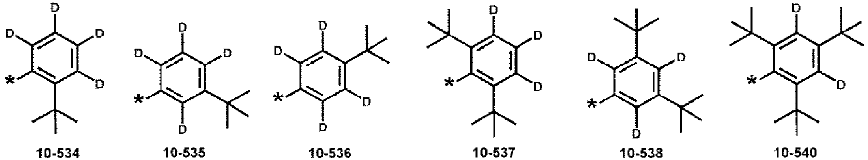

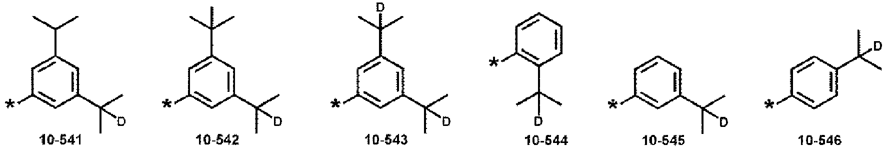

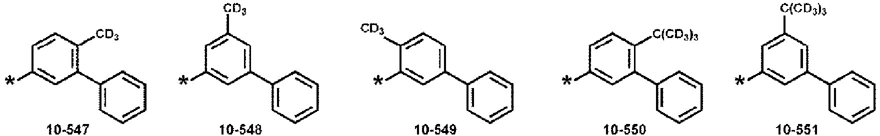

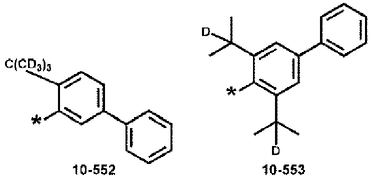

The groups represented by Formulae 10-1 to 10-129 in which at least one hydrogen is substituted with -F, and the groups represented by Formula 10-201 to 10-350 in which at least one hydrogen is substituted with -F may be, for examples, groups represented by Formulae 10-601 to 10-617.

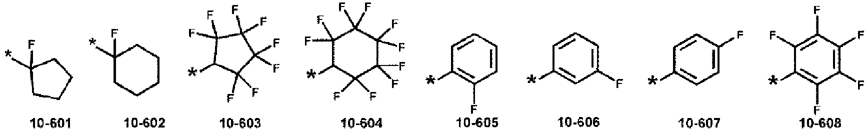

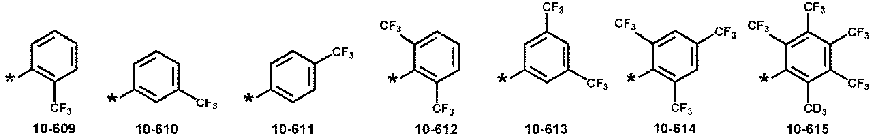

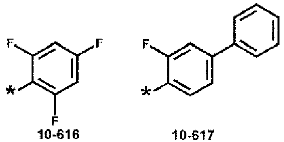

In Formula 1, a1 and a2 indicate the number of T₁(s) and the number of T₂(s), respectively. When a1 is 2 or greater, two or more T₁(s) may be identical to or different from each other. When a2 is two or greater, two or more T₂(s) may be identical to or different from each other. In Formula 1, the sum of a1 and a2 is 2 or greater. In Formula 1, a1 is an integer of 1 to 20, for example, an integer of 1 to 8. In Formula 1, a2 is 1, 2, or 3.

In Formula 1, at least one of T₁(s) in number of a1 includes at least one fluoro group (-F).

In one or more embodiments, in Formula 1, T₁ and A₁ to A₇ may each independently be hydrogen, deuterium, -F, a substituted or unsubstituted C₁-C₂₀ alkyl group, a substituted or unsubstituted C₃-C₁₀ cycloalkyl group, a substituted or unsubstituted C₁-C₁₀ heterocycloalkyl group, a substituted or unsubstituted a phenyl group, a substituted or unsubstituted a biphenyl group, a substituted or unsubstituted a terphenyl group, -Si(Q₃)(Q₄)(Q₅), or -Ge(Q₃)(Q₄)(Q₅).

In one or more embodiments, in Formula 1, T₁ and A₁ to A₇ may each independently be:
hydrogen, deuterium, -F, C₁-C₂₀ alkyl group, a C₃-C₁₀ cycloalkyl group, a C₁-C₁₀ heterocycloalkyl group, a phenyl group, a biphenyl group, or a terphenyl group;
a fluorinated C₁-C₂₀ alkyl group, a fluorinated C₃-C₁₀ cycloalkyl group, a fluorinated C₁-C₁₀ heterocycloalkyl group, a fluorinated phenyl group, a fluorinated biphenyl group, or a fluorinated terphenyl group, each unsubstituted or substituted with deuterium, C₁-C₂₀ alkyl group, a C₃-C₁₀ cycloalkyl group, a C₁-C₁₀ heterocycloalkyl group, a phenyl group, a biphenyl group, a terphenyl group, or any combination thereof;
a deuterated C₁-C₂₀ alkyl group, a deuterated C₃-C₁₀ cycloalkyl group, a deuterated C₁-C₁₀ heterocycloalkyl group, a deuterated phenyl group, a deuterated biphenyl group, or a deuterated terphenyl group, each ussubstituted or unsubstituted with -F, C₁-C₂₀ alkyl group, a C₃-C₁₀ cycloalkyl group, a C₁-C₁₀ heterocycloalkyl group, a phenyl group, a biphenyl group, a terphenyl group, or any combination thereof; or
-Si(Q₃)(Q₄)(Q₅), or -Ge(Q₃)(Q₄)(Q₅).

In one or more embodiments, in Formula 1, a1 is an integer of 1 to 20, and at least one of T₁(s) in number of a1 may each independently be:
-F; or
a fluorinated C₁-C₂₀ alkyl group, a fluorinated C₃-C₁₀ cycloalkyl group, a fluorinated C₁-C₁₀ heterocycloalkyl group, a fluorinated phenyl group, a fluorinated biphenyl group, or a fluorinated terphenyl group, each unsubstituted or substituted with deuterium, C₁-C₂₀ alkyl group, a C₃-C₁₀ cycloalkyl group, a C₁-C₁₀ heterocycloalkyl group, a phenyl group, a biphenyl group, a terphenyl group, or any combination thereof.

**In** one or more embodiments, in Formula 1, A₁ to A₇ may each independently be:
hydrogen, deuterium, C₁-C₂₀ alkyl group, a C₃-C₁₀ cycloalkyl group, a C₁-C₁₀ heterocycloalkyl group, a phenyl group, a biphenyl group, or a terphenyl group;
a deuterated C₁-C₂₀ alkyl group, a deuterated C₃-C₁₀ cycloalkyl group, a deuterated C₁-C₁₀ heterocycloalkyl group, a deuterated phenyl group, a deuterated biphenyl group, or a deuterated terphenyl group, each unsubstituted or substituted with C₁-C₂₀ alkyl group, a C₃-C₁₀ cycloalkyl group, a C₁-C₁₀ heterocycloalkyl group, a phenyl group, a biphenyl group, a terphenyl group, or any combination thereof; or
-Si(Q₃)(Q₄)(Q₅), **or** -Ge(Q₃)(Q₄)(Q₅).

**In** Formula 1, 1) two or more of T₁(s) in number of a1 are optionally linked to one another to form a C₅-C₃₀ carbocyclic group unsubstituted or substituted with at least one R₁ₐ, or a C₁-C₃₀ heterocyclic group unsubstituted or substituted with at least one R₁ₐ, 2) two or more of T₂(s) in number of a2 are optionally linked to one another to form a C₅-C₃₀ carbocyclic group unsubstituted or substituted with at least one R₁ₐ, or a C₁-C₃₀ heterocyclic group unsubstituted or substituted with at least one R₁ₐ, 3) T₁ and T₂ are optionally linked to one another to form a C₅-C₃₀ carbocyclic group unsubstituted or substituted with at least one R₁ₐ, or a C₁-C₃₀ heterocyclic group unsubstituted or substituted with at least one R₁ₐ, 4) two or more of A₁ to A₇ are optionally linked to one another to form a C₅-C₃₀ carbocyclic group unsubstituted or substituted with at least one R₁ₐ, or a C₁-C₃₀ heterocyclic group unsubstituted or substituted with at least one R₁ₐ. Here, R₁ₐ will be understood with reference to the description of A₇ provided above.

**In** one or more embodiments, in Formula 1, the group represented by 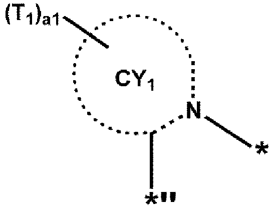 may be represented by Formula CY1(1).

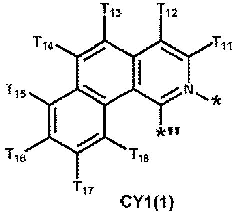

In Formula CY1(1),
T₁₁ to T₁₈ will be understood with reference to the description of T₁ herein, provided that at least one of T₁₁ to T₁₈ may include a fluoro group (-F),
* indicates a binding site to Ir in Formula 1, and
*" indicates a binding site to ring CY₂ in Formula 1.

For example, one, two, or three of T₁₁ to T₁₈ in Formula CY1(1) may include at least one -F, and
the remaining T₁₁ to T₁₈ in Formula CY1(1), not including -F, may each independently be:
hydrogen, deuterium, a C₁-C₂₀ alkyl group, a C₃-C₁₀ cycloalkyl group, a C₁-C₁₀ heterocycloalkyl group, a phenyl group, a biphenyl group, or a terphenyl group;
a deuterated C₁-C₂₀ alkyl group, a deuterated C₃-C₁₀ cycloalkyl group, a deuterated C₁-C₁₀ heterocycloalkyl group, a deuterated phenyl group, a deuterated biphenyl group, or a deuterated terphenyl group, each unsubstituted or substituted with a C₁-C₂₀ alkyl group, a C₃-C₁₀ cycloalkyl group, a C₁-C₁₀ heterocycloalkyl group, a phenyl group, a biphenyl group, a terphenyl group, or any combination thereof; or
-Si(Q₃)(Q₄)(Q₅), or -Ge(Q₃)(Q₄)(Q₅).

In one or more embodiments, one, two, or three of T₁₁ to T₁₈ in Formula CY1 (1) may each independently be:
-F; or
a fluorinated C₁-C₂₀ alkyl group, a fluorinated C₃-C₁₀ cycloalkyl group, a fluorinated C₁-C₁₀ heterocycloalkyl group, a fluorinated phenyl group, a fluorinated biphenyl group, or a fluorinated terphenyl group, each unsubstituted or substituted with deuterium, a C₁-C₂₀ alkyl group, a C₃-C₁₀ cycloalkyl group, a C₁-C₁₀ heterocycloalkyl group, a phenyl group, a biphenyl group, a terphenyl group, or any combination thereof.

In one or more embodiments, in Formula 1, the group represented by 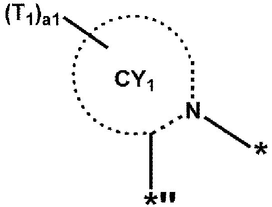 may be one of groups represented by Formulae CY1(1)-1 to CY1(1)-64.

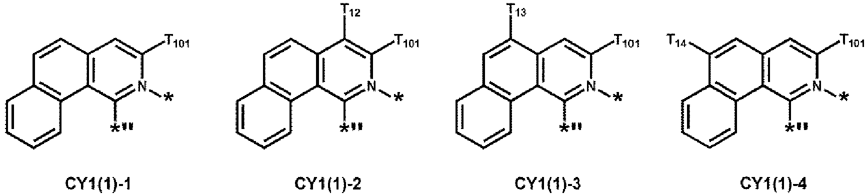

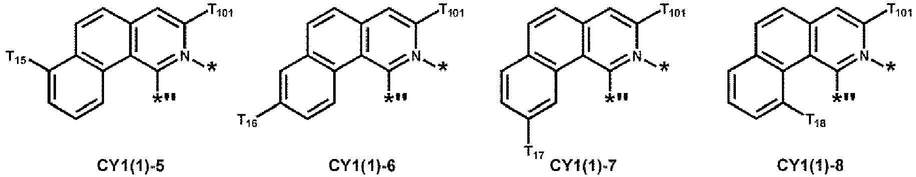

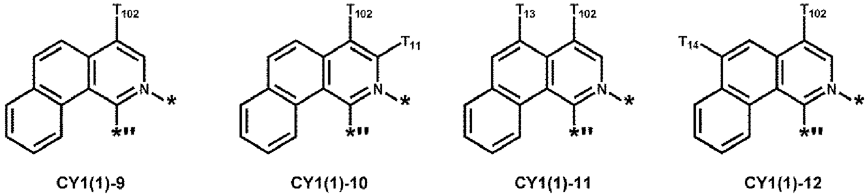

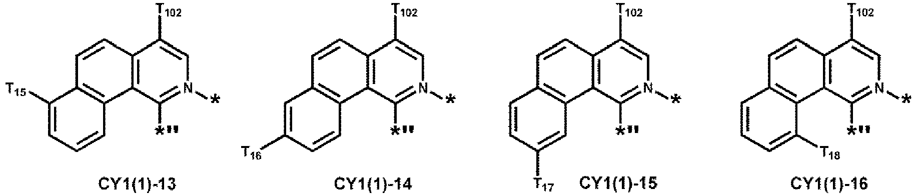

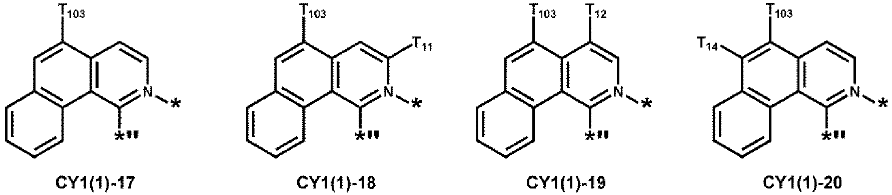

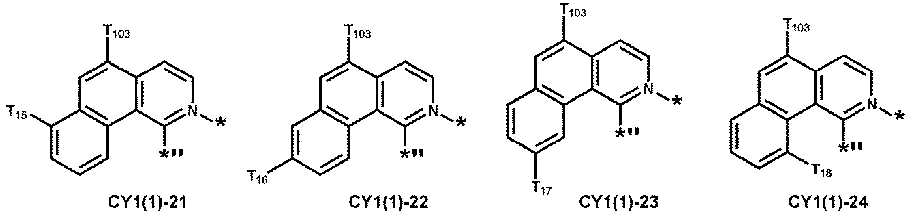

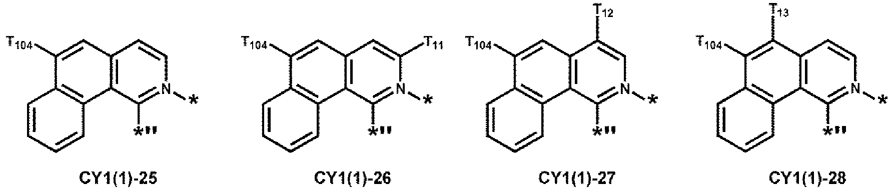

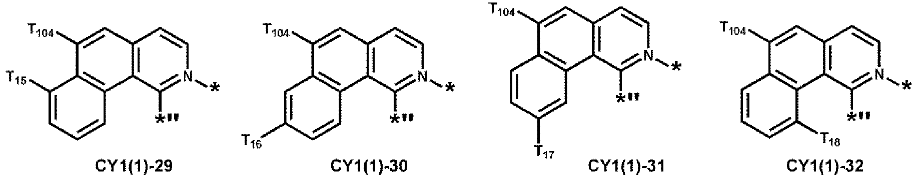

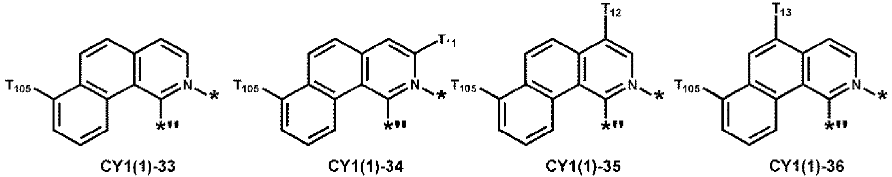

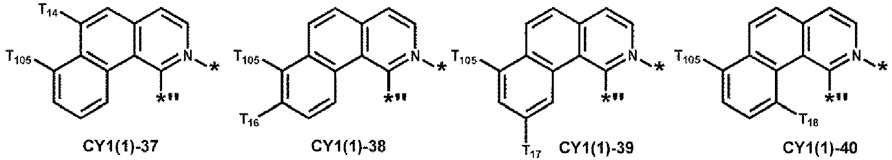

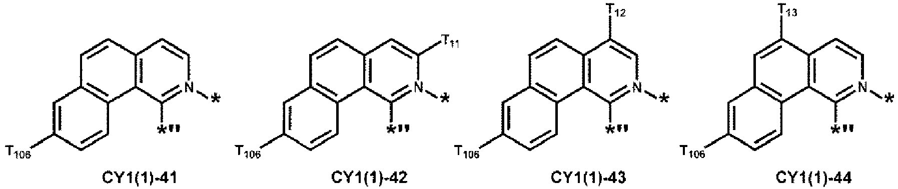

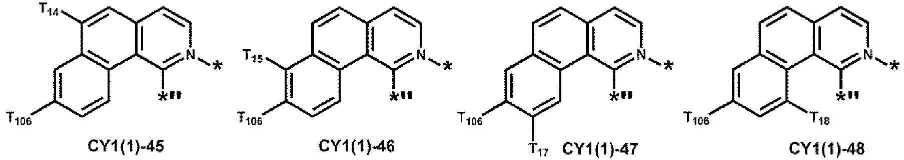

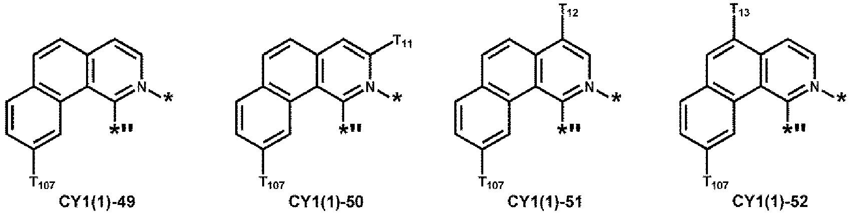

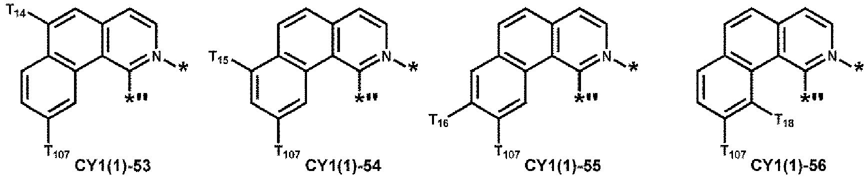

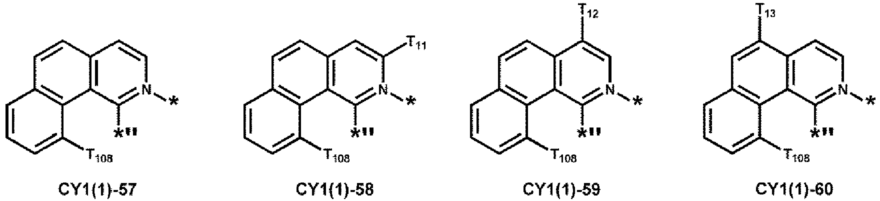

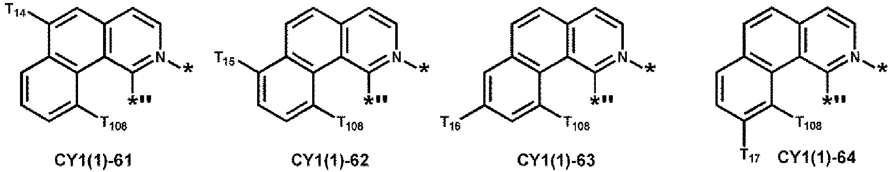

In Formulae CY1(1)-1 to CY1(1)-64,
T₁₁ to T₁₈ will be understood with reference to the description of T₁ herein, provided that each of T₁₁ to T₁₈ is not hydrogen,
T₁₀₁ to T₁₀₈ may each include at least one fluoro group (-F),
* indicates a binding site to Ir in Formula 1, and
*" indicates a binding site to ring CY₂ in Formula 1.

For example, in Formulae CY1(1)-1 to CY1(1)-64, T₁₁ to T₁₈ may each independently be:
deuterium, a C₁-C₂₀ alkyl group, a C₃-C₁₀ cycloalkyl group, a C₁-C₁₀ heterocycloalkyl group, a phenyl group, a biphenyl group, or a terphenyl group;
a deuterated C₁-C₂₀ alkyl group, a deuterated C₃-C₁₀ cycloalkyl group, a deuterated C₁-C₁₀ heterocycloalkyl group, a deuterated phenyl group, a deuterated biphenyl group, or a deuterated terphenyl group, each unsubstituted or substituted with a C₁-C₂₀ alkyl group, a C₃-C₁₀ cycloalkyl group, a C₁-C₁₀ heterocycloalkyl group, a phenyl group, a biphenyl group, a terphenyl group, or any combination thereof; or
-Si(Q₃)(Q₄)(Q₅), or -Ge(Q₃)(Q₄)(Q₅).

In one or more embodiments, in Formulae CY1(1)-1 to CY1(1)-64, T₁₀₁ to T₁₀₈ may each independently be:
-F; or
a fluorinated C₁-C₂₀ alkyl group, a fluorinated C₃-C₁₀ cycloalkyl group, a fluorinated C₁-C₁₀ heterocycloalkyl group, a fluorinated phenyl group, a fluorinated biphenyl group, or a fluorinated terphenyl group, each unsubstituted or substituted with deuterium, a C₁-C₂₀ alkyl group, a C₃-C₁₀ cycloalkyl group, a C₁-C₁₀ heterocycloalkyl group, a phenyl group, a biphenyl group, a terphenyl group, or any combination thereof.

In one or more embodiments, in Formula 1, the group represented by 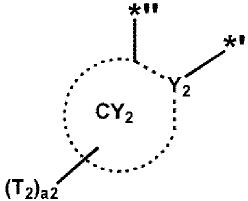 may be one of groups represented by Formulae CY2-1 to CY2-31.

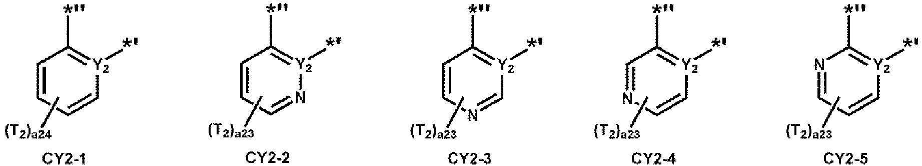

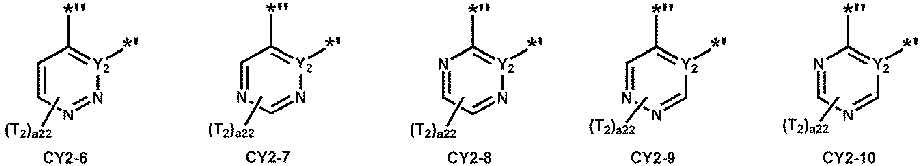

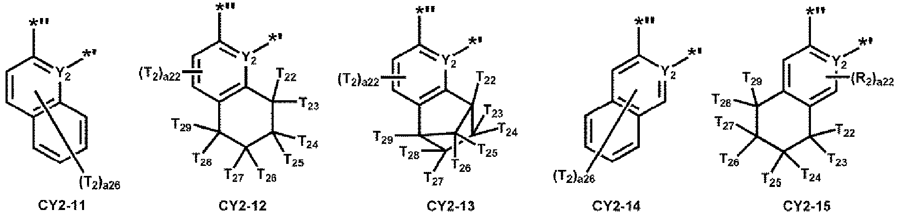

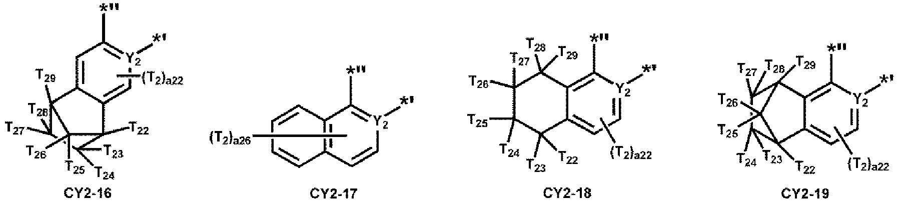

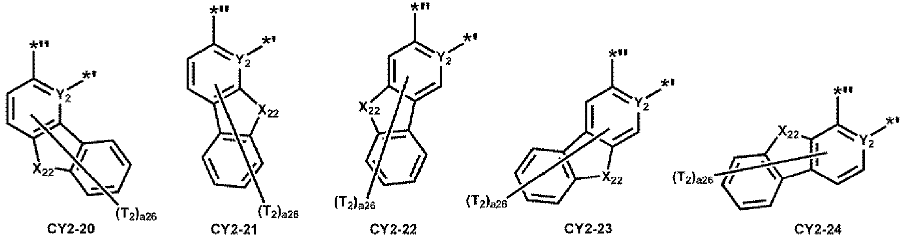

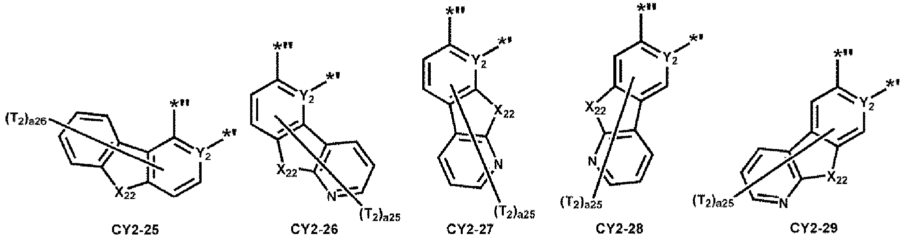

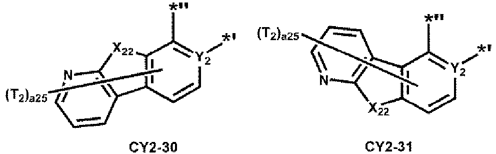

In Formulae CY2-1 to CY2-31,
Y₂ and T₂ will be understood with reference to the descriptions thereof in the present specification,
X₂₂ may be C(T₂₈)(T₂₉), N(T₂₈), O, S, or Si(T₂₈)(T₂₉),
T₂₂ to T₂₉ will be understood with reference to the description of T₂ in the present specification,
a26 may be an integer of 1, 2, or 3,
a25 may be an integer of 1, 2, or 3,
a24 may be an integer of 1, 2, or 3,
a23 may be an integer of 1, 2, or 3,
a22 may be an integer of 1 or 2,
*" indicates a binding site to ring CY₁ in Formula 1, and
*' indicates a binding site to Ir in Formula 1.

In one or more embodiments, in Formula 1, the group represented by 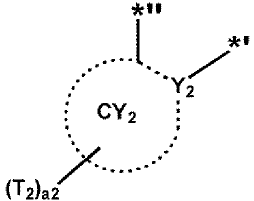 may be one of groups represented by Formulae CY2(2) to CY2(15), CY2(18) to CY2(24), CY2(26) to CY2(32), CY2(34) to CY3(40), CY3(42) to CY3(48), CY2(50) to CY2(52), CY2(55), CY2(56), CY2(64), CY2(65), CY2(67), and CY2(68) .

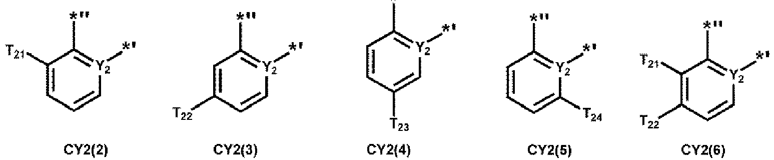

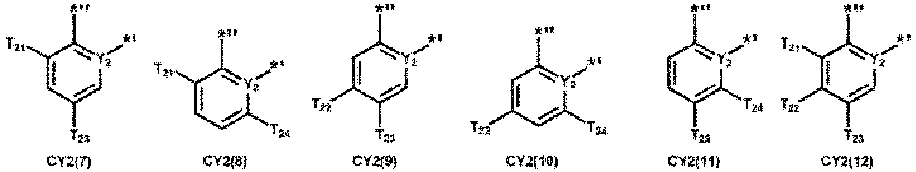

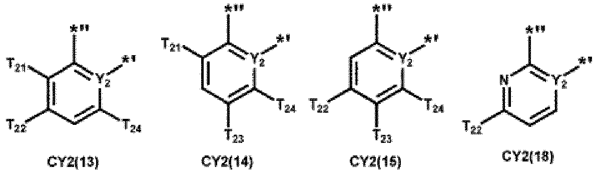

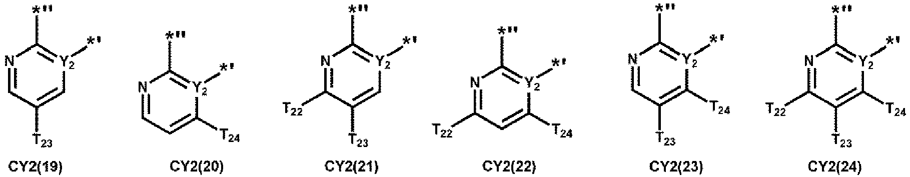

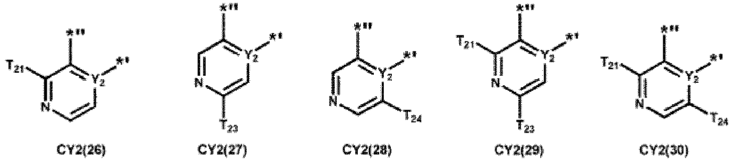

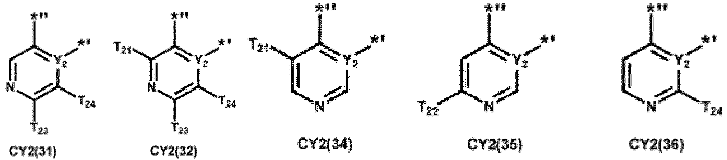

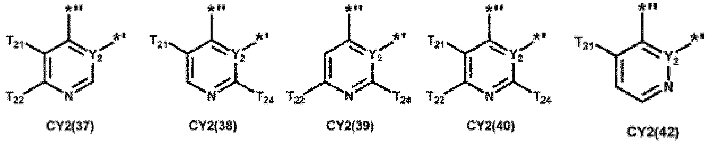

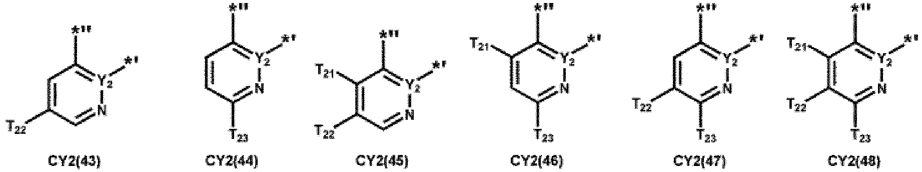

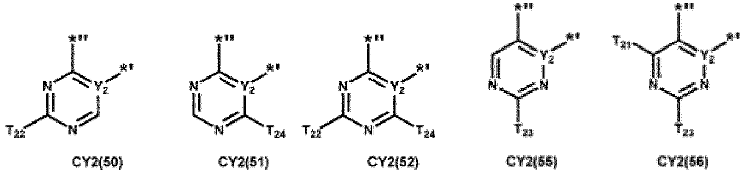

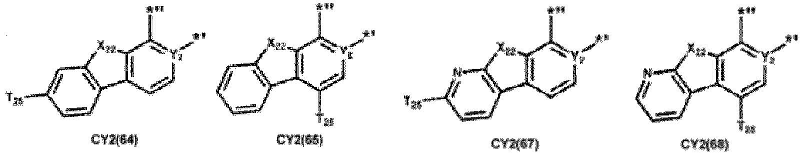

In Formulae CY2(2) to CY2(15), CY2(18) to CY2(24), CY2(26) to CY2(32), CY2(34) to CY3(40), CY3(42) to CY3(48), CY2(50) to CY2(52), CY2(55), CY2(56), CY2(64), CY2(65), CY2(67), and CY2(68),
Y₂ will be understood with reference to the description thereof provided in the present specification,
X₂₂ may be C(T₂₈)(T₂₉), N(T₂₈), O, S, or Si(T₂₈)(T₂₉),
T₂₁ to T₂₅, T₂₈, and T₂₉ will be understood with reference to the description of T₂ in the present specification,
*" indicates a binding site to ring CY₁ in Formula 1, and
*' indicates a binding site to Ir in Formula 1.

In one or more embodiments, in Formula 1, the group represented by 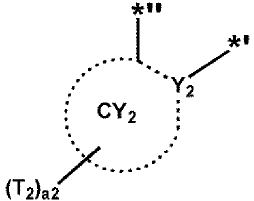 may be a group represented by Formula CY2(10).

For example, in Formula CY2(10), T₂₂ and T₂₄ may each independently be a C₁-C₂₀ alkyl group, a C₃-C₁₀ cycloalkyl group, a phenyl group, a biphenyl group, or a terphenyl group, each unsubstituted or substituted with deuterium, a C₁-C₂₀ alkyl group, a C₃-C₁₀ cycloalkyl group, a phenyl group, a biphenyl group, a terphenyl group, or any combination thereof.

In one or more embodiments, in Formula CY2(10), T₂₂ and T₂₄ may be identical to each other.

In one or more embodiments, in Formula CY2(10), T₂₂ and T₂₄ may be different from each other.

In one or more embodiments, in Formula CY2(10), the number of carbons in T₂₂ may be greater than the number of carbons in T₂₄.

In one or more embodiments, in Formula 1,
at least one of T₁(s) in number of a1 may be:
a fluoro group (-F); or
a fluorinated C₁-C₂₀ alkyl group, a fluorinated C₃-C₁₀ cycloalkyl group, a fluorinated C₁-C₁₀ heterocycloalkyl group, a fluorinated phenyl group, a fluorinated biphenyl group, or a fluorinated terphenyl group, each unsubstituted or substituted with deuterium, C₁-C₂₀ alkyl group, a C₃-C₁₀ cycloalkyl group, a C₁-C₁₀ heterocycloalkyl group, a phenyl group, a biphenyl group, a terphenyl group, or any combination thereof, and
the organometallic compound represented by Formula 1 may further satisfy at least one of Condition A to Condition G:
   Condition A
      All the remaining T₁ (s) are hydrogen;
   Condition B
      At least one of the remaining T₁(s) in Formula 1 is a C₁-C₂₀ alkyl group, a C₃-C₁₀ cycloalkyl group, a C₁-C₁₀ heterocycloalkyl group, a phenyl group, a biphenyl group, or a terphenyl group;
   Condition C
      At least one of the remaining T₁(s) in Formula 1 is:
      deuterium, or
      a deuterated C₁-C₂₀ alkyl group, a deuterated C₃-C₁₀ cycloalkyl group, a deuterated C₁-C₁₀ heterocycloalkyl group, a deuterated phenyl group, a deuterated biphenyl group, or a deuterated terphenyl group, each unsubstituted or substituted with -F, C₁-C₂₀ alkyl group, a C₃-C₁₀ cycloalkyl group, a C₁-C₁₀ heterocycloalkyl group, a phenyl group, a biphenyl group, a terphenyl group, or any combination thereof;
   Condition D
      At least one of the remaining T₁(s) in Formula 1 is -Si(Q₃)(Q₄)(Q₅);
   Condition E
      At least one of the remaining T₁(s) in Formula 1 is -Ge(Q₃)(Q₄)(Q₅);
   Condition F
      In Formula 1, at least one of T₂(s) in number of a2 is a C₁-C₂₀ alkyl group, a C₃-C₁₀ cycloalkyl group, a C₁-C₁₀ heterocycloalkyl group, a phenyl group, a biphenyl group, or a terphenyl group; and
   Condition G
      In Formula 1, at least one of T₂(s) in number of a2 is:
      deuterium, or
      a deuterated C₁-C₂₀ alkyl group, a deuterated C₃-C₁₀ cycloalkyl group, a deuterated C₁-C₁₀ heterocycloalkyl group, a deuterated phenyl group, a deuterated biphenyl group, or a deuterated terphenyl group, each unsubstituted or substituted with a C₁-C₂₀ alkyl group, a C₃-C₁₀ cycloalkyl group, a C₁-C₁₀ heterocycloalkyl group, a phenyl group, a biphenyl group, a terphenyl group, or any combination thereof.

**In** one or more embodiments, the organometallic compound represented by Formula 1 may satisfy at least one of Condition 1 to Condition 3:
Condition 1
   **In** Formula 1, A₁ to A₆ are each independently a substituted or unsubstituted C₁-C₆₀ alkyl group, a substituted or unsubstituted C₃-C₁₀ cycloalkyl group, a substituted or unsubstituted C₂-C₁₀ heterocycloalkyl group, a substituted or unsubstituted C₆-C₆₀ aryl group, a substituted or unsubstituted C₂-C₆₀ heteroaryl group, a substituted or unsubstituted monovalent non-aromatic condensed polycyclic group, or a substituted or unsubstituted monovalent non-aromatic condensed heteropolycyclic group;
Condition 2
   **In** Formula 1, at least one of A₁ to A₆ (for example, at least one of A₁ to A₃, and at least one of A₄ to A₆) are each independently a substituted or unsubstituted C₂-C₆₀ alkyl group, a substituted or unsubstituted C₃-C₁₀ cycloalkyl group, a substituted or unsubstituted C₂-C₁₀ heterocycloalkyl group, a substituted or unsubstituted C₆-C₆₀ aryl group, a substituted or unsubstituted C₂-C₆₀ heteroaryl group, a substituted or unsubstituted monovalent non-aromatic condensed polycyclic group, or a substituted or unsubstituted monovalent non-aromatic condensed heteropolycyclic group; and
Condition 3
   In Formula 1, A₇ is deuterium, a substituted or unsubstituted C₁-C₆₀ alkyl group, a substituted or unsubstituted C₃-C₁₀ cycloalkyl group, a substituted or unsubstituted C₂-C₁₀ heterocycloalkyl group, a substituted or unsubstituted C₆-C₆₀ aryl group, a substituted or unsubstituted C₂-C₆₀ heteroaryl group, a substituted or unsubstituted monovalent non-aromatic condensed polycyclic group, or a substituted or unsubstituted monovalent non-aromatic condensed heteropolycyclic group.

In one or more embodiments, the organometallic compound represented by Formula 1 may satisfy at least one of Condition 1 and Condition 2 described above.

In one or more embodiments, in Formula 1, a2 may be 2, and T₂ may be a substituted or unsubstituted C₁-C₆₀ alkyl group.

In one or more embodiments, the organometallic compound represented by Formula 1 may satisfy at least one of Condition 4 and Condition 5:
Condition 4
   In Formula 1, two or more of A₁ to A₃ of the group represented by *-C(A₁)(A₂)(A₃) are linked with C in the group represented by *-C(A₁)(A₂)(A₃) to form a C₅-C₃₀ carbocyclic group unsubstituted or substituted with at least one R₁ₐ, or a C₁-C₃₀ heterocyclic group unsubstituted or substituted with at least one R₁ₐ so that the group represented by *-C(A₁)(A₂)(A₃) is a C₅-C₃₀ carbocyclic group unsubstituted or substituted with at least one R₁ₐ, or a C₁-C₃₀ heterocyclic group unsubstituted or substituted with at least one R₁ₐ; and
Condition 5

In Formula 1, two or more of A₄ to A₆ of the group represented by *-C(A₄)(A₅)(A₆) are linked with C in the group represented by *-C(A₄)(A₅)(A₆) to form a C₅-C₃₀ carbocyclic group unsubstituted or substituted with at least one R₁ₐ, or a C₁-C₃₀ heterocyclic group unsubstituted or substituted with at least one R₁ₐ so that the group represented by *-C(A₄)(A₅)(A₆) is a C₅-C₃₀ carbocyclic group unsubstituted or substituted with at least one R₁ₐ, or a C₁-C₃₀ heterocyclic group unsubstituted or substituted with at least one R₁ₐ.

In one or more embodiments, in Formula 1, the group represented by *-C(A₁)(A₂)(A₃) and the group represented by *-C(A₄)(A₅)(A₆) may be identical to each other.

In one or more embodiments, in Formula 1, the group represented by *-C(A₁)(A₂)(A₃) and the group represented by *-C(A₄)(A₅)(A₆) may be different from each other.

In one or more embodiments, in Formula 1, the number of carbons in the group represented by *-C(A₁)(A₂)(A₃) may be 4 or greater, 5 or greater, or 6 or greater.

In one or more embodiments, in Formula 1, the number of carbons in the group represented by *-C(A₄)(A₅)(A₆) may be 4 or greater, 5 or greater, or 6 or greater.

In Formula 1, the following case is excluded: 1) A₇ is hydrogen, and 2) both the group represented by *-C(A₁)(A₂)(A₃) and the group represented by *-C(A₄)(A₅)(A₆) are a methyl group.

In one or more embodiments, in Formula 1, the following case is excluded: 1) A₇ is hydrogen; and 2) all of A₁ to A₆ are a methyl group.

In one or more embodiments, in Formula 1, at least one deuterium may be included.

In one or more embodiments, in Formula 1, and at least one of T₂(s) in number of a2 may include at least one deuterium.

In one or more embodiments, the organometallic compound represented by Formula 1 may emit red light or green light, for example, red light or green light having a maximum emission wavelength of about 500 nm or greater, for example, in the range of about 500 nm or greater and about 650 nm or less.

The organometallic compound represented by Formula 1 may be one of Compounds 1 to 3, 10 to 12, 14, 15, 17 to 24, 26, 27, 29 to 33, 35, 36, 38, 39, 41, 42, 44, 45, 47 to 51, 53, 54, 56, 57, 59, 60, 62, 63, 65, 66, 68, 69, 71, 72, 74, 75, 77, 78, 80, 81, 83, 84, 86, 87, 89, and 90 below.

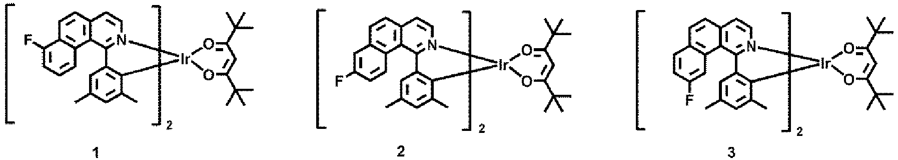

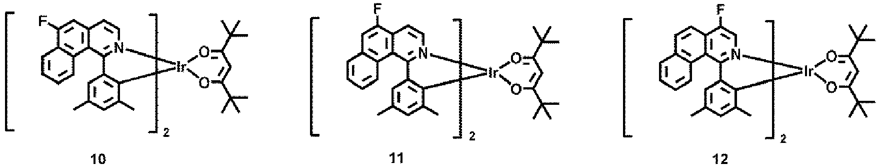

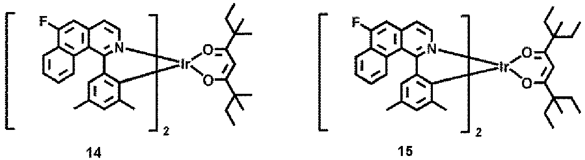

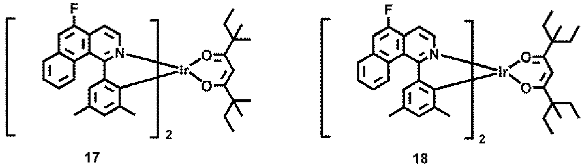

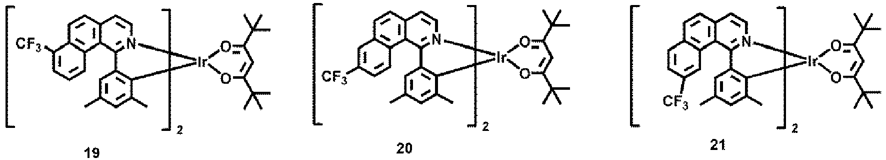

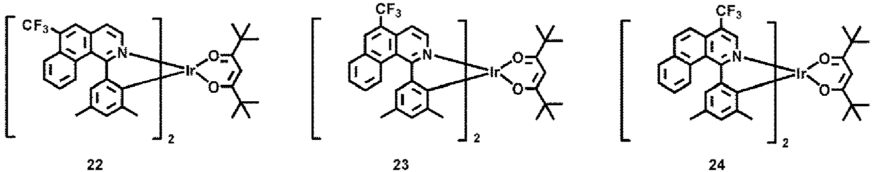

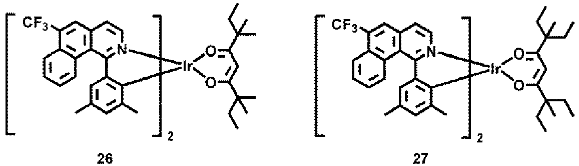

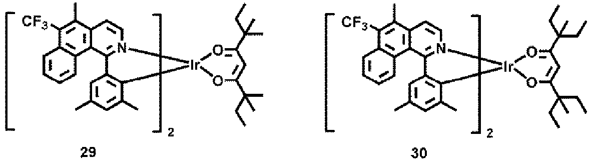

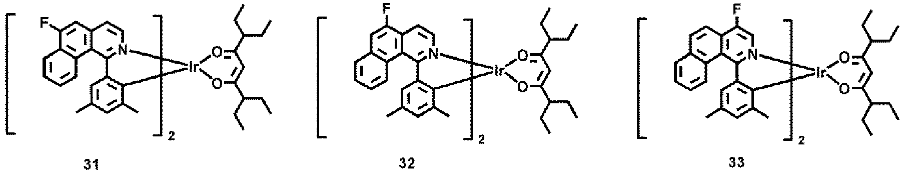

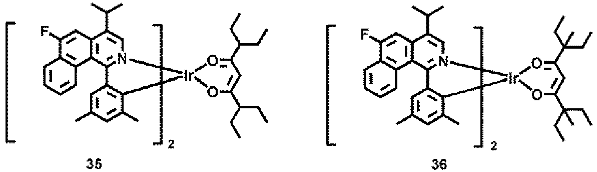

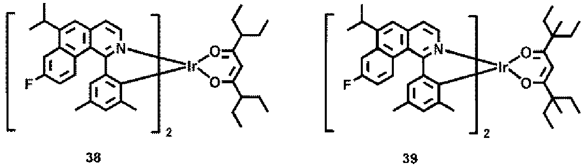

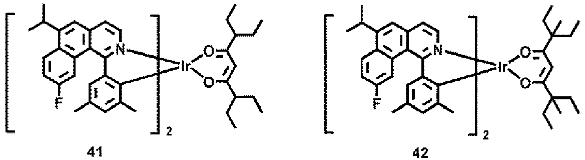

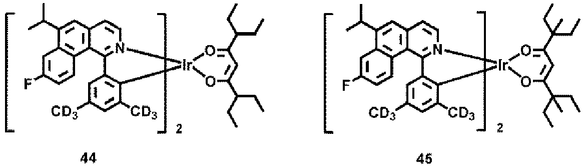

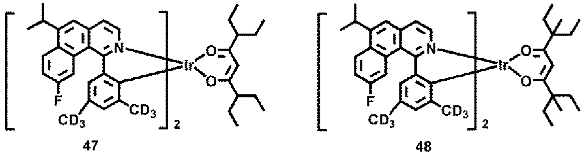

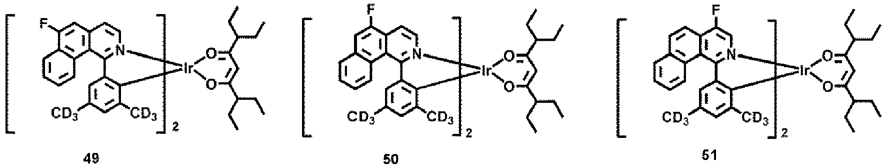

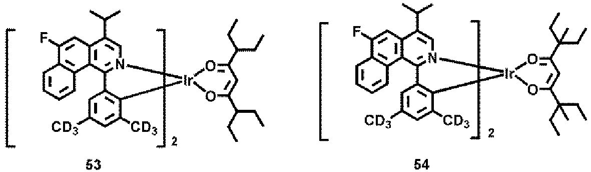

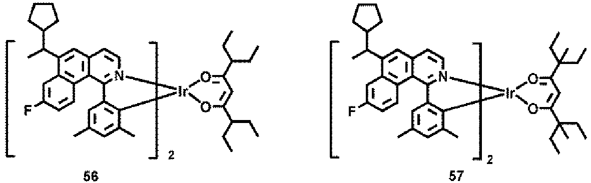

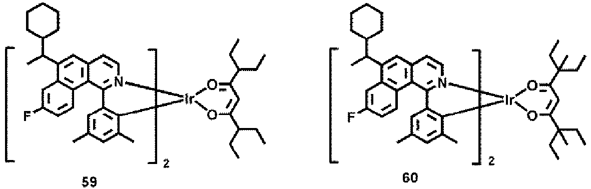

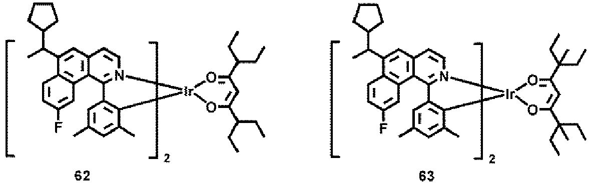

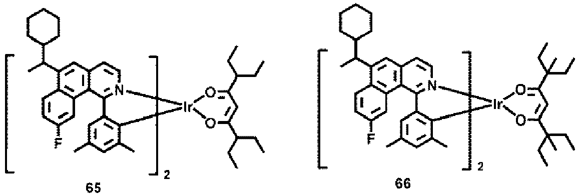

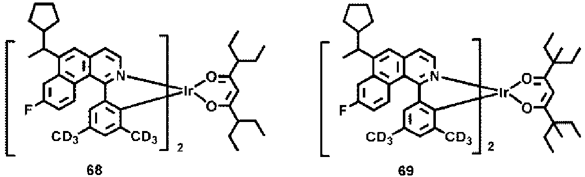

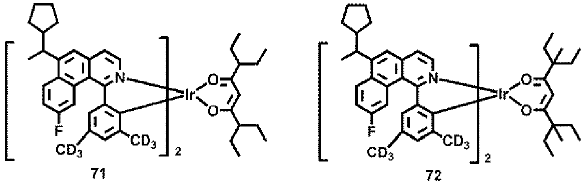

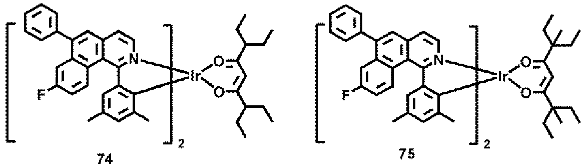

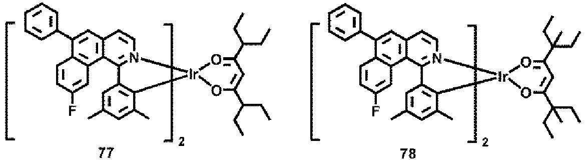

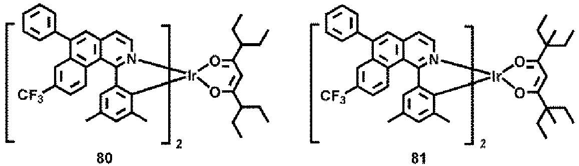

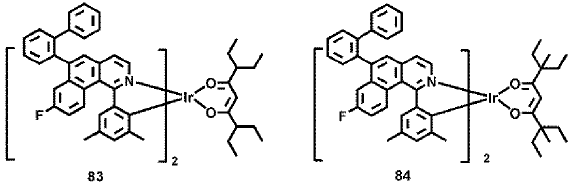

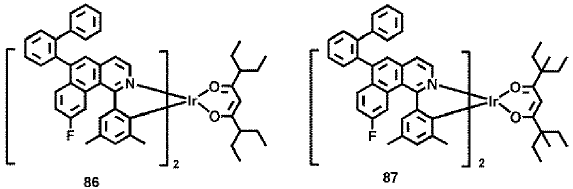

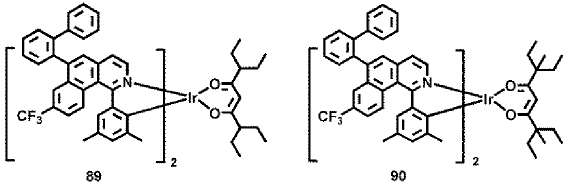

Since, in Formula 1, at least one of T₁(s) in number of a1 includes at least one fluoro group (-F), an electronic device including the organometallic compound represented by Formula 1, for example, an organic light-emitting device, may have a high emission efficiency.

In one or more embodiments, the organometallic compound represented by Formula 1 may satisfy Condition 1 described above, and thus lead to reduced intermolecular interaction in the organometallic compound. Although not limited by a specific theory, α-protons may have chemical reactivity about 10⁵ times higher than β-protons to produce intermediates in various forms during compound synthesis and/or storage, thus causing side reactions. However, since A₁ to A₆ in Formula 1 may satisfy Condition 1, carbons bound to A₁ to A₆ of Formula 1 may not include α-protons, and the organometallic compound, represented by Formula 1, satisfying Condition 1, may have a stable chemical structure in which side reactions before/after synthesis are minimized, and at the same time may have reduced intermolecular interaction during operation of an electronic device (for example, an organic light-emitting device) employing the organometallic compound.

In one or more embodiments, as the organometallic compound represented by Formula 1 satisfies at least one of Condition 2 to Condition 5, the organometallic compound represented by Formula 1 may have relatively large steric hindrance, thus reducing triplet-triplet annihilation. Accordingly, an electronic device, for example, an organic light-emitting device, using the organometallic compound represented by Formula 1 and satisfying at least one of Condition 2 to Condition 5 may have improved internal quantum luminescence efficiency.

In one or more embodiments, when the organometallic compound represented by Formula 1 satisfies at least one of Condition 1 to Condition 5, and/or when, Formula 1, a2 is 2 and T₂ is a substituted or unsubstituted C₁-C₆₀ alkyl group, due to enhanced interaction between Ligand 2 and Ligand 1 (see Formula 1' as below), the organometallic compound represented by Formula 1 may have improved rigidity. Thus, in the photoluminescent (PL) or electroluminescent (EL) spectrum of the organometallic compound represented by Formula 1, the full width at half maximum (FWHM) of the emission peak may be reduced, and the molecular vibrionic state of the organometallic compound represented by Formula 1 may be reduced, leading to reduction in non-radiative decay. Accordingly, an electronic device, for example, an organic light-emitting device, including the organometallic compound represented by Formula 1 may emit light of high color purity (for example, red light of high color purity), and at the same time have high emission efficiency and improved lifetime.

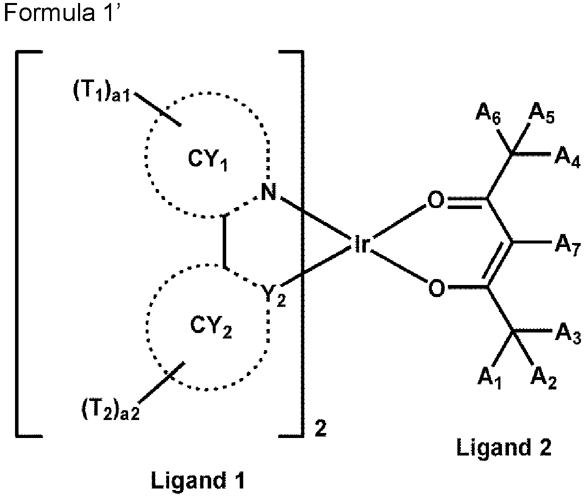

Accordingly, an electronic device, for example, an organic light-emitting device, using the organometallic compound represented by Formula 1, may have high emission efficiency and long lifetime.

A highest occupied molecular orbital (HOMO) energy level, a lowest unoccupied molecular orbital (LUMO) energy level, a singlet (S₁) energy level, and a triplet (T₁) energy level of some compounds of the organometallic compounds represented by Formula 1 were evaluated by a density functional theory (DFT) of Gaussian program with molecular structure optimization based on B3LYP, and the results are shown in Table 1.

**Table 1**

| Compound No. | HOMO (eV) | LUMO (eV) | gap (eV) | S₁ (eV) | T₁ (eV) |
|---|---|---|---|---|---|
| 1 | -4.725 | -1.854 | 2.871 | 2.240 | 1.991 |
| 2 | -4.696 | -1.842 | 2.854 | 2.190 | 1.971 |
| 3 | -4.681 | -1.790 | 2.891 | 2.254 | 1.996 |
| 4 | -4.937 | -2.243 | 2.694 | 2.071 | 1.823 |
| 5 | -4.790 | -1.939 | 2.851 | 2.209 | 1.943 |
| 6 | -4.748 | -1.998 | 2.750 | 2.127 | 1.859 |
| 7 | -4.781 | -1.766 | 3.015 | 2.384 | 2.181 |
| 8 | -4.769 | -1.947 | 2.822 | 2.188 | 1.960 |
| 9 | -4.870 | -2.085 | 2.785 | 2.142 | 1.950 |
| 10 | -4.678 | -1.786 | 2.892 | 2.222 | 1.996 |
| 11 | -4.716 | -1.844 | 2.872 | 2.188 | 1.960 |
| 12 | -4.675 | -1.805 | 2.870 | 2.204 | 1.979 |
| 19 | -4.812 | 1.993 | 2.819 | 2.161 | 1.947 |
| 20 | -4.828 | -2.005 | 2.823 | 2.157 | 1.938 |
| 21 | -4.727 | -1.974 | 2.753 | 2.104 | 1.916 |
| 22 | -4.865 | -2.129 | 2.736 | 2.127 | 1.913 |
| 23 | -4.790 | -2.041 | 2.749 | 2.106 | 1.909 |
| 24 | -4.870 | -2.118 | 2.752 | 2.077 | 1.866 |

Referring to Table 1, it is confirmed that the organometallic compound represented by Formula 1 have electric characteristics that are suitable for use in an electronic device, for example, as a dopant of an organic light-emitting device.

Methods of synthesizing the organometallic compound represented by Formula 1 can be understood by a person of ordinary skill in the art with reference to the synthesis examples described below.

Accordingly, the organometallic compound represented by Formula 1 is suitable for use in an organic layer of an organic light-emitting device, for example, as a dopant in an emission layer of the organic layer. Therefore, another aspect of the present disclosure provides an organic light-emitting device including: a first electrode; a second electrode; and an organic layer disposed between the first electrode and the second electrode and including an emission layer, the organic layer including at least one organometallic compound represented by Formula 1.

The organic light-emitting device may have, due to the inclusion of an organic layer including the above-described organometallic compound represented by Formula 1, an excellent driving voltage, excellent external quantum efficiency, an excellent roll-off ratio, a relatively small FWHM of the emission peak in the EL spectrum, and improved lifetime characteristics.

The organometallic compound represented by Formula 1 may be used between a pair of electrodes of an organic light-emitting device. For example, the organometallic compound represented by Formula 1 may be included in the emission layer. In this regard, the organometallic compound may act as a dopant, and the emission layer may further include a host (that is, an amount of the organometallic compound represented by Formula 1 is smaller than an amount of the host). The emission layer may emit, for example, red light or green light, for example, red light or green light having a maximum emission wavelength of about 500 nm or greater, for example, 500 nm or greater and 650 nm or less.

In one or more embodiments, the emission layer may emit red light.

The expression "(an organic layer) includes at least one organometallic compound represented by Formula 1" as used herein may be construed as that the organic layer includes one organometallic compound belonging to the category of Formula 1, or that the organic layer includes two or more different organometallic compounds belonging to the category of Formula 1.

For example, the organic layer may include, as the organometallic compound, only Compound 1. In this regard, Compound 1 may be only in the emission layer of the organic light-emitting device. In one or more embodiments, the organic layer may include, as the organometallic compound, Compound 1 and Compound 2. In this regard, Compound 1 and Compound 2 may be in the same layer (For example, both Compound 1 and Compound 2 may be in the emission layer).

The first electrode may be an anode, which is a hole injection electrode, and the second electrode may be a cathode, which is an electron injection electrode. In other embodiments, the first electrode may be a cathode, which is an electron injection electrode, and the second electrode may be an anode, which is a hole injection electrode.

For example, in the organic light-emitting device, the first electrode may be an anode, the second electrode may be a cathode, the organic layer may further include a hole transport region between the first electrode and the emission layer and an electron transport region between the emission layer and the second electrode, the hole transport region may include a hole injection layer, a hole transport layer, an electron blocking layer, a buffer layer, or any combination thereof, and the electron transport region may include a hole blocking layer, an electron transport layer, an electron injection layer, or any combination thereof.

The term "organic layer" as used herein refers to a single layer and/or a plurality of layers disposed between the first electrode and the second electrode of an organic light-emitting device. The "organic layer" may include, in addition to an organic compound, an organometallic complex including metal.

FIGURE is a schematic cross-sectional view of an organic light-emitting device 10 according to an embodiment. Hereinafter, the structure of an organic light-emitting device according to an embodiment and a method of manufacturing an organic light-emitting device according to an embodiment will be described with reference to FIGURE. The organic light-emitting device 10 may have a structure in which a first electrode 11, an organic layer 15, and a second electrode 19 which are sequentially stacked.

A substrate may be additionally disposed under the first electrode 11 or above the second electrode 19. For use as the substrate, any substrate that is used in general organic light-emitting devices may be used. The substrate may be a glass substrate or a transparent plastic substrate, each having excellent mechanical strength, thermal stability, transparency, surface smoothness, ease of handling, and water resistance.

The first electrode 11 may be formed by depositing or sputtering a material for forming the first electrode 11 on the substrate. The first electrode 11 may be an anode. The material for forming the first electrode 11 may include a material(s) with a high work function to facilitate hole injection. The first electrode 11 may be a reflective electrode, a semi-reflective electrode, or a transmissive electrode. The material for forming the first electrode may be, for example, indium tin oxide (ITO), indium zinc oxide (IZO), tin oxide (SnO₂), and zinc oxide (ZnO). In one or more embodiments, the material for forming the first electrode 11 may be metal, such as magnesium (Mg), aluminum (Al), aluminum-lithium (Al-Li), calcium (Ca), magnesium-indium (Mg-In), or magnesium-silver (Mg-Ag).

The first electrode 11 may have a single-layered structure or a multi-layered structure including two or more layers. For example, the first electrode 11 may have a three-layered structure of ITO/Ag/ITO.

The organic layer 15 may be disposed on the first electrode 11.

The organic layer 15 may include a hole transport region, an emission layer, and an electron transport region.

The hole transport region may be disposed between the first electrode 11 and the emission layer.

The hole transport region may include a hole injection layer, a hole transport layer, an electron blocking layer, a buffer layer, or any combination thereof.

The hole transport region may include only either a hole injection layer or a hole transport layer. In one or more embodiments, the hole transport region may have a hole injection layer/hole transport layer structure or a hole injection layer/hole transport layer/electron blocking layer structure, which are sequentially stacked in this stated order from the first electrode 11.

When the hole transport region includes a hole injection layer (HIL), the hole injection layer may be formed on the first electrode 11 by using one or more suitable methods, for example, vacuum deposition, spin coating, casting, and/or Langmuir-Blodgett (LB) deposition.

When a hole injection layer is formed by vacuum deposition, the deposition conditions may vary according to a material that is used to form the hole injection layer, and the structure and thermal characteristics of the hole injection layer. For example, the deposition conditions may include a deposition temperature of about 100 °C to about 500 °C, a vacuum pressure of about 1.33×10⁻⁶ to about 0.133 Pa (about 10⁻⁶ to about 10⁻³ torr), and a deposition rate of about 0.01 Å/sec to about 100 Å/sec. However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2,000 rpm to about 5,000 rpm, and a temperature at which a heat treatment is performed to remove a solvent after coating may be from about 80 °C to about 200 °C. However, the coating conditions are not limited thereto.

Conditions for forming a hole transport layer and an electron blocking layer may be understood by referring to the conditions for forming the hole injection layer.

The hole transport region may include m-MTDATA, TDATA, 2-TNATA, NPB, β-NPB, TPD, spiro-TPD, spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201 below, a compound represented by Formula 202 below, or any combination thereof:

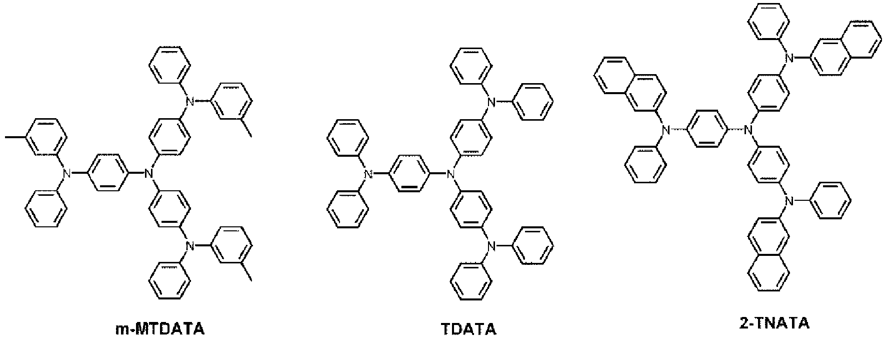

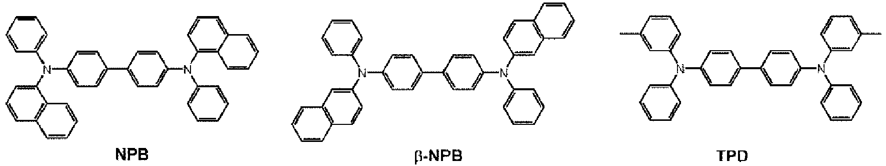

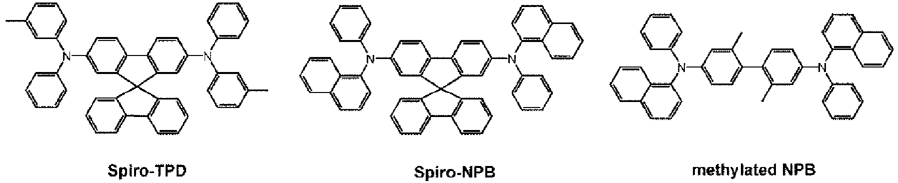

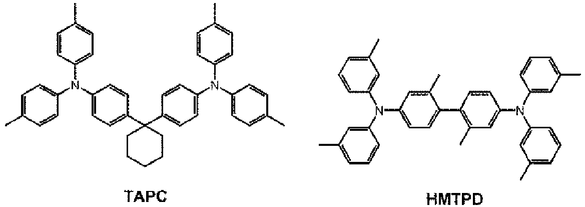

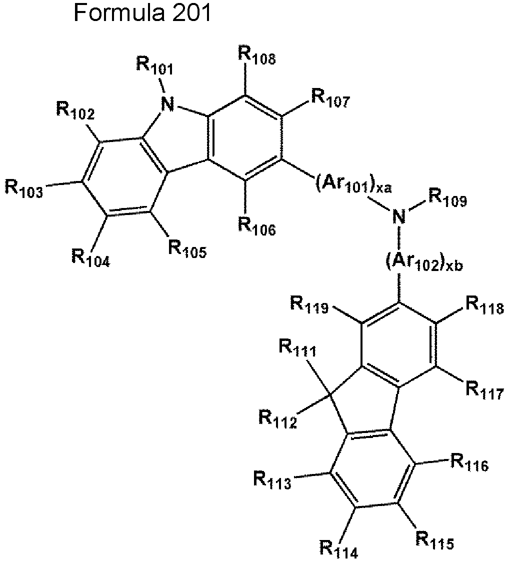

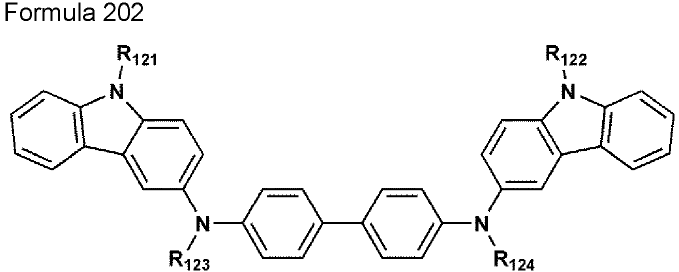

In Formula 201, Ar₁₀₁ and Ar₁₀₂ may each independently be a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group, each unsubstituted or substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₁-C₁₀ heterocycloalkyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, or any combination thereof.

The designations xa and xb in Formula 201 may each independently be an integer from 0 to 5, or may be 0, 1 or 2. For example, xa may be 1 and xb may be 0. However, embodiments are not limited thereto.

In Formulae 201 and 202, R₁₀₁ to R₁₀₈, R₁₁₁ to R₁₁₉, and R₁₂₁ to R₁₂₄ may each independently be:
hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group (for example, a methyl group, an ethyl group, a propyl group, a butyl group, pentyl group, a hexyl group, or the like), or a C₁-C₁₀ alkoxy group (for example, a methoxy group, an ethoxy group, a propoxy group, a butoxy group, a pentoxy group, or the like);
a C₁-C₁₀ alkyl group or a C₁-C₁₀ alkoxy group, each unsubstituted or substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, or any combination thereof; or
a phenyl group, a naphthyl group, an anthracenyl group, a fluorenyl group, or a pyrenyl group, each unsubstituted or substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, or any combination thereof.

In Formula 201, R₁₀₉ may be a phenyl group, a naphthyl group, an anthracenyl group, or a pyridinyl group, each unsubstituted or substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, an anthracenyl group, a pyridinyl group, or any combination thereof.

In one or more embodiments, the compound represented by Formula 201 may be represented by Formula 201A.

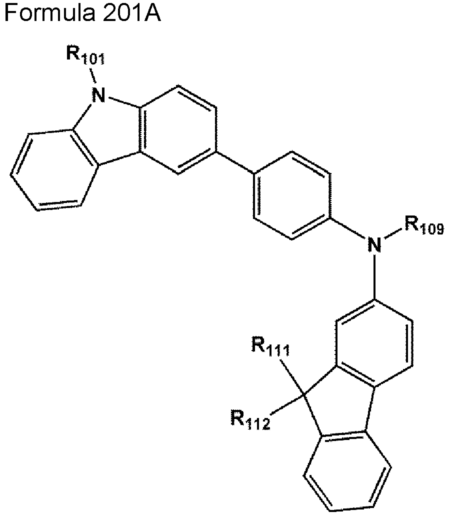

Detailed descriptions of R₁₀₁, R₁₁₁, R₁₁₂, and R₁₀₉ in Formula 201A are the same as described above.

For example, the hole transport region may include one of Compounds HT1 to HT21 or any combination thereof.

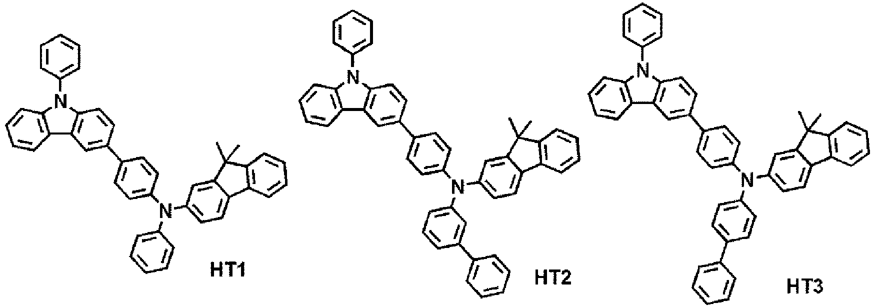

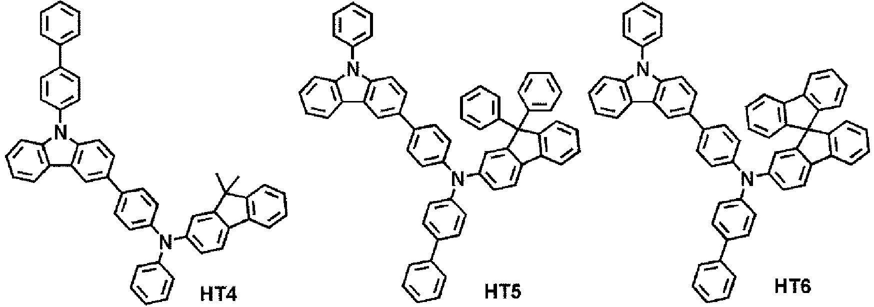

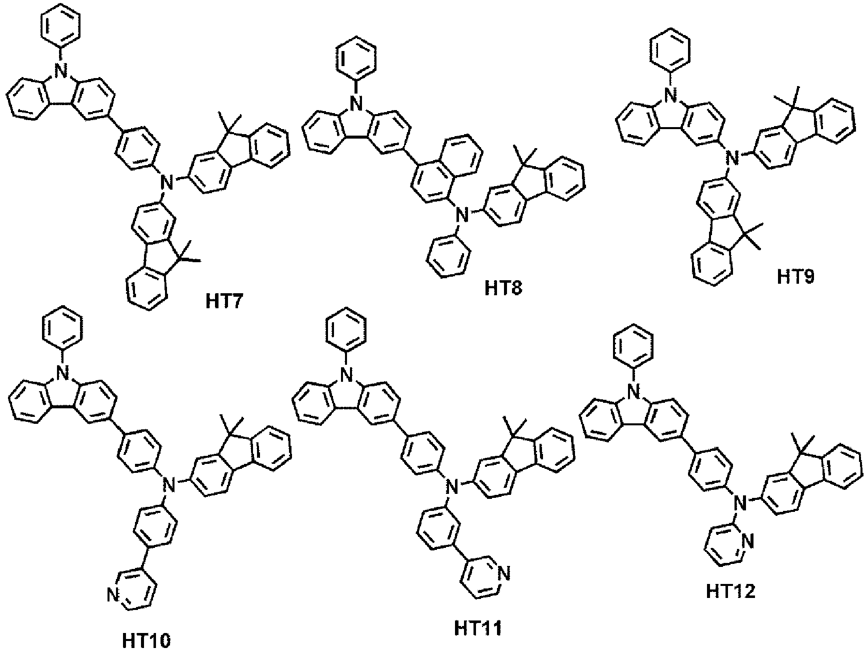

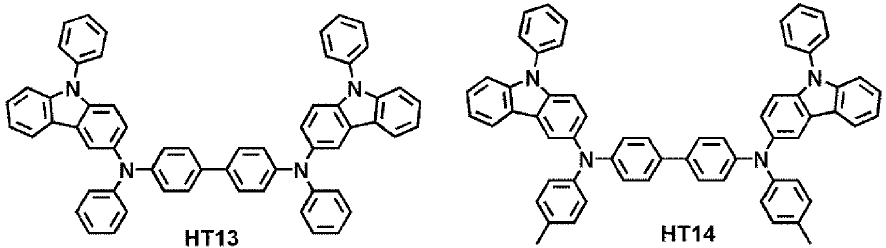

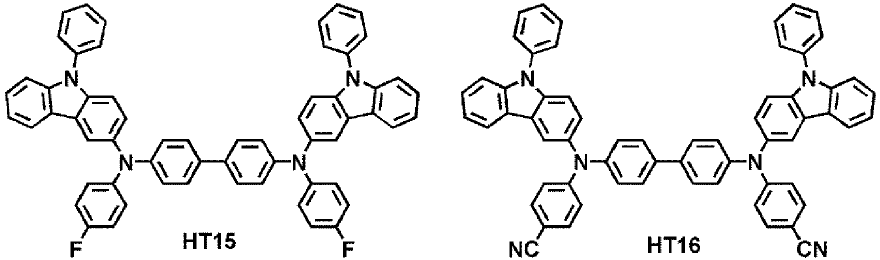

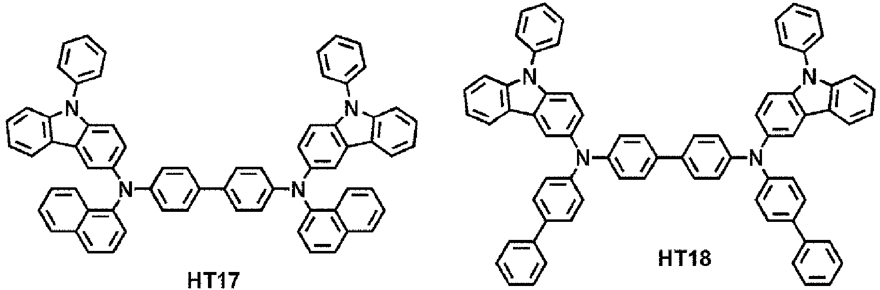

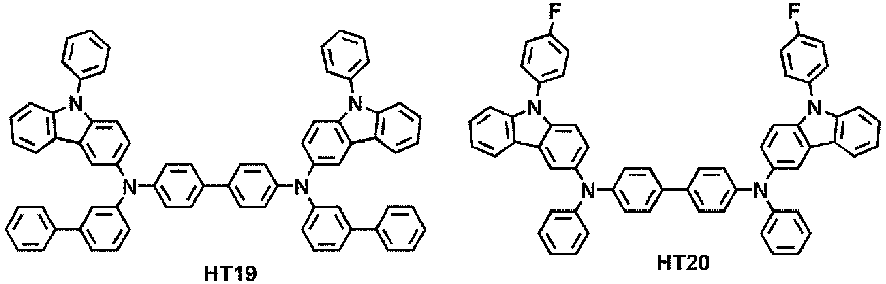

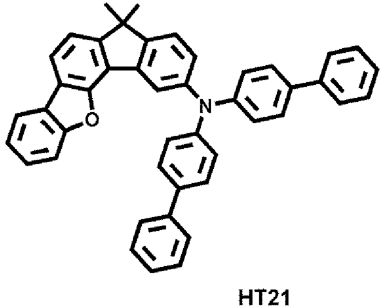

A thickness of the hole transport region may be from about 100 Å to about 10,000 Å, for example, about 100 Å to about 1,000 Å. When the hole transport region includes a hole injection layer, a hole transport layer, an electron blocking layer, or any combination thereof, a thickness of the hole injection layer may be in a range of about 100 Å to about 10000 Å, for example, about 100 Å to about 1,000 Å, and a thickness of the hole transport layer may be in a range of about 50 Å to about 2,000 Å, for example about 100 Å to about 1,500 Å. When the thicknesses of the hole transport region, the hole injection layer, and the hole transport layer are within these ranges, satisfactory hole transporting characteristics may be obtained without a substantial increase in driving voltage.

The hole transport region may further include, in addition to the above-described materials, a charge-generation material for the improvement of conductive properties. The charge-generation material may be homogeneously or non-homogeneously dispersed in the hole transport region.

The charge-generation material may be, for example, a p-dopant. The p-dopant may be a quinone derivative, a metal oxide, a cyano group-containing compound, or any combination thereof. For example, the p-dopant may be a quinone derivative, such as tetracyanoquinonedimethane (TCNQ), 2,3,5,6-tetrafluoro-tetracyano-1,4-benzoquinonedimethane (F4-TCNQ), or F6-TCNNQ; a metal oxide, such as a tungsten oxide or a molybdenum oxide; or a cyano group-containing compound, such as Compound HT-D1; or any combination thereof.

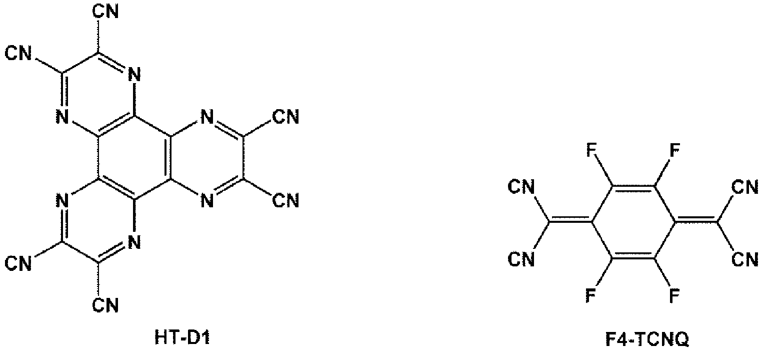

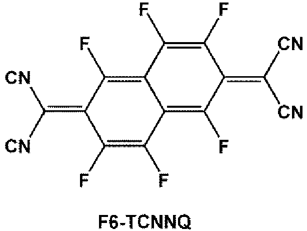

The hole transport region may further include a buffer layer.

The buffer layer may compensate for an optical resonance distance according to a wavelength of light emitted from the emission layer, and thus increase efficiency.

Meanwhile, when the hole transport region includes an electron blocking layer, a material for the electron blocking layer may be materials for the hole transport region described above, materials for a host which will be described later, or any combination thereof. For example, when the hole transport region includes an electron blocking layer, a material for the electron blocking layer may be mCP, which will be explained later, Compound H21, or any combination thereof.

Then, an emission layer may be formed on the hole transport region by vacuum deposition, spin coating, casting, LB deposition, or the like. When the emission layer is formed by vacuum deposition or spin coating, the deposition or coating conditions may be similar to those applied in forming the hole injection layer although the deposition or coating conditions may vary according to a compound that is used to form the emission layer.

The emission layer may include a host and a dopant, and the dopant may include the organometallic compound represented by Formula 1 described herein.

The host may include TPBi, TBADN, ADN (also referred to as "DNA"), CBP, CDBP, TCP, mCP, Compounds H50, Compound H51, Compound H52, or any combination thereof.

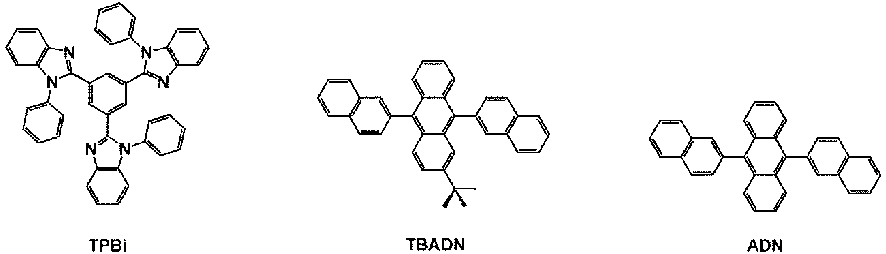

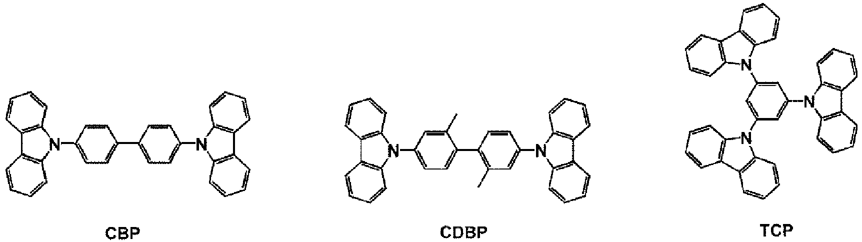

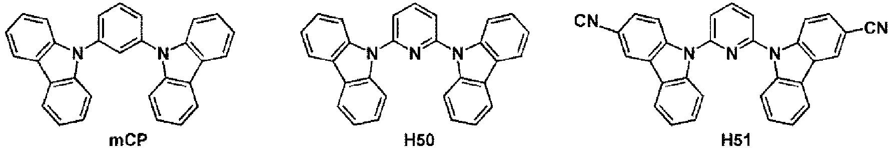

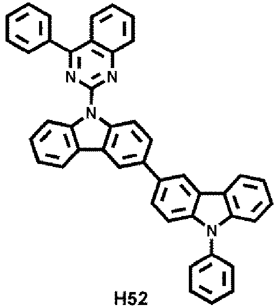

When the organic light-emitting device is a full-color organic light-emitting device, the emission layer may be patterned into a red emission layer, a green emission layer, and/or a blue emission layer. In one or more embodiments, due to having a stacked structure including a red emission layer, a green emission layer, and/or a blue emission layer, the emission layer may emit white light.

When the emission layer includes a host and a dopant, an amount of the dopant may be in a range of about 0.01 parts by weight to about 15 parts by weight based on 100 parts by weight of the host.

A thickness of the emission layer may be in a range of about 100 Å to about 1,000 Å, for example, about 200 Å to about 600 Å. When the thickness of the emission layer is within this range, excellent light-emission characteristics may be obtained without a substantial increase in driving voltage.

Then, an electron transport region may be disposed on the emission layer.

The electron transport region may include a hole blocking layer, an electron transport layer, an electron injection layer, or any combination thereof.

For example, the electron transport region may have a hole blocking layer/electron transport layer/electron injection layer structure or an electron transport layer/electron injection layer structure. The electron transport layer may have a single-layered structure or a multi-layered structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, BCP, Bphen, BAlq, or any combination thereof.

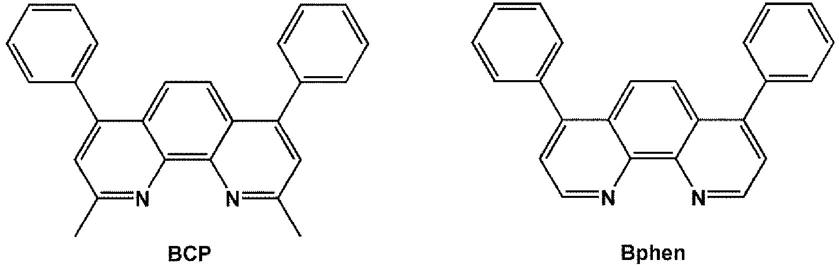

In one or more embodiment, the hole blocking layer may include the host, materials for an electron transport layer and an electron injection layer, which will be described later, or any combination thereof.

A thickness of the hole blocking layer may be from about 20 Å to about 1,000 Å, for example, about 30 Å to about 600 Å. When the thickness of the hole blocking layer is within these ranges, the hole blocking layer may have excellent hole blocking characteristics without a substantial increase in driving voltage.

The electron transport layer may include BCP, Bphen, TPBi, Alq₃, BAlq, TAZ, NTAZ, or any combination thereof.

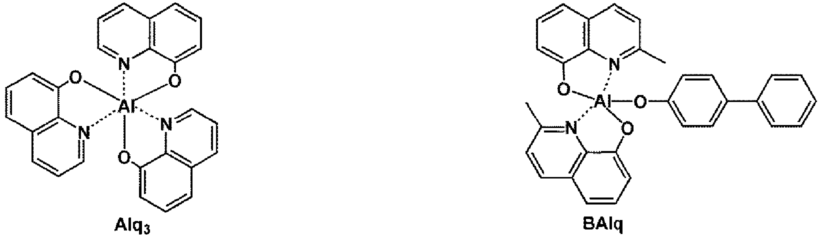

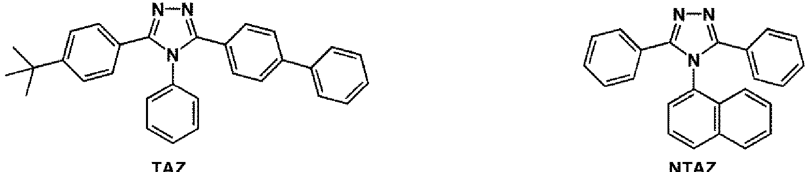

In one or more embodiments, the electron transport layer may include one of Compounds ET1 to ET25, or any combination thereof.

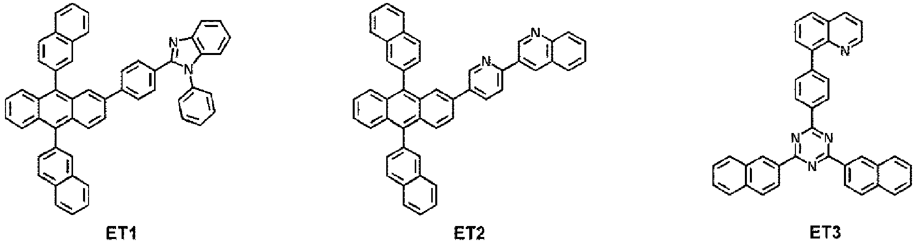

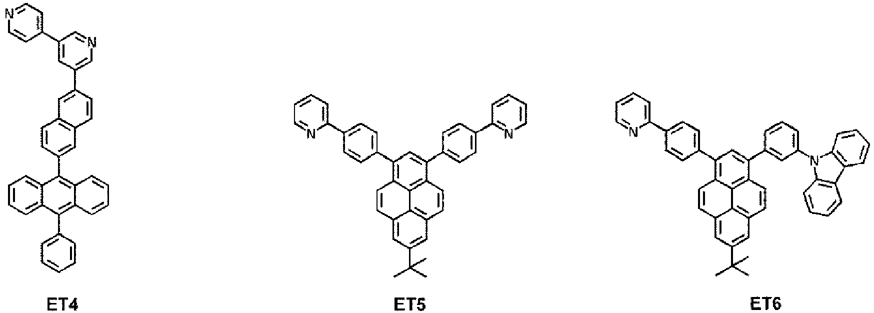

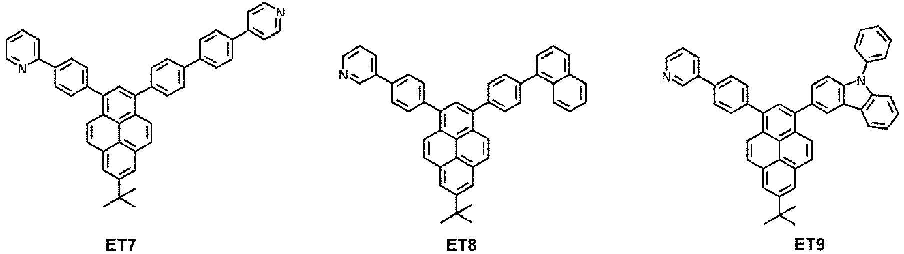

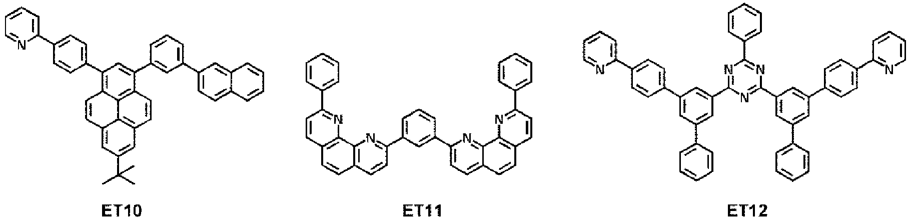

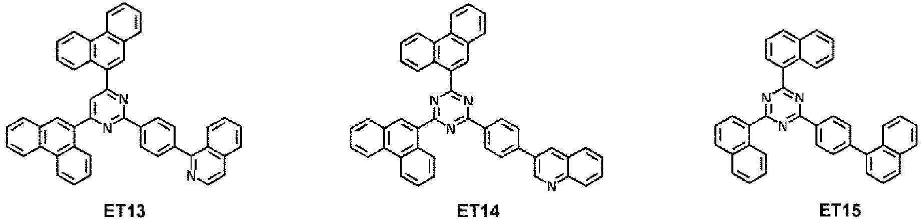

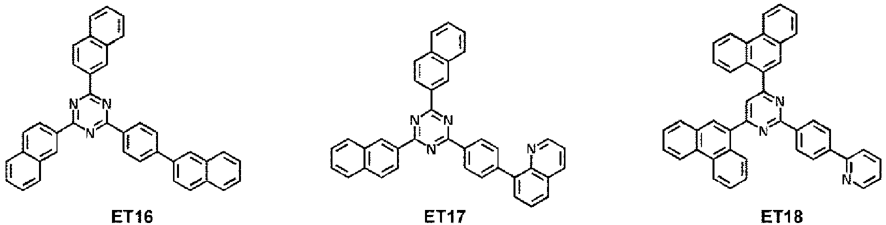

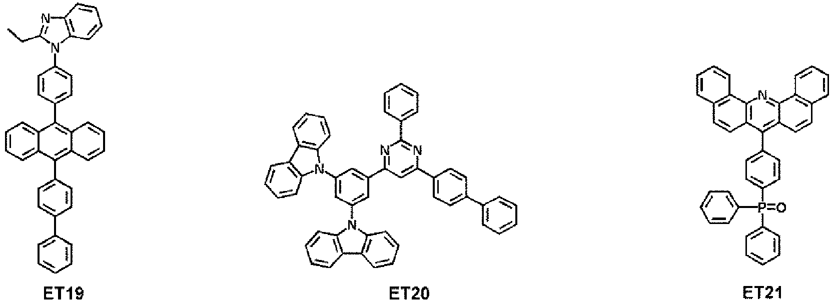

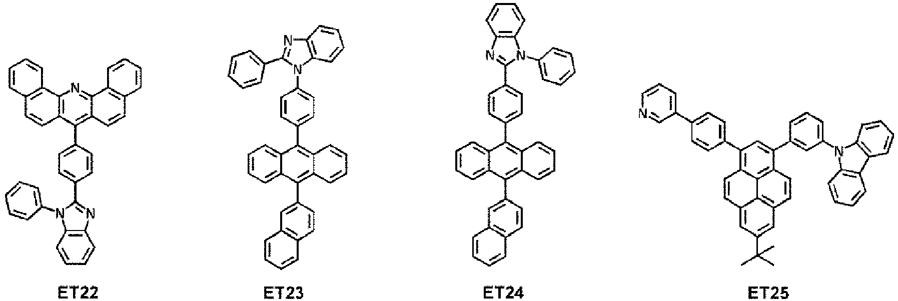

A thickness of the electron transport layer may be from about 100 Å to about 1,000 Å, for example, about 150 Å to about 500 Å. When the thickness of the electron transport layer is within the above-described ranges, the electron transport layer may have satisfactory electron transport characteristics without a substantial increase in driving voltage.

The electron transport layer may further include, in addition to the materials described above, a metal-containing material.

The metal-containing material may include a Li complex. The Li complex may include, for example, Compound ET-D1 (LiQ), Compound ET-D2, or any combination thereof:

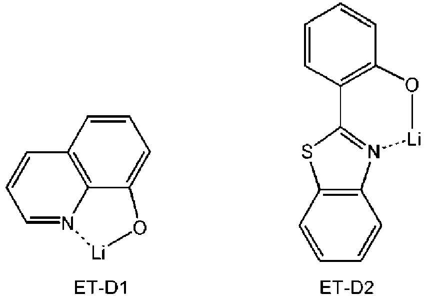

The electron transport region may include an electron injection layer that facilitates flow of electrons from the second electrode 19.

The electron injection layer may include LiF, NaCl, CsF, Li₂O, BaO, or any combination thereof.

A thickness of the electron injection layer may be from about 1 Å to about 100 Å, for example, about 3 Å to about 90 Å. When a thickness of the electron injection layer is within these ranges, satisfactory electron injection characteristics may be obtained without substantial increase in driving voltage.

The second electrode 19 is disposed on the organic layer 15. The second electrode 19 may be a cathode. A material for forming the second electrode 19 may be a metal, an alloy, an electrically conductive compound, or any combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be used as the material for forming the second electrode 19. To manufacture a top-emission type light-emitting device, a transmissive electrode formed using ITO or IZO may be used as the second electrode 19.

Hereinbefore, the organic light-emitting device according to an embodiment has been described with reference to FIGURE. However, embodiments are not limited thereto

In one or more embodiments, the organic light-emitting device may be included in an electronic apparatus. Accordingly, another aspect provides an electronic apparatus including the organic light-emitting device. For example, the electronic apparatus may be a display, an illuminator, or a sensor.

Another aspect of the present disclosure provides a diagnostic composition including at least one organometallic compounds represented by Formula 1.

The organometallic compound represented by Formula 1 provides high luminescent efficiency. Accordingly, a diagnostic composition including the organometallic compound may have high diagnostic efficiency.

The diagnostic composition may have various applications, for example, in a diagnosis kit, a diagnosis reagent, a biosensor, and a biomarker.

The term "C₁-C₆₀ alkyl group" as used herein refers to a linear or branched saturated aliphatic hydrocarbon monovalent group having 1 to 60 carbon atoms. The term "C₁-C₆₀ alkylene group" as used herein refers to a divalent group having the same structure as the C₁-C₆₀ alkyl group.

Non-limiting examples of the C₁-C₆₀ alkyl group, the C₁-C₂₀ alkyl group and/or the C₁-C₁₀ alkyl group as used herein may include a methyl group, an ethyl group, an n-propyl group, an iso-propyl group, an n-butyl group, a sec-butyl group, an isobutyl group, a tert-butyl group, an n-pentyl group, a tert-pentyl group, a neopentyl group, an isopentyl group, a sec-pentyl group, 3-pentyl group, a sec-isopentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, or a tert-decyl group, each unsubstituted or substituted with a methyl group, an ethyl group, an n-propyl group, an iso-propyl group, an n-butyl group, a sec-butyl group, an isobutyl group, a tert-butyl group, an n-pentyl group, a tert-pentyl group, a neopentyl group, an isopentyl group, a sec-pentyl group, 3-pentyl group, a sec-isopentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, or any combination thereof. For example, the group represented by Formula 9-33, as described above, which is a branched C₆ alkyl group, may be a tert-butyl group substituted with two methyl groups.

The term "C₁-C₆₀ alkoxy group" used herein refers to a monovalent group represented by -OA₁₀₁ (wherein A₁₀₁ is a C₁-C₆₀ alkyl group). Non-limiting examples of the C₁-C₆₀ alkoxy group, the C₁-C₂₀ alkoxy group, or the C₁-C₁₀ alkoxy group may include a methoxy group, an ethoxy group, a propoxy group, a butoxy group, and a pentoxy group.

The term "C₂-C₆₀ alkenyl group" as used herein refers to a hydrocarbon group having at least one carbon-carbon double bond in the middle or at the terminus of the C₂-C₆₀ alkyl group, and examples thereof include an ethenyl group, a propenyl group, and a butenyl group. The term "C₂-C₆₀ alkenylene group" as used herein refers to a divalent group having the same structure as the C₂-C₆₀ alkenyl group.

The term "C₂-C₆₀ alkynyl group" as used herein refers to a hydrocarbon group having at least one carbon-carbon triple bond in the middle or at the terminus of the C₂-C₆₀ alkyl group, and examples thereof include an ethynyl group, and a propynyl group. The term "C₂-C₆₀ alkynylene group" as used herein refers to a divalent group having the same structure as the C₂-C₆₀ alkynyl group.

The term "C₃-C₁₀ cycloalkyl group" as used herein refers to a monovalent saturated hydrocarbon monocyclic group having 3 to 10 carbon atoms, and the term "C₃-C₁₀ cycloalkylene group" as used herein refers to a divalent group having the same structure as the C₃-C₁₀ cycloalkyl group.

Non-limiting examples of the C₃-C₁₀ cycloalkyl group include a cyclopropyl group, a cyclobutyl group, a cyclopentyl group, a cyclohexyl group, a cycloheptyl group, a cyclooctyl group, an adamantyl group, a bicyclo[1.1.1]pentyl group, a bicyclo[2.1.1]hexyl group, a bicyclo[2.2.1]heptyl group(a norbornyl group), and a bicyclo[2.2.2]octyl group.

The term "C₁-C₁₀ heterocycloalkyl group" as used herein refers to a monovalent monocyclic group having 1 to 10 carbon atoms and including, as a ring-forming atom, at least one of N, O, P, Si, and S. The term "C₁-C₁₀ heterocycloalkylene group" as used herein refers to a divalent group having the same structure as the C₁-C₁₀ heterocycloalkyl group.

Examples of the C₁-C₁₀ heterocycloalkyl group herein may include a silolanyl group, a silinanyl group, a tetrahydrofuranyl group, a tetrahydro-2H-pyranyl group, a tetrahydrothiophenyl group.

The term "C₃-C₁₀ cycloalkenyl group" as used herein refers to a monovalent monocyclic group having 3 to 10 carbon atoms and at least one carbon-carbon double bond in the ring thereof but no aromaticity, and non-limiting examples thereof include a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₂-C₁₀ heterocycloalkenyl group" as used herein refers to a monovalent monocyclic group having 1 to 10 carbon atoms and including, as a ring-forming atom, at least one of N, O, P, Si, S, or any combination thereof, and at least one double bond in the ring thereof. Non-limiting examples of the C₂-C₁₀ heterocycloalkenyl group include a 2,3-dihydrofuranyl group and a 2,3-dihydrothiophenyl group. The term "C₂-C₁₀ heterocycloalkenylene group" as used herein refers to a divalent group having the same structure as the C₂-C₁₀ heterocycloalkenyl group.

The term "C₆-C₆₀ aryl group" as used herein refers to a monovalent group having a carbocyclic aromatic system having 6 to 60 carbon atoms, and the term "C₆-C₆₀ arylene group" as used herein refers to a divalent group having a carbocyclic aromatic system having 6 to 60 carbon atoms. Non-limiting examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other.

The term "C₇-C₆₀ alkylaryl group" as used herein refers to a C₆-C₆₀ aryl group substituted with at least one C₁-C₆₀ alkyl group.

The term "C₁-C₆₀ heteroaryl group" as used herein refers to a monovalent group having a heterocyclic aromatic system having 1 to 60 carbon atoms and including, as a ring-forming atom, at least one of N, O, P, Si, Se, Ge, B, S, or any combination thereof. The term "C₁-C₆₀ heteroarylene group" as used herein refers to a divalent group having a heterocyclic aromatic system having 1 to 60 carbon atoms and including, as a ring-forming atom, at least one N, O, P, Si, Se, Ge, B, S, or any combination thereof. Non-limiting examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the rings may be fused to each other.

The term "C₂-C₆₀ alkylheteroaryl group" as used herein refers to a C₁-C₆₀ heteroaryl group substituted with at least one C₁-C₆₀ alkyl group.

The term "C₆-C₆₀ aryloxy group" used herein indicates -OA₁₀₂ (wherein A₁₀₂ is a C₆-C₆₀ aryl group as described above), the term "C₆-C₆₀ arylthio group" used herein indicates -SA₁₀₃ (wherein A₁₀₃ is a C₆-C₆₀ aryl group as described above), and the term "C₁-C₆₀ alkylthio group" used herein indicates -SA₁₀₄ (wherein A₁₀₄ indicates a C₁-C₆₀ alkyl group as described above).

The term "monovalent non-aromatic condensed polycyclic group" as used herein refers to a monovalent group (for example, having 8 to 60 carbon atoms) having two or more rings condensed to each other, and only carbon atoms as ring-forming atoms, and in which the whole molecular structure has no aromaticity. Examples of the monovalent non-aromatic condensed polycyclic group include a fluorenyl group. The term "divalent non-aromatic condensed polycyclic group" as used herein refers to a divalent group having the same structure as the monovalent non-aromatic condensed polycyclic group.

The term "monovalent non-aromatic condensed heteropolycyclic group" as used herein refers to a monovalent group (for example, having 1 to 60 carbon atoms) having two or more rings condensed to each other, and including as ring-forming atoms, in addition to carbon atoms, at least one heteroatom selected from N, O, P, Si, Se, Ge, B, S, or any combination thereof and in which the whole molecular structure has no aromaticity. Non-limiting examples of the monovalent non-aromatic condensed heteropolycyclic group include a carbazolyl group. The term "divalent non-aromatic condensed heteropolycyclic group" as used herein refers to a divalent group having the same structure as the monovalent non-aromatic condensed heteropolycyclic group.

The term "C₅-C₃₀ carbocyclic group" as used herein refers to a saturated or unsaturated cyclic group having, as a ring-forming atom, 5 to 30 carbon atoms only. The C₅-C₃₀ carbocyclic group may be a monocyclic group or a polycyclic group. The term "C₅-C₃₀ carbocyclic group (unsubstituted or substituted with at least one R₁ₐ)" may include, for example, an adamantane group, a norbornene group, a bicyclo[1.1.1]pentane group, a bicyclo[2.1.1]hexane group, a bicyclo[2.2.1]heptane group(norbornane group), a bicyclo[2.2.2]octane group, a cyclopentane group, a cyclohexane group, a cyclohexene group, a benzene group, a naphthalene group, an anthracene group, a phenanthrene group, a triphenylene group, a pyrene group, a chrysene group, a 1,2,3,4-tetrahydronaphthalene group, a cyclopentadiene group, a silole group, and a fluorene group ( which are each unsubstituted or substituted with at least one R₁ₐ).

The term "C₁-C₃₀ heterocyclic group" as used herein refers to a saturated or unsaturated cyclic group having, as ring-forming atoms, in addition to 1 to 30 carbon atoms, at least N, O, P, Si, Se, Ge, B, S, or any combination thereof. The C₁-C₃₀ heterocyclic group may be a monocyclic group or a polycyclic group. The "C₁-C₃₀ heterocyclic group (unsubstituted or substituted with at least one R₁ₐ) " may be, for example, a thiophene group, a furan group, a pyrrole group, a silole group, a borole group, a phosphole group, a selenophene group, a germole group, a benzothiophene group, a benzofuran group, an indole group, an indene group, a benzosilole group, a benzoborole group, a benzophosphole group, a benzoselenophene group, a benzogermole group, a dibenzothiophene group, a dibenzofuran group, a carbazole group, a dibenzosilole group, a dibenzoborole group, a dibenzophosphole group, a dibenzoselenophene group, a dibenzogermole group, a dibenzothiophene 5-oxide group, a 9H-fluorene-9-one group, a dibenzothiophene 5,5-dioxide group, an azabenzothiophene group, an azabenzofuran group, an azaindole group, an azaindene group, an azabenzosilole group, an azabenzoborole group, an azabenzophosphole group, an azabenzoselenophene group, an azabenzogermole group, an azadibenzothiophene group, an azadibenzofuran group, an azacarbazole group, an azafluorene group, an azadibenzosilole group, an azadibenzoborole group, an azadibenzophosphole group, an azadibenzoselenophene group, an azadibenzogermole group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrazole group, an imidazole group, a triazole group, an oxazole group, an isoxazole group, a thiazole group, an isothiazole group, an oxadiazole group, a thiadiazol group, a benzopyrazole group, a benzimidazole group, a benzoxazole group, a benzothiazole group, a benzoxadiazole group, a benzothiadiazol group, a 5,6,7,8-tetrahydroisoquinoline group, or a 5,6,7,8-tetrahydroquinoline group, (which are each unsubstituted or substituted with at least one R₁ₐ).

The terms "fluorinated C₁-C₆₀ alkyl group (or a fluorinated C₁-C₂₀ alkyl group, etc.)," "fluorinated C₃-C₁₀ cycloalkyl group," "fluorinated C₁-C₁₀ heterocycloalkyl group," and "fluorinated phenyl group" as used herein, refer to, respectively a C₁-C₆₀ alkyl group, a C₃-C₁₀ cycloalkyl group, a C₁-C₁₀ heterocycloalkyl group, and a phenyl group, each substituted with at least one fluoro group (-F). Examples of the "fluorinated C₁ alkyl group (i.e., a fluorinated methyl group)" may include -CF₃, - CF₂H, and -CFH₂. The term "fluorinated C₁-C₆₀ alkyl group (or fluorinated C₁-C₂₀ alkyl group, etc.)," "fluorinated C₃-C₁₀ cycloalkyl group", "fluorinated C₁-C₁₀ heterocycloalkyl group", or "fluorinated phenyl group" may be, i) a fully fluorinated C₁-C₆₀ alkyl group (or a fully fluorinated C₁-C₂₀ alkyl group, etc.), a fully fluorinated C₃-C₁₀ cycloalkyl group, a fully fluorinated C₁-C₁₀ heterocycloalkyl group, a fully fluorinated phenyl group, all the hydrogens in each group substituted with fluoro groups, or ii) a partially fluorinated C₁-C₆₀ alkyl group (or partially fluorinated C₁-C₂₀ alkyl group, etc.), a partially fluorinated C₃-C₁₀ cycloalkyl group, a partially fluorinated C₁-C₁₀ heterocycloalkyl group, or partially fluorinated phenyl group, all the hydrogens in each group partially, i.e., not fully, substituted with fluoro groups.

The terms "deuterated C₁-C₆₀ alkyl group (or deuterated C₁-C₂₀ alkyl group, etc.) ", " deuterated C₃-C₁₀ cycloalkyl group", " deuterated C₁-C₁₀ heterocycloalkyl group," and "deuterated phenyl group" as used herein may refer to, respectively, a C₁-C₆₀ alkyl group (or a C₁-C₂₀ alkyl group, etc.), a C₃-C₁₀ cycloalkyl group, a C₁-C₁₀ heterocycloalkyl group, and a phenyl group, each substituted with at least one deuterium. Examples of the "deuterated C₁ alkyl group (i.e., a deuterated methyl group)" may include -CD₃, -CD₂H, and -CDH₂. An example of the "deuterated C₃-C₁₀ cycloalkyl group" may be the group represented by Formula 10-501 as described above. The terms "deuterated C₁-C₆₀ alkyl group (or deuterated C₁-C₂₀ alkyl group, etc.)," "deuterated C₃-C₁₀ cycloalkyl group", "deuterated C₁-C₁₀ heterocycloalkyl group", or "deuterated phenyl group" may be i) a fully deuterated C₁-C₆₀ alkyl group (or fully a deuterated C₁-C₂₀ alkyl group, etc.), a fully deuterated C₃-C₁₀ cycloalkyl group, a fully deuterated C₁-C₁₀ heterocycloalkyl group, or a fully deuterated phenyl group, all the hydrogens in each group substituted with deuterium, or ii) a partially deuterated C₁-C₆₀ alkyl group (or partially deuterated C₁-C₂₀ alkyl group, etc.), a partially deuterated C₃-C₁₀ cycloalkyl group, a partially deuterated C₁-C₁₀ heterocycloalkyl group, or a partially deuterated phenyl group, all the hydrogens in each group partially, not fully, substituted with deuterium.

The term "(C₁-C₂₀ alkyl) 'X' group" used herein refers to an 'X' group substituted with at least one C₁-C₂₀ alkyl group. For example, the term "(C₁-C₂₀ alkyl)C₃-C₁₀ cycloalkyl group" as used herein indicates a C₃-C₁₀ cycloalkyl group substituted with at least one C₁-C₂₀ alkyl group, and the term "(C₁-C₂₀ alkyl)phenyl group" indicates a phenyl group substituted with at least one C₁-C₂₀ alkyl group. For example, the (C₁ alkyl)phenyl group may be a toluyl group.

As used herein, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, and an azadibenzothiophene 5,5-dioxide group may refer to hetero rings having the same backbone as an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, and a dibenzothiophene 5,5-dioxide group, respectively, at least one of the carbons forming the ring of each group substituted with nitrogen.

A substituent(s) of the substituted C₅-C₃₀ carbocyclic group, the substituted C₂-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₁-C₆₀ alkylthio group, the substituted C₃-C₁₀ cycloalkyl group, the substituted C₁-C₁₀ heterocycloalkyl group, the substituted C₃-C₁₀ cycloalkenyl group, the substituted C₂-C₁₀ heterocycloalkenyl group, the substituted C₆-C₆₀ aryl group, the substituted C₇-C₆₀ alkyl aryl group, the substituted C₆-C₆₀ aryloxy group, the substituted C₆-C₆₀ arylthio group, the substituted C₁-C₆₀ heteroaryl group, the substituted C₂-C₆₀ alkyl heteroaryl group, the substituted monovalent non-aromatic condensed polycyclic group, and the substituted monovalent non-aromatic condensed heteropolycyclic group may each independently be:
deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group or C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, - CD₂H, -CDH₂, -CF₃, -CF₂H, -CFH₂, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₇-C₆₀ alkyl aryl group, a C₆-C₆₀ aryloxy group, a C₆-C₆₀ arylthio group, a C₁-C₆₀ heteroaryl group, a C₂-C₆₀ alkyl heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), - B(Q₁₆)(Q₁₇), -P(=O)(Q₁₈)(Q₁₉), -P(Q₁₈)(Q₁₉), or any combination thereof ;
a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₇-C₆₀ alkyl aryl group, a C₆-C₆₀ aryloxy group, a C₆-C₆₀ arylthio group, a C₁-C₆₀ heteroaryl group, a C₂-C₆₀ alkyl heteroaryl group, a monovalent non-aromatic condensed polycyclic group, or a monovalent non-aromatic condensed heteropolycyclic group, each unsubstituted or substituted with deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₇-C₆₀ alkyl aryl group, a C₆-C₆₀ aryloxy group, a C₆-C₆₀ arylthio group, a C₁-C₆₀ heteroaryl group, a C₂-C₆₀ alkyl heteroaryl group, a monovalent non-aromatic condensed polycyclic group, a monovalent non-aromatic condensed heteropolycyclic group, - N(Q₂₁)(Q₂₂), -Si(Q₂₃)(Q₂₄)(Q₂₅), -B(Q₂₆)(Q₂₇), -P(=O)(Q₂₈)(Q₂₉), -P(Q₂₈)(Q₂₉), or any combination thereof ;
-N(Q₃₁)(Q₃₂), -Si(Q₃₃)(Q₃₄)(Q₃₅), -B(Q₃₆)(Q₃₇), -P(=O)(Q₃₈)(Q₃₉), or - P(Q₃₈)(Q₃₉); or
any combination thereof.

As used herein, Q₁ to Q₉, Q₁₁ to Q₁₉, Q₂₁ to Q₂₉, and Q₃₁ to Q₃₉ may each independently be hydrogen; deuterium; -F; -Cl; -Br; -I; a hydroxyl group; a cyano group; a nitro group; an amidino group; a hydrazine group; a hydrazone group; a carboxylic acid group or a salt thereof; a sulfonic acid group or a salt thereof; a phosphoric acid group or a salt thereof; a C₁-C₆₀ alkyl group unsubstituted or substituted with deuterium, a C₁-C₆₀ alkyl group, a C₆-C₆₀ aryl group, or any combination thereof; a C₂-C₆₀ alkenyl group; a C₂-C₆₀ alkynyl group; a C₁-C₆₀ alkoxy group; a C₃-C₁₀ cycloalkyl group; a C₁-C₁₀ heterocycloalkyl group; a C₃-C₁₀ cycloalkenyl group; a C₂-C₁₀ heterocycloalkenyl group; a C₆-C₆₀ aryl group unsubstituted or substituted with deuterium, a C₁-C₆₀ alkyl group, a C₆-C₆₀ aryl group, or any combination thereof; a C₆-C₆₀ aryloxy group; a C₆-C₆₀ arylthio group; a C₁-C₆₀ heteroaryl group; a monovalent non-aromatic condensed polycyclic group; or a monovalent non-aromatic condensed heteropolycyclic group.

Hereinafter, compounds and organic light-emitting devices according to embodiments will now be described in detail with reference to synthesis examples and examples. However, these examples are only for illustrative purposes and are not intended to limit the scope of the one or more embodiments of the present disclosure. The wording "B was used instead of A" used in describing synthesis examples means that the amount of A used was identical to the amount of B used, in terms of a molar equivalent.

### Examples

### Synthesis Example 1 (Compound 2)

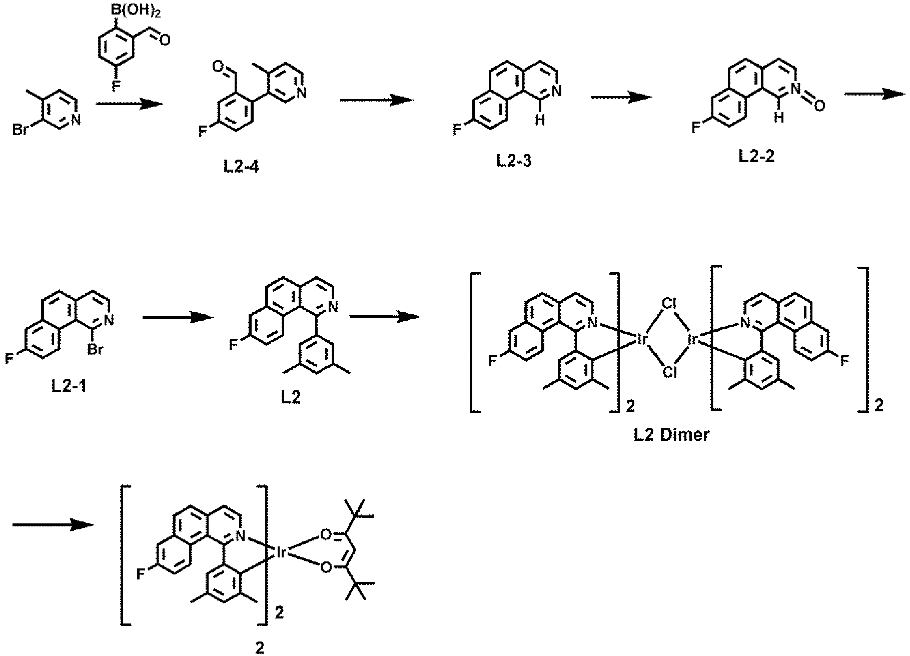

### Synthesis of Compound L2-4

5 g (29.1mmol) of 3-bromo-4-methylpyridine was mixed with 60 mL of acetonitrile and 15 mL of water, and then 1.43 g (2.04 mmol) of PdCl₂(PPh₃)₂, 4.88 g (29.1mmol) of (4-fluoro-2-formylphenyl)boronic acid, and 10.04 g (72.8mmol) of K₂CO₃ were added thereto and heated under reflux at 80°C for 18 hours. After the reaction was completed, the reaction mixture was concentrated under reduced pressure and then extracted with dichloromethane and water to obtain an organic phase. The obtained organic phase was dried with magnesium sulfate, distilled under reduced pressure, and then purified by liquid chromatography to obtain 5.13 g of Compound L2-4 (Yield: 82%).
HRMS(MALDI) calcd for C₁₃H₁₀FNO: m/z 215.0746, Found: 215.0747

### Synthesis of Compound L2-3

5.13 g (23.8mmol) of Compound L2-4 was mixed with 70 mL of dimethylformamide (DMF). After a solution of 3.49g (47.6mmol) of t-BuOK dissolved in 10 mL of DMF was slowly dropwise added to the mixture and then stirred at room temperature for 24 h. After the reaction was completed, the reaction mixture was concentrated under reduced pressure and then extracted with dichloromethane and water to obtain an organic phase. The obtained organic phase was dried with magnesium sulfate, distilled under reduced pressure, and then purified by liquid chromatography to obtain 3.57 g of Compound L2-3 (Yield: 76%).
HRMS(MALDI) calcd for C₁₃H₈FN: m/z 197.0641, Found: 197.0642

### Synthesis of Compound L2-2

After 3.57g (18.1mmol) of Compound L2-3 was dissolved in 60 mL of CH₂Cl₂, meta-cholorperoxybenzoic acid (mCPBA) was slowly dropwise added at 0°C thereto and then stirred for 24 h. After the reaction was completed, the reaction mixture was extracted with 6N KOH to obtain an organic phase. The obtained organic phase was dried using magnesium sulfate, distilled under reduced pressure, and then purified by liquid chromatography to obtain 3.67 g of Compound L2-2 (Yield: 95%).

### Synthesis of Compound L2-1

After 3.67 g (17.2mmol) of Compound L2-2 was mixed with 80 mL of CH₂Cl₂, 5.68 g (19.8 mmol) of POBr₃ was slowly dropwise added at 0°C thereto, and then 0.3 mL of DMF was added thereto and stirred at room temperature for 24 h. After the reaction was completed, the reaction mixture was extracted with saturated sodium bicarbonate to obtain an organic phase. The obtained organic phase was dried using magnesium sulfate, distilled under reduced pressure, and then purified by liquid chromatography to obtain 3.23 g of Compound L2-1 (Yield: 68%).
HRMS(MALDI) calcd for C₁₃H₇BrFN: m/z 274.9746, Found: 274.9747

### Synthesis of Compound L2

After 3.23 g (11.7 mmol) of Compound L2-1 was mixed with 45 mL of THF and 15 mL of water, 1.93 g (12.9 mmol) of 3,5-dimethylphenylboronic acid, 1.23 g (0.82 mmol) of Pd(PPh₃)₄, and 4.04 g (29.3 mmol) of K₂CO₃ were added thereto and then heated under reflux at 75°C for 24 h. After the reaction was completed, the reaction mixture was extracted with ethyl acetate and water to obtain an organic phase. The obtained organic phase was dried using magnesium sulfate, distilled under reduced pressure, and then purified by liquid chromatography to obtain 2.61 g of Compound L2 (Yield: 74%).
HRMS(MALDI) calcd for C₂₁H₁₆FN: m/z 301.1267, Found: 301.1268

### Synthesis of Compound L2 Dimer

2.00 g (6.6 mmol) of Compound L2 and 1.11 g (3.1 mmol) of iridium chloride were mixed with 40 mL of ethoxyethanol and 15 mL of distilled water, and then heated under reflux for 24 hours. After the reaction was completed, the temperature was lowered to room temperature to obtain a solid product. This solid product was filtered and then washed sufficiently with water, methanol, and then hexane. The obtained solid was dried in a vacuum oven to obtain 2.4 g of Compound L2 Dimer.

### Synthesis of Compound 2

2.4 g (1.45 mmol) of Compound L2 Dimer, 1.33 g (7.25 mmol) of 2,2,6,6-tetramethylheptane-3,5-dione, and 0.76 g (7.25 mmol) of Na₂CO₃ were mixed with 40 mL of ethoxyethanol, and then stirred at 90°C for 24 hours to allow reaction. After the reaction was completed, the temperature was lowered to room temperature to obtain a solid product. The solid product produced by lowering the temperature to room temperature was filtered and then purified by liquid chromatography to obtain 1.56 g of Compound 2 (Yield: 55%).
HRMS(MALDI) calcd for C₈₃H₄₉F₂FIrN₂O₂: m/z 976.3391, Found: 976.3393

### Synthesis Example 2 (Compound 10)

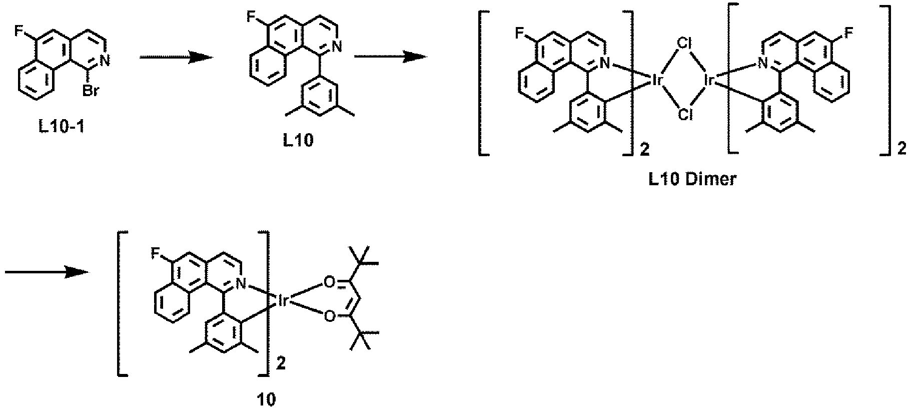

### Synthesis of Compound L10

1.50 g of Compound L10 (Yield: 76%) was obtained in the same manner as in the synthesis method of Compound L2 of Synthesis Example 1, except that Compound L10-1(1-bromo-6-fluorobenzo[h]isoquinoline) was used instead of Compound L2-1.
HRMS(MALDI) calcd for C₂₁H₁₆FN : m/z 301.3644, Found: 301.3646

### Synthesis of Compound L10 Dimer

1.82 g of Compound L10 Dimer was obtained in the same manner as in the synthesis method of Compound L2 Dimer of Synthesis Example 1, except that Compound L10 was used instead of Compound L2.

### Synthesis of Compound 10

1.10 g of Compound 10 (Yield: 52.0 %) was obtained in the same manner as in the synthesis method of Compound 2 of Synthesis Example 1, except that Compound L10 Dimer was used instead of Compound L2 Dimer.
HRMS(MALDI) calcd for C₅₃H₄₉F₂IrN₂O₂ : m/z 976.2008, Found: 976.2009

### Synthesis Example 3 (Compound 11)

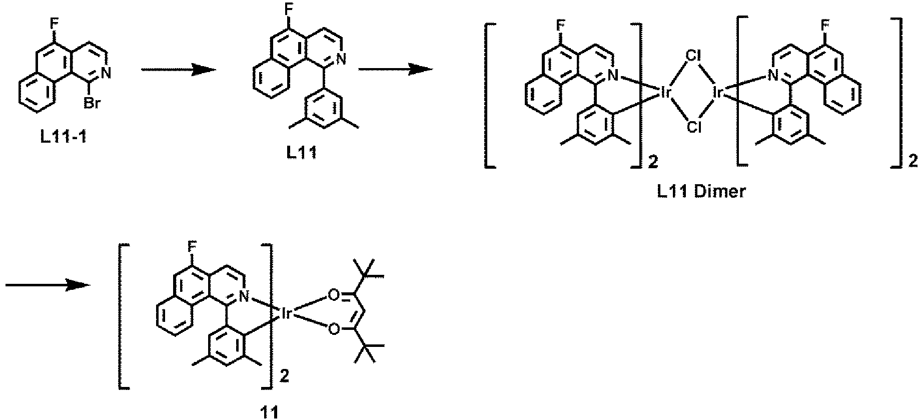

### Synthesis of Compound L11

1.42 g of Compound L11 (Yield: 77%) was obtained in the same manner as in the synthesis method of Compound L2 of Synthesis Example 1, except that Compound L11-1 (1-bromo-7-fluorobenzo[h]isoquinoline) was used instead of Compound L2-1.
HRMS(MALDI) calcd for C₂₁H₁₆FN : m/z 301.3644, Found: 301.3645

### Synthesis of Compound L11 Dimer

1.70 g of Compound 11 Dimer was obtained in the same manner as in the synthesis method of Compound L2 Dimer of Synthesis Example 1, except that Compound L11 was used instead of Compound L2.

### Synthesis of Compound 11

0.98 g of Compound 11 (Yield: 49.0 %) was obtained in the same manner as in the synthesis method of Compound 2 of Synthesis Example 1, except that Compound L11 Dimer was used instead of Compound L2 Dimer.
HRMS(MALDI) calcd for C₅₃H₄₉F₂IrN₂O₂ : m/z 976.2008, Found: 976.2008

### Synthesis Example 4 (Compound 22)

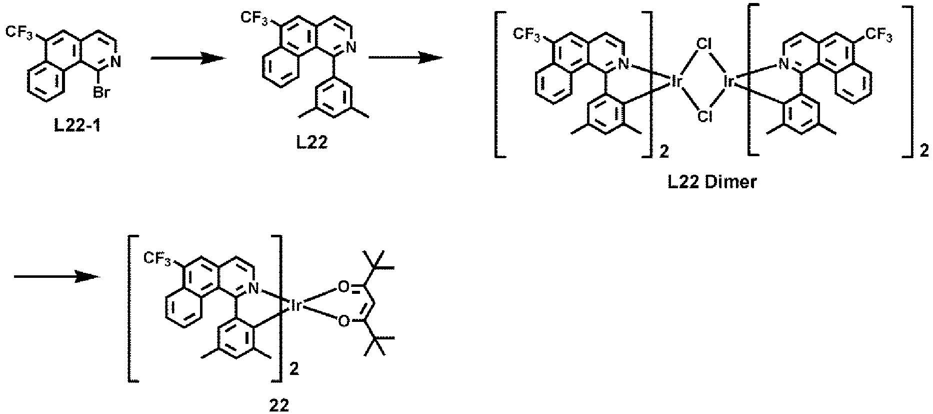

### Synthesis of Compound L22

1.65 g of Compound L22 (Yield: 64.0 %) was obtained in the same manner as in the synthesis method of Compound L2 of Synthesis Example 1, except that Compound L22-1 was used instead of Compound L2-1.
HRMS(MALDI) calcd for C₂₂H₁₆F₃N : m/z 351.3722, Found: 351.3724

### Synthesis of Compound L22 Dimer

1.62 g of Compound L22 Dimer was obtained in the same manner as in the synthesis method of Compound L2 Dimer of Synthesis Example 1, except that Compound L22 was used instead of Compound L2.

### Synthesis of Compound 22

0.71 g of Compound 22 (Yield: 40%) was obtained in the same manner as in the synthesis method of Compound 2 of Synthesis Example 1, except that Compound L22 Dimer was used instead of Compound L2 Dimer.
HRMS(MALDI) calcd for C₅₅H₄₉F₆IrN₂O₂ : m/z 1076.2164, Found: 1076.2166

### Synthesis Example 5 (Compound 41)

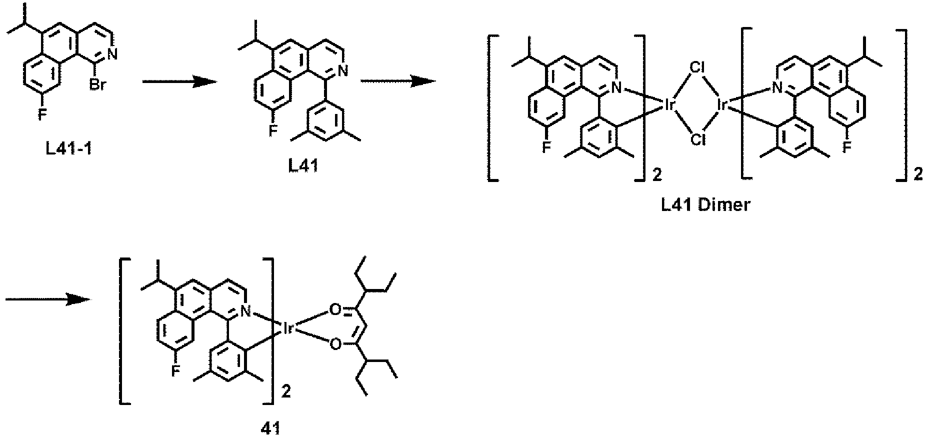

### Synthesis of Compound L41

2.10 g of Compound L41 (Yield: 78%) was obtained in the same manner as in the synthesis method of Compound L2 of Synthesis Example 1, except that Compound L41-1 was used instead of Compound L2-1.
HRMS(MALDI) calcd for C₂₄H₂₂FN : m/z 343.4454, Found: 343.445

### Synthesis of Compound L41 Dimer

2.2 g of Compound L41 Dimer was obtained in the same manner as in the synthesis method of Compound L2 Dimer of Synthesis Example 1, except that Compound L41 was used instead of Compound L2.

### Synthesis of Compound 41

1.44 g of Compound 41 (Yield: 55.0%) was obtained in the same manner as in the synthesis method of Compound 2 of Synthesis Example 1, except that Compound L41 Dimer and 3,7-diethylnonane-4,6-dione were used instead of Compound L2 Dimer and 2,2,6,6-tetramethylheptane-3,5-dione, respectively.
HRMS(MALDI) calcd for C₆₁H₆₅F₂IrN₂O₂ : m/z 1088.4168, Found: 1088.4169

### Synthesis Example 6 (Compound 42)

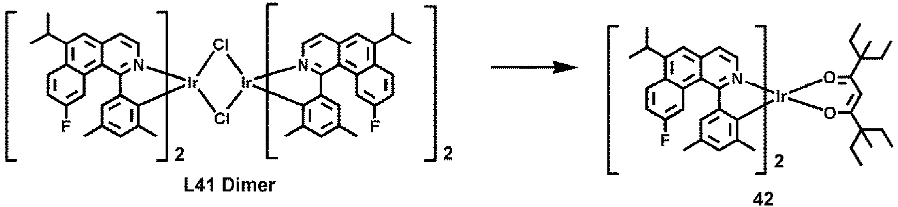

1.58 g of Compound 42 (Yield: 52.0%) was obtained in the same manner as in the synthesis method of Compound 2 of Synthesis Example 1, except that Compound L41 Dimer and 3,7-diethyl-3,7-dimethylnonane-4,6-dione were used instead of Compound L2 Dimer and 2,2,6,6-tetramethylheptane-3,5-dione, respectively.
HRMS(MALDI) calcd for C₆₃H₆₉F₂IrN₂O₂ : m/z 1116.4708, Found: 1116.4709

### Synthesis Example 7 (Compound 56)

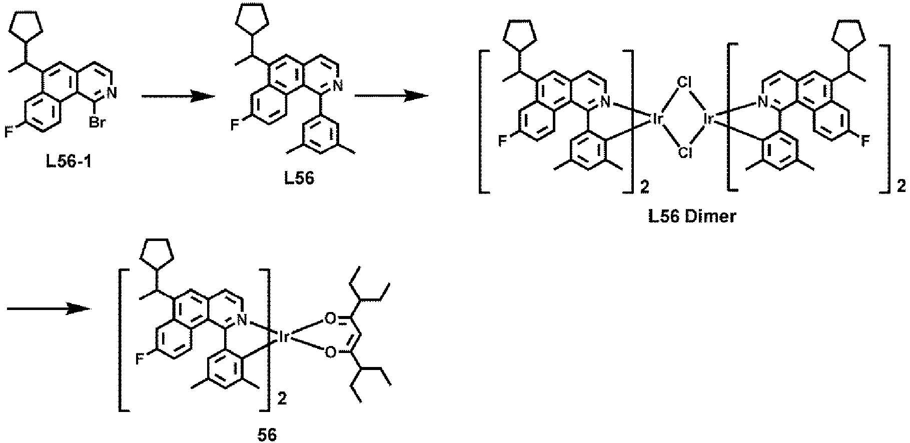

### Synthesis of Compound L56

1.65 g of Compound L56 (Yield: 68.0%) was obtained in the same manner as in the synthesis method of Compound L2 of Synthesis Example 1, except that Compound L56-1 was used instead of Compound L2-1.
HRMS(MALDI) calcd for C₂₈H₂₈FN : m/z 397.5374, Found: 397.5375

### Synthesis of Compound L56 Dimer

1.70 g of Compound L56 Dimer was obtained in the same manner as in the synthesis method of Compound L2 Dimer of Synthesis Example 1, except that Compound L56 was used instead of Compound L2.

### Synthesis of Compound 56

0.78 g of Compound 56 (Yield: 39.0%) was obtained in the same manner as in the synthesis method of Compound 2 of Synthesis Example 1, except that Compound L56 Dimer and 3,7-diethylnonane-4,6-dione were used instead of Compound L2 Dimer and 2,2,6,6-tetramethylheptane-3,5-dione, respectively.
HRMS(MALDI) calcd for C₆₉H₇₇F₂IrN₂O₂ : m/z 1196.6008, Found: 1196.6009

### Synthesis Example 8 (Compound 57)

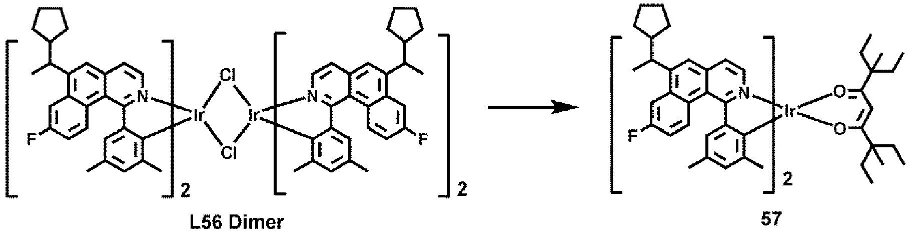

1.00 g of Compound 57 (Yield: 42.0%) was obtained in the same manner as in the synthesis method of Compound 2 of Synthesis Example 1, except that Compound L56 Dimer and 3,7-diethyl-3,7-dimethylnonane-4,6-dione were used instead of Compound L2 Dimer and 2,2,6,6-tetramethylheptane-3,5-dione, respectively.
HRMS(MALDI) calcd for C₇₁H₈₁F₂IrN₂O₂ : m/z 1224.6548, Found: 1224.6550

### Evaluation Example 1: Evaluation of phospholuminescence quantum yield (PLQY)

Compound H52 and Compound 2 in a weight ratio of 98:2 were co-deposited under a vacuum of 1.33×10⁻⁵ Pa (10⁻⁷ torr) to manufacture a film having a thickness of 40 nm.

The photoluminescence quantum yield (PLQY) in film of Compound 2 was evaluated using a Hamamatsu Photonics absolute PL quantum yield measurement system equipped with a xenon light source, a monochromator, a photonic multichannel analyzer), and integrating spheres with PLQY measurement software (Hamamatsu Photonics, Ltd., Shizuoka, Japan). The same evaluation was repeated using Compounds 10, 11, 22, 41, 42, 56 and 57. The results are shown in Table 2.

**Table 2**

| Compound No. | PLQY |
|---|---|
| 2 | 0.946 |
| 10 | 0.931 |
| 11 | 0.935 |
| 22 | 0.942 |
| 41 | 0.9652 |
| 42 | 0.9680 |
| 56 | 0.9752 |
| 57 | 0.9785 |

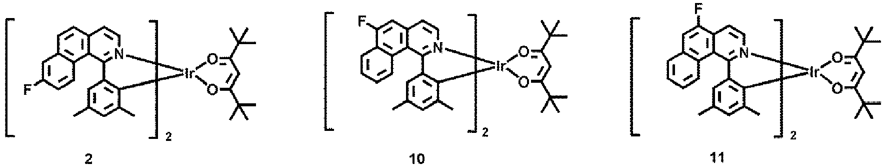

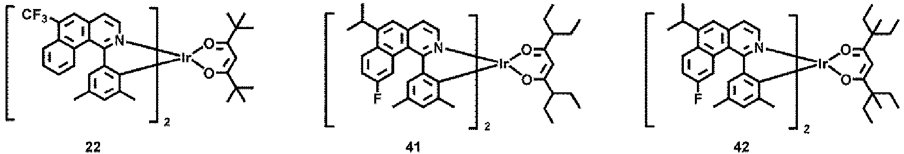

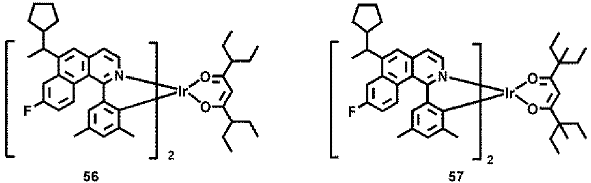

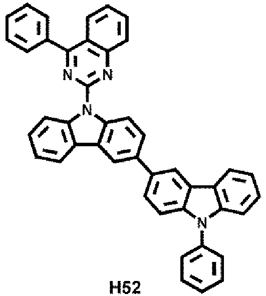

### Example 1

A glass substrate with a 1500 A-thick ITO pattern thereon as an anode was cut to a size of 50 mm x 50 mm x 0.5 mm, ultrasonicated using isopropyl alcohol and pure water for 5 minutes each, and then irradiated with ultraviolet light for 30 minutes and exposed to ozone for cleaning. Then, the resultant glass substrate was loaded onto a vacuum deposition apparatus.

Compound HT3 and F6-TCNNQ were co-deposited under vacuum in a weight ratio of 98:2 on the ITO anode to form a hole injection layer having a thickness of 100 Å, Compound HT3 was vacuum-deposited on the hole injection layer to form a hole transport layer having a thickness of 1,350 Å, and then Compound HT21 was vacuum-deposited on the hole transport layer to form an electron blocking layer having a thickness of 300Å.

Subsequently, Compound H52 (host) and Compound 2 (dopant) were co-deposited in a weight ratio of 98:2 on the electron blocking layer to form an emission layer having a thickness of 400 Å.

Then, Compound ET3 and ET-D1 were co-deposited in a volume ratio of 50:50 on the emission layer to form an electron transport layer having a thickness of 350 Å, ET-D1 was vacuum-deposited on the electron transport layer to form an electron injection layer having a thickness of 10 Å, and Al was vacuum-deposited on the electron injection layer to form a cathode having a thickness of 1,000 Å, thereby manufacturing an organic light-emitting device having a structure of ITO (1500 Å) / HT3+F6-TCNNQ (2 wt%) (100Å) / HT3 (1350Å) / HT21 (300 Å) / H52 + Compound 2(2 wt%) (400Å) / ET3+ET-D1 (50%) (350Å) / ET-D1 (10Å) / Al(1000Å),

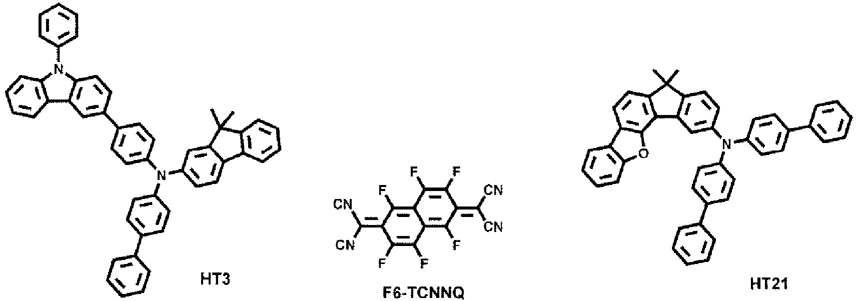

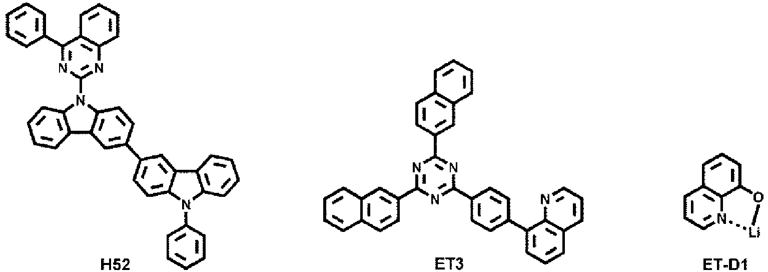

### Examples 2 to 8 and Comparative Examples A to F

Organic light-emitting devices were manufactured in the same manner as in Example 1, except that Compounds shown in Table 3 were used, respectively, instead of Compound 2, as a dopant in forming an emission layer.

### Evaluation Example 2: Characteristics evaluation of organic light-emitting devices

The driving voltage, current density, external quantum efficiency (EQE), roll-off ratio, the full width at half maximum (FWHM) of the emission peak in EL spectra, color coordinate, and lifetime (LT₉₇) of each of were evaluated using the organic light-emitting devices manufactured in Examples 1 to 8 and Comparative Examples A to F. The results are shown in Table 3. This evaluation was performed using a current-voltage meter (Keithley 2400) and a luminescence meter (Minolta Cs-1,000A), and the lifetime (LT₉₇) (at 3500 cd/m² (3500 nit)) was evaluated as the time (hr) it took until the luminance was reduced to 97% with respect to 100% of the initial luminance and represented as a relative value (%) in Table 3. The roll-off ratio was calculated using Equation 1. Roll-off ratio = {1 - (efficiency (at 3500 cd/m2 (3500nit))/maximum luminescence efficiency)} × 100%

**Table 3**

| | Dopant Compound No. | Driving voltage (V) | Current density (mA/cm²) | Max EQE (%) | Roll-Off ratio (%) | FWHM (nm) | Emission color | Color coordinate (CIE) | LT₉₇ (Relative value, %) |
|---|---|---|---|---|---|---|---|---|---|
| Example 1 | 2 | 4.3 | 10 | 26 | 10 | 48 | Red | 0.67, 0.31 | 120 |
| Example 2 | 10 | 4.2 | 10 | 27 | 9 | 50 | Red | 0.68, 0.34 | 120 |
| Example 3 | 11 | 4.0 | 10 | 28 | 8 | 49 | Red | 0.68, 0.33 | 125 |
| Example 4 | 22 | 4.4 | 10 | 26 | 9 | 51 | Red | 0.68, 0.30 | 115 |
| Example 5 | 41 | 4.2 | 10 | 29 | 8 | 51 | Red | 0.68, 0.33 | 120 |
| Example 6 | 42 | 4.3 | 10 | 29 | 9 | 52 | Red | 0.68, 0.32 | 125 |
| Example 7 | 56 | 4.3 | 10 | 30 | 7 | 50 | Red | 0.69, 0.33 | 125 |
| Example 8 | 57 | 4.4 | 10 | 30 | 8 | 51 | Red | 0.69, 0.34 | 125 |
| Comparative Example A | A | 4.5 | 10 | 23 | 10 | 58 | Red | 0.68, 0.32 | 95 |
| Comparative Example B | B | 4.4 | 10 | 25 | 9 | 55 | Red | 0.68, 0.31 | 100 |
| Comparative Example C | C | 5.2 | 10 | 22 | 12 | 58 | Red | 0.69, 0.30 | 95 |
| Comparative Example D | D | 4.7 | 10 | 22 | 17 | 78 | Red | 0.69, 0.33 | 80 |
| Comparative Example E | E | 5.42 | 10 | 23.4 | 24 | 60 | Red | 0.69, 0.29 | 90 |
| Comparative Example F | F | 5.50 | 10 | 24.1 | 19 | 60 | Red | 0.69, 0.29 | 90 |

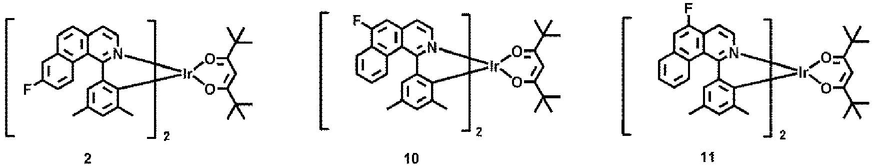

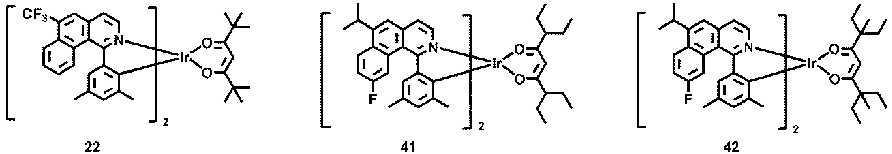

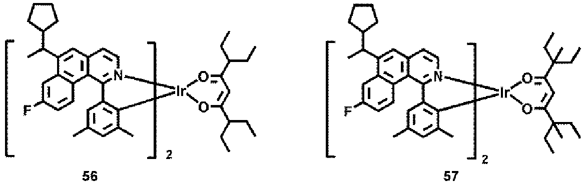

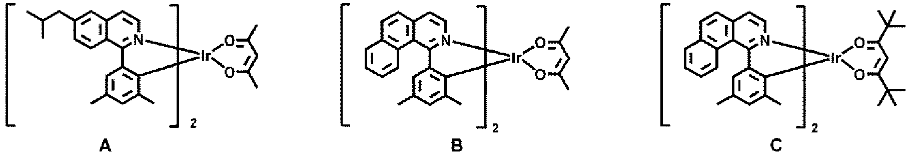

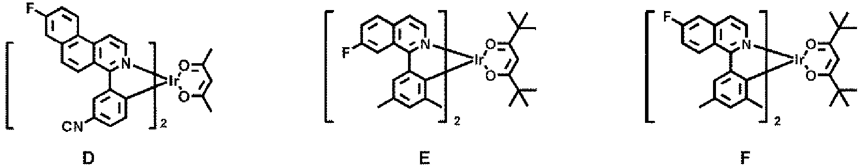

Referring to Table 3, the organic light-emitting devices of Examples 1 to 8 were found to emit red light of high color purity, and have improved driving voltage, improved EQE, improved roll-off ratio, relatively small FWHM, and improved lifetime characteristics, as compared with the organic light-emitting devices of Comparative Examples A to F.

According to the one or more embodiments, the organometallic compound represented by Formula 1 has excellent electronic characteristics and stability, and thus an electronic device, for example, an organic light-emitting device, including the organometallic compound may have improved driving voltage, improved external quantum efficiency, improved roll-off ratio, relatively small FWHM, and improved lifetime characteristics. Accordingly, a high-quality organic light-emitting device or electronic apparatus may be implemented using the organometallic compound.

**It** should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the scope of the disclosure as defined by the following claims.

## Claims

1. An organometallic compound represented by Formula 1, 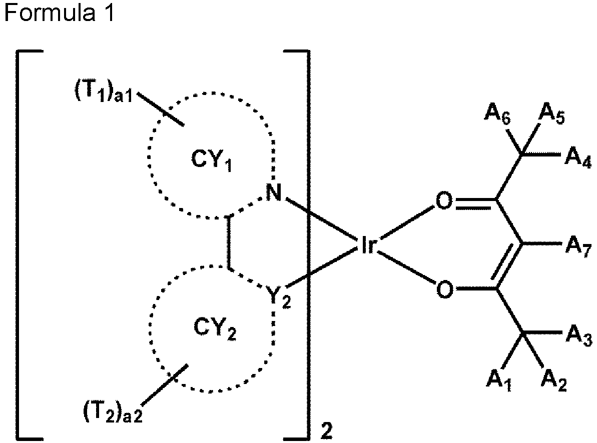 wherein, in Formula 1,
Y₂ is C,
ring CY₁ is a C₅-C₃₀ polycyclic group in which three or more monocyclic groups are condensed to one another, wherein the group represented by 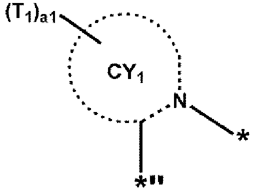 in Formula 1 is one of the groups represented by Formulae CY1-1, and CY1-7 to CY1-9: 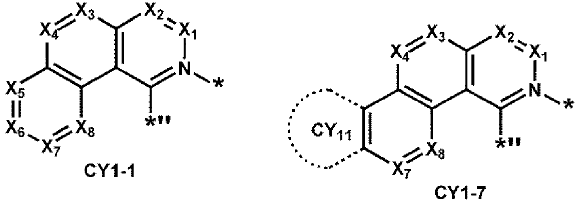 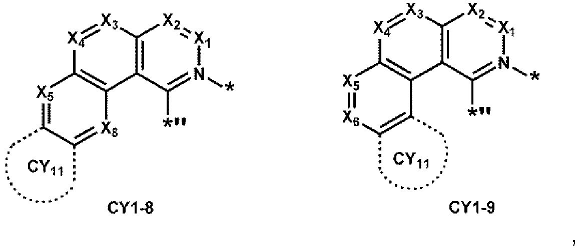
* is a binding site to iridium (Ir) in Formula 1, and *" is a binding site to the ring CY₂ in Formula 1,
ring CY₂ is a C₅-C₃₀ carbocyclic group or a C₁-C₃₀ heterocyclic group,
X₁ to X₈ are each independently C or N, wherein the case where all of X₁ to X₈ are N is excluded,
ring CY₁₁ is a cyclopentane group, a cyclopentene group, a cyclohexane group, a cyclohexene group, an adamantane group, a norbornane group, a norbornene group, a benzene group, a pyridine group, a pyrimidine group, a pyrazine group, a pyridazine group, a naphthalene group, a quinoline group, an isoquinoline group, or a quinoxaline group,
T₁ and A₁ to A₇ are each independently hydrogen, deuterium, -F, -Cl, -Br, -I, - SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), - Ge(Q₃)(Q₄)(Q₅), -B(Q₆)(Q₇), -P(=O)(Q₈)(Q₉), or -P(Q₈)(Q₉),
T₂ is deuterium, -Cl, -Br, -I, -SF₅, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or - P(Q₈)(Q₉),
a1 is an integer of 1 to 20; when a1 is 2 or greater, two or more T₁(s) are identical to or different from each other; and at least one of T₁(s) in number of a1 comprises at least one -F,
two or more of T₁(s) in number of a1 are optionally linked to one another to form a C₅-C₃₀ carbocyclic group unsubstituted or substituted with at least one R₁ₐ, or a C₁-C₃₀ heterocyclic group unsubstituted or substituted with at least one R₁ₐ,
two or more of T₂(s) in number of a2 are optionally linked to one another to form a C₅-C₃₀ carbocyclic group unsubstituted or substituted with at least one R₁ₐ, or a C₁-C₃₀ heterocyclic group unsubstituted or substituted with at least one R₁ₐ,
T₁ and T₂ are optionally linked to one another to form a C₅-C₃₀ carbocyclic group unsubstituted or substituted with at least one R₁ₐ, or a C₁-C₃₀ heterocyclic group unsubstituted or substituted with at least one R₁ₐ,
two or more of A1 to A₇ are optionally linked to one another to form a C₅-C₃₀ carbocyclic group unsubstituted or substituted with at least one R₁ₐ, or a C₁-C₃₀ heterocyclic group unsubstituted or substituted with at least one R1a.
R₁ₐ is the same as defined in connection with A₇,
a substituent(s) of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₁-C₆₀ alkylthio group, the substituted C₃-C₁₀ cycloalkyl group, the substituted C₁-C₁₀ heterocycloalkyl group, the substituted C₃-C₁₀ cycloalkenyl group, the substituted C₂-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group are each independently:
deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, - N(Q₁₁)(Q₁₂), -Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉), - P(Q₁₈)(Q₁₉), or any combination thereof ;
a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), -Ge(Q₂₃)(Q₂₄)(Q₂₅), - B(Q₂₆)(Q₂₇), -P(=O)(Q₂₈)(Q₂₉), -P(Q₂₈)(Q₂₉), or any combination thereof;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), - P(=O)(Q₃₈)(Q₃₉), or -P(Q₃₈)(Q₃₉); or
any combination thereof,
Q₁ to Q₉, Q₁₁ to Q₁₉, Q₂₁ to Q₂₉ and Q₃₁ to Q₃₉ are each independently:
hydrogen; deuterium; -F; -Cl; -Br; -I; a hydroxyl group; a cyano group; a nitro group; an amidino group; a hydrazine group; a hydrazone group; a carboxylic acid group or a salt thereof; a sulfonic acid group or a salt thereof; a phosphoric acid group or a salt thereof; a C₁-C₆₀ alkyl group unsubstituted or substituted with deuterium, a C₁-C₆₀ alkyl group, a C₆-C₆₀ aryl group, or any combination thereof; a C₂-C₆₀ alkenyl group; a C₂-C₆₀ alkynyl group; a C₁-C₆₀ alkoxy group; a C₃-C₁₀ cycloalkyl group; a C₁-C₁₀ heterocycloalkyl group; a C₃-C₁₀ cycloalkenyl group; a C₂-C₁₀ heterocycloalkenyl group; a C₆-C₆₀ aryl group unsubstituted or substituted with deuterium, a C₁-C₆₀ alkyl group, a C₆-C₆₀ aryl group, or any combination thereof; a C₆-C₆₀ aryloxy group; a C₆-C₆₀ arylthio group; a C₁-C₆₀ heteroaryl group; a monovalent non-aromatic condensed polycyclic group; or a monovalent non-aromatic condensed heteropolycyclic group,
wherein a2 is 1, 2 or 3 and when a2 is 2 or greater, two or more T₂(s) are identical to or different from each other, and
wherein in Formula 1, the following case is excluded: 1) A₇ is hydrogen, and 2) both the group represented by *-C(A₁)(A₂)(A₃) and the group represented by *-C(A₄)(A₅)(A₆) are a methyl group.

2. The organometallic compound of claim 1, wherein the ring CY₂ is a benzene group, a naphthalene group, a 1,2,3,4-tetrahydronaphthalene group, a thiophene group, a furan group, a pyrrole group, a cyclopentadiene group, a silole group, a benzothiophene group, a benzofuran group, an indole group, an indene group, a benzosilole group, a dibenzothiophene group, a dibenzofuran group, a carbazole group, a fluorene group, or a dibenzosilole group; and/or
wherein T₁ and A₁ to A₇ are each independently hydrogen, deuterium, -F, a substituted or unsubstituted C₁-C₂₀ alkyl group, a substituted or unsubstituted C₃-C₁₀ cycloalkyl group, a substituted or unsubstituted C₁-C₁₀ heterocycloalkyl group, a substituted or unsubstituted a phenyl group, a substituted or unsubstituted a biphenyl group, a substituted or unsubstituted a terphenyl group, -Si(Q₃)(Q₄)(Q₅), or - Ge(Q₃)(Q₄)(Q₆);
wherein T₂ is deuterium, a substituted or unsubstituted C₁-C₂₀ alkyl group, a substituted or unsubstituted C₃-C₁₀ cycloalkyl group, a substituted or unsubstituted C₁-C₁₀ heterocycloalkyl group, a substituted or unsubstituted a phenyl group, a substituted or unsubstituted a biphenyl group, a substituted or unsubstituted a terphenyl group, -Si(Q₃)(Q₄)(Q₅), or -Ge(Q₃)(Q₄)(Q₅);
wherein at least one of T₁(s) in number of a1 comprises at least one -F; and
wherein in Formula 1, the following case is excluded: 1) A₇ is hydrogen, and 2) both the group represented by *-C(A₁)(A₂)(A₃) and the group represented by *-C(A₄)(A₅)(A₆) are a methyl group.

3. The organometallic compound of claims 1 or 2, wherein T₁, and A₁ to A₇ are each independently:
hydrogen, deuterium, -F, C₁-C₂₀ alkyl group, a C₃-C₁₀ cycloalkyl group, a C₁-C₁₀ heterocycloalkyl group, a phenyl group, a biphenyl group, or a terphenyl group;
a fluorinated C₁-C₂₀ alkyl group, a fluorinated C₃-C₁₀ cycloalkyl group, a fluorinated C₁-C₁₀ heterocycloalkyl group, a fluorinated phenyl group, a fluorinated biphenyl group, or a fluorinated terphenyl group, each unsubstituted or substituted with deuterium, C₁-C₂₀ alkyl group, a C₃-C₁₀ cycloalkyl group, a C₁-C₁₀ heterocycloalkyl group, a phenyl group, a biphenyl group, a terphenyl group, or any combination thereof;
a deuterated C₁-C₂₀ alkyl group, a deuterated C₃-C₁₀ cycloalkyl group, a deuterated C₁-C₁₀ heterocycloalkyl group, a deuterated phenyl group, a deuterated biphenyl group, or a deuterated terphenyl group, each unsubstituted or substituted with -F, C₁-C₂₀ alkyl group, a C₃-C₁₀ cycloalkyl group, a C₁-C₁₀ heterocycloalkyl group, a phenyl group, a biphenyl group, a terphenyl group, or any combination thereof; or
-Si(Q₃)(Q₄)(Q₅), or -Ge(Q₃)(Q₄)(Q₆);
wherein at least one of T₁(s) in number of a1 comprises at least one -F; and
wherein in Formula 1, the following case is excluded: 1) A₇ is hydrogen, and 2) both the group represented by *-C(A₁)(A₂)(A₃) and the group represented by *-C(A₄)(A₅)(A₆) are a methyl group.

4. The organometallic compound of any of claims 1-3, wherein A₁ to A₇ are each independently:
hydrogen, deuterium, C₁-C₂₀ alkyl group, a C₃-C₁₀ cycloalkyl group, a C₁-C₁₀ heterocycloalkyl group, a phenyl group, a biphenyl group, or a terphenyl group;
a deuterated C₁-C₂₀ alkyl group, a deuterated C₃-C₁₀ cycloalkyl group, a deuterated C₁-C₁₀ heterocycloalkyl group, a deuterated phenyl group, a deuterated biphenyl group, or a deuterated terphenyl group, each unsubstituted or substituted with a C₁-C₂₀ alkyl group, a C₃-C₁₀ cycloalkyl group, a C₁-C₁₀ heterocycloalkyl group, a phenyl group, a biphenyl group, a terphenyl group, or any combination thereof; or
-Si(Q₃)(Q₄)(Q₅), or -Ge(Q₃)(Q₄)(Q₆);
wherein T₂ is:
deuterium, C₁-C₂₀ alkyl group, a C₃-C₁₀ cycloalkyl group, a C₁-C₁₀ heterocycloalkyl group, a phenyl group, a biphenyl group, or a terphenyl group;
a deuterated C₁-C₂₀ alkyl group, a deuterated C₃-C₁₀ cycloalkyl group, a deuterated C₁-C₁₀ heterocycloalkyl group, a deuterated phenyl group, a deuterated biphenyl group, or a deuterated terphenyl group, each unsubstituted or substituted with a C₁-C₂₀ alkyl group, a C₃-C₁₀ cycloalkyl group, a C₁-C₁₀ heterocycloalkyl group, a phenyl group, a biphenyl group, a terphenyl group, or any combination thereof; or
-Si(Q₃)(Q₄)(Q₅), or -Ge(Q₃)(Q₄)(Q₅); and
wherein in Formula 1, the following case is excluded: 1)A₇ is hydrogen, and 2) both the group represented by *-C(A₁)(A₂)(A₃) and the group represented by *-C(A₄)(A₅)(A₆) are a methyl group.

5. The organometallic compound of any of claims 1-4, wherein a group represented by 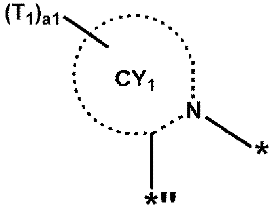 in Formula 1 is represented by Formula CY1(1): 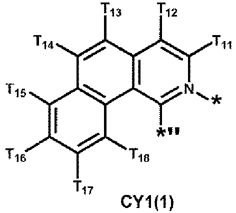 wherein, in Formula CY1(1),
T₁₁ to T₁₈ are the same as defined in connection with T₁ in claim 1, provided that at least one of T₁₁ to T₁₈ comprises at least one fluoro group (-F),
* indicates a binding site to Ir in Formula 1, and
*" indicates a binding site to ring CY₂ in Formula 1.

6. The organometallic compound of any of claims 1-5, wherein a group represented by 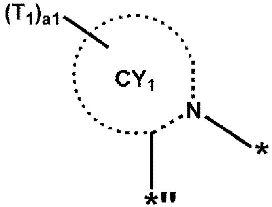 in Formula 1 is one of groups represented by Formulae CY1(1)-1 to CY1(1)-64: 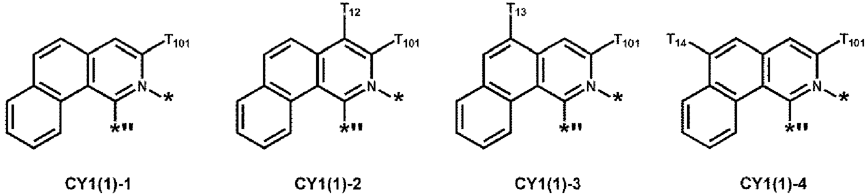 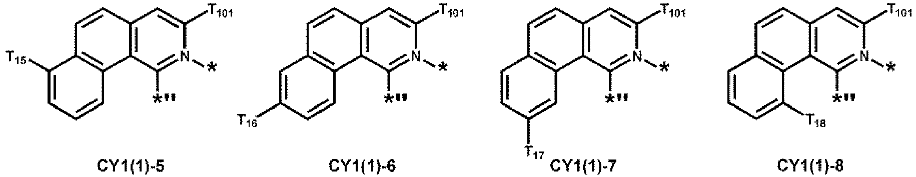 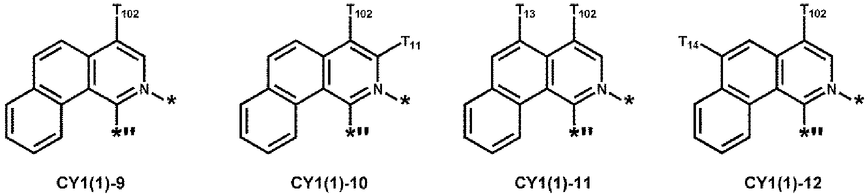 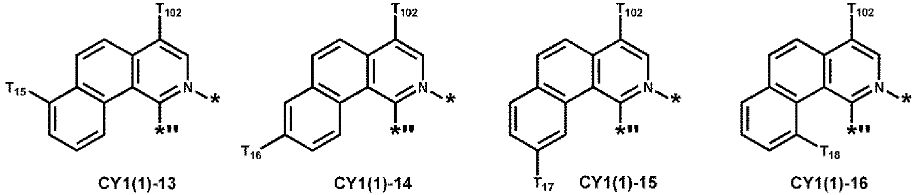 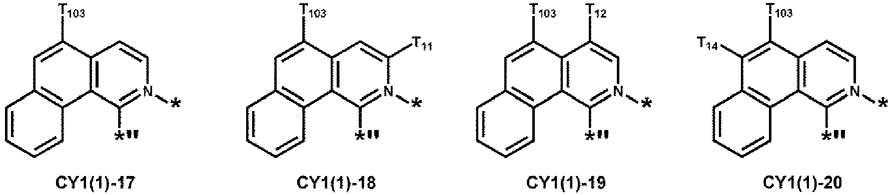 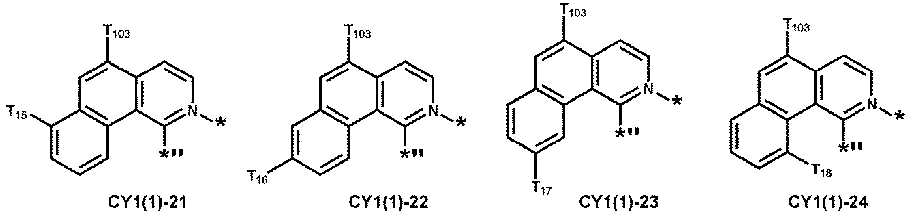 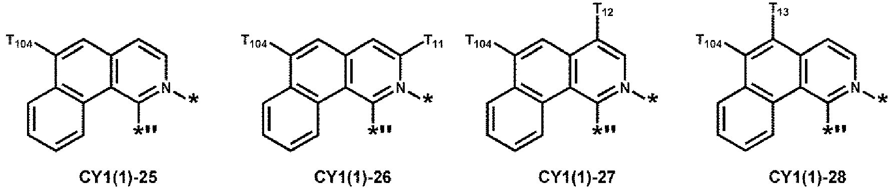 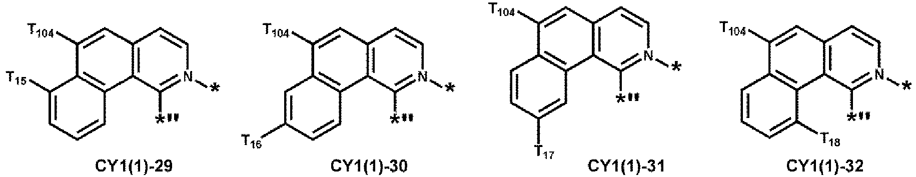 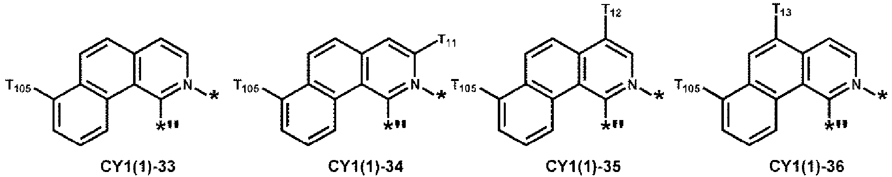 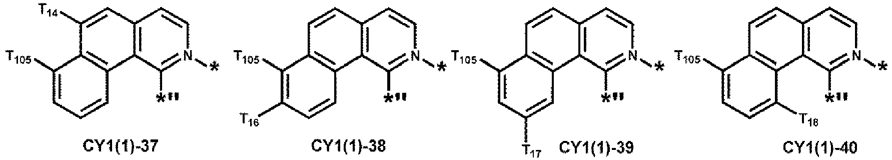 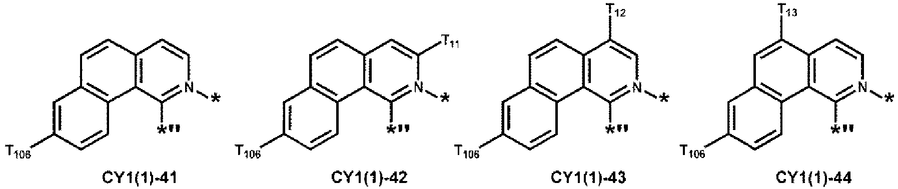 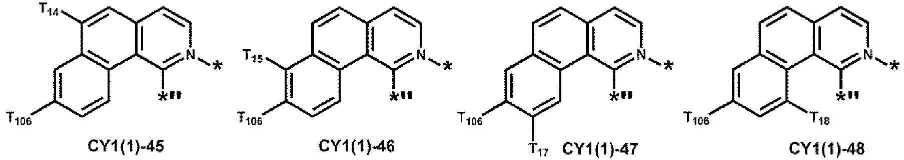 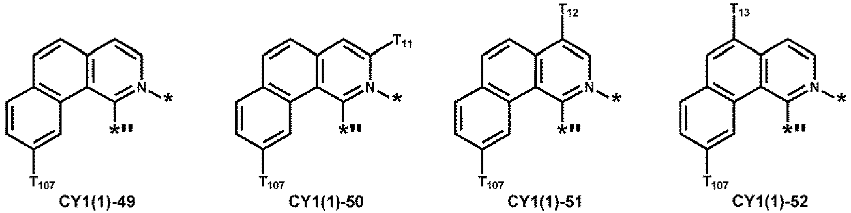 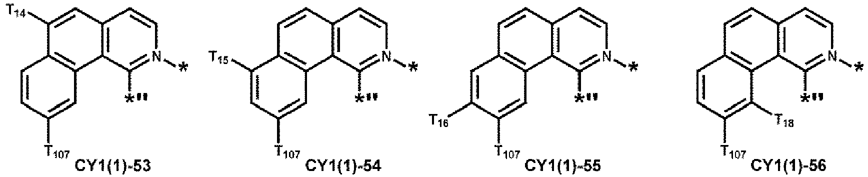 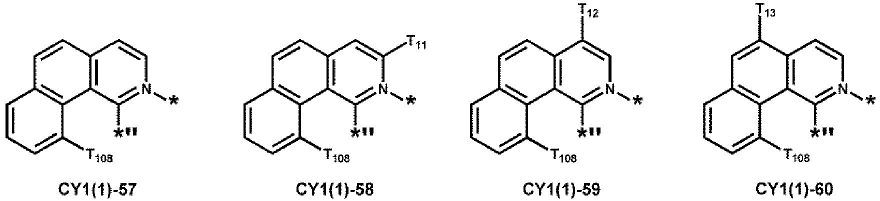 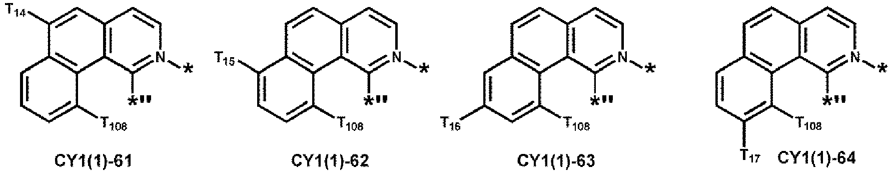 wherein, in Formulae CY1(1)-1 to CY1(1)-64,
T₁₁ to T₁₈ are the same as defined in connection with T₁ in claim 1, provided that each of T₁₁ to T₁₈ is not hydrogen,
T₁₀₁ to T₁₀₈ each comprises at least one fluoro group (-F),
* indicates a binding site to Ir in Formula 1, and,
*" indicates a binding site to ring CY₂ in Formula 1;
preferably wherein, in Formulae CY1(1)-1 to CY1(1)-64,
T₁₁ to T₁₈ are each independently:
deuterium, a C₁-C₂₀ alkyl group, a C₃-C₁₀ cycloalkyl group, a C₁-C₁₀ heterocycloalkyl group, a phenyl group, a biphenyl group, or a terphenyl group;
a deuterated C₁-C₂₀ alkyl group, a deuterated C₃-C₁₀ cycloalkyl group, a deuterated C₁-C₁₀ heterocycloalkyl group, a deuterated phenyl group, a deuterated biphenyl group, or a deuterated terphenyl group, each unsubstituted or substituted with a C₁-C₂₀ alkyl group, a C₃-C₁₀ cycloalkyl group, a C₁-C₁₀ heterocycloalkyl group, a phenyl group, a biphenyl group, a terphenyl group, or any combination thereof; or
-Si(Q₃)(Q₄)(Q₅), or -Ge(Q₃)(Q₄)(Q₅), and
T₁₀₁ to T₁₀₈ are each independently:
-F; or
a fluorinated C₁-C₂₀ alkyl group, a fluorinated C₃-C₁₀ cycloalkyl group, a fluorinated C₁-C₁₀ heterocycloalkyl group, a fluorinated phenyl group, a fluorinated biphenyl group, or a fluorinated terphenyl group, each unsubstituted or substituted with deuterium, a C₁-C₂₀ alkyl group, a C₃-C₁₀ cycloalkyl group, a C₁-C₁₀ heterocycloalkyl group, a phenyl group, a biphenyl group, a terphenyl group, or any combination thereof.

7. The organometallic compound of any of claims 1-6, wherein a group represented by 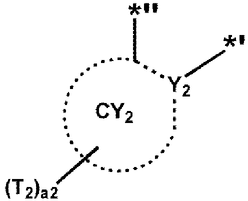 in Formula 1 is one of groups represented by Formulae CY2-1 to CY2-31: 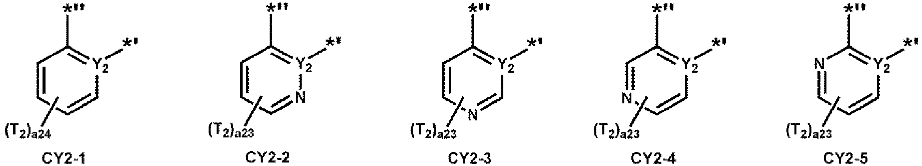 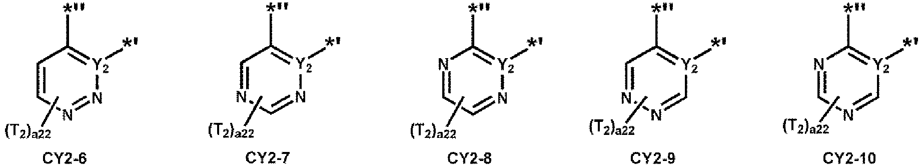 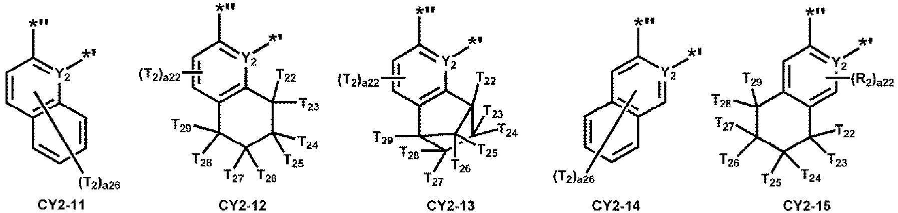 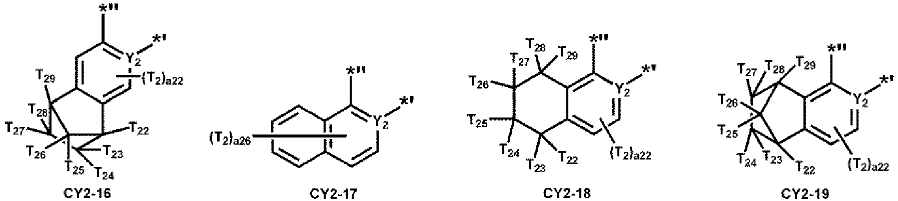 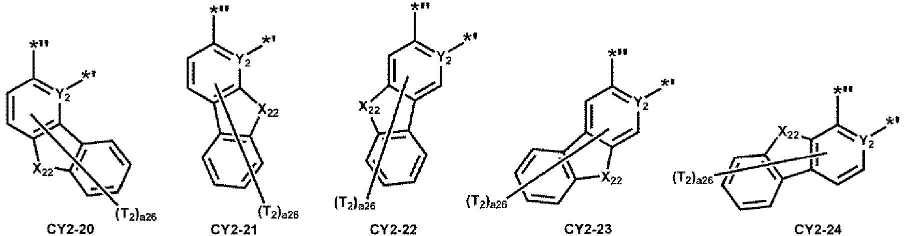 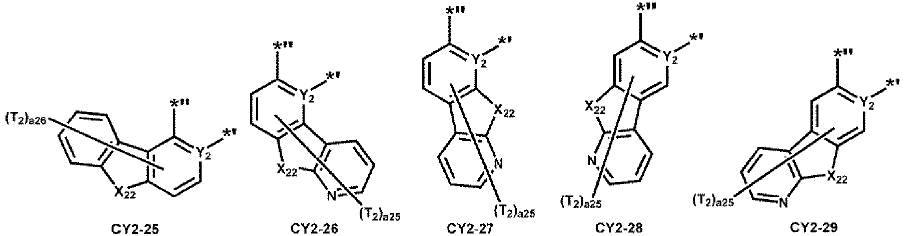 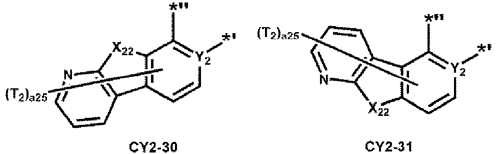 wherein, in Formulae CY2-1 to CY2-31,
Y₂ and T₂ are the same as defined in claim 1,
X₂₂ is C(T₂₈)(T₂₉), N(T₂₈), O, S or Si(T₂₈)(T₂₉),
T_{22 to} T₂₉ are the same as defined in connection with T₂ in claim 1,
a26 is an integer of 1, 2, or 3,
a25 is an integer of 1, 2, or 3,
a24 is an integer of 1, 2, or 3,
a23 is an integer of 1, 2, or 3,
a22 is an integer of 1 or 2,
*" indicates a binding site to ring CY₁ in Formula 1, and
*' indicates a binding site to Ir in Formula 1.

8. The organometallic compound of any of claims 1-7, wherein a group represented by 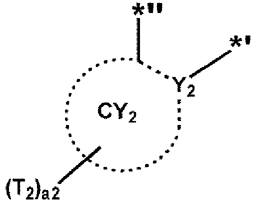 in Formula 1 is one of groups represented by Formulae CY2(2) to CY2(15), CY2(18) to CY2(24), CY2(26) to CY2(32), CY2(34) to CY3(40), CY3(42) to CY3(48), CY2(50) to CY2(52), CY2(55), CY2(56), CY2(64), CY2(65), CY2(67), and CY2(68): 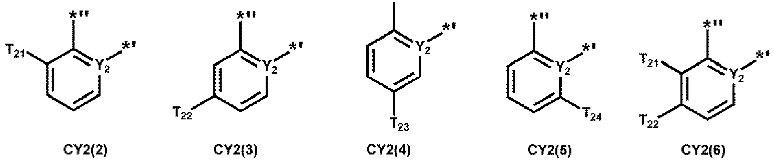 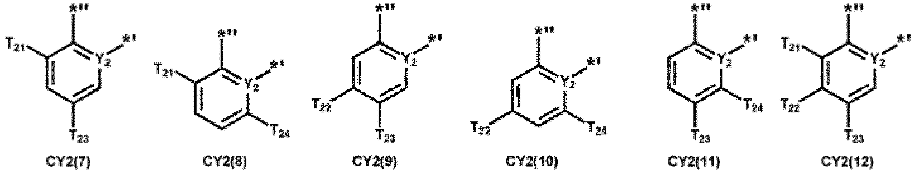 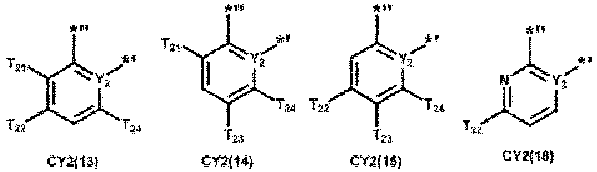 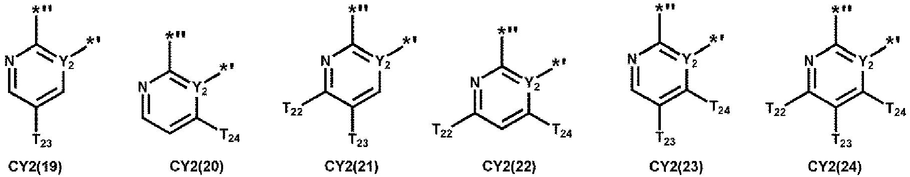 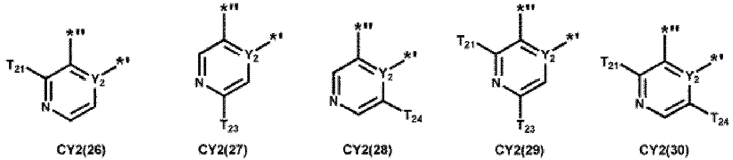 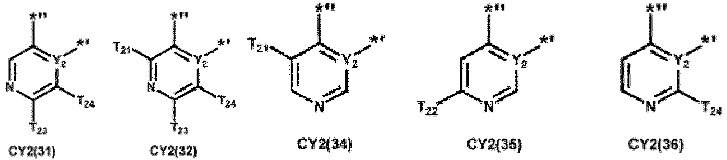 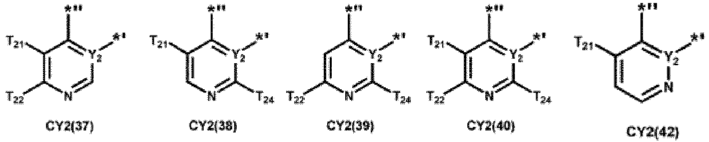 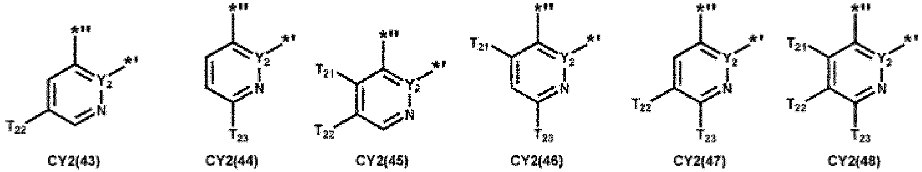 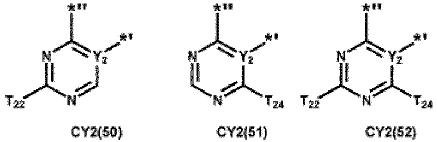 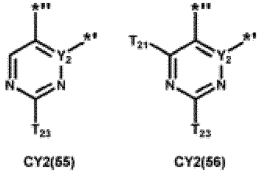 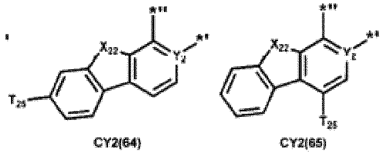 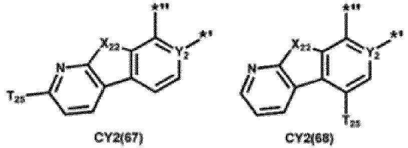
wherein, in Formulae CY2(2) to CY2(15), CY2(18) to CY2(24), CY2(26) to CY2(32), CY2(34) to CY3(40), CY3(42) to CY3(48), CY2(50) to CY2(52), CY2(55), CY2(56), CY2(64), CY2(65), CY2(67), and CY2(68),
Y₂ is the same as defined in claim 1,
X₂₂ is C(T₂₈)(T₂₉), N(T₂₈), O, S, or Si(T₂₈)(T₂₉),
T_{21 to} T₂₅, T₂₈, and T₂₉ are the same as defined in connection with T₂ in claim 1,
*" indicates a binding site to ring CY₁ in Formula 1, and
*' indicates a binding site to Ir in Formula 1.

9. The organometallic compound of any of claims 1-8, wherein the organometallic compound satisfies at least one of Condition 1 to Condition 3:
Condition 1
A₁ to A₆ in Formula 1 are each independently a substituted or unsubstituted C₁-C₆₀ alkyl group, a substituted or unsubstituted C₃-C₁₀ cycloalkyl group, a substituted or unsubstituted C₂-C₁₀ heterocycloalkyl group, a substituted or unsubstituted C₆-C₆₀ aryl group, a substituted or unsubstituted C₂-C₆₀ heteroaryl group, a substituted or unsubstituted monovalent non-aromatic condensed polycyclic group, or a substituted or unsubstituted monovalent non-aromatic condensed heteropolycyclic group;
Condition 2
at least one of A₁ to A₆ in Formula 1 are each independently a substituted or unsubstituted C₂-C₆₀ alkyl group, a substituted or unsubstituted C₃-C₁₀ cycloalkyl group, a substituted or unsubstituted C₂-C₁₀ heterocycloalkyl group, a substituted or unsubstituted C₆-C₆₀ aryl group, a substituted or unsubstituted C₂-C₆₀ heteroaryl group, a substituted or unsubstituted monovalent non-aromatic condensed polycyclic group, or a substituted or unsubstituted monovalent non-aromatic condensed heteropolycyclic group; and
Condition 3
A₇ in Formula 1 is deuterium, a substituted or unsubstituted C₁-C₆₀ alkyl group, a substituted or unsubstituted C₃-C₁₀ cycloalkyl group, a substituted or unsubstituted C₂-C₁₀ heterocycloalkyl group, a substituted or unsubstituted C₆-C₆₀ aryl group, a substituted or unsubstituted C₂-C₆₀ heteroaryl group, a substituted or unsubstituted monovalent non-aromatic condensed polycyclic group, or a substituted or unsubstituted monovalent non-aromatic condensed heteropolycyclic group.

10. The organometallic compound of any of claims 1-9, wherein the organometallic compound satisfies at least one of Condition 4 and Condition 5:
Condition 4
in Formula 1, two or more of A₁ to A₃ of the group represented by *-C(A₁)(A₂)(A₃) are linked with C in the group represented by *-C(A₁)(A₂)(A₃) to form a C₅-C₃₀ carbocyclic group unsubstituted or substituted with at least one R₁ₐ, or a C₁-C₃₀ heterocyclic group unsubstituted or substituted with at least one R₁ₐ so that the group represented by *-C(A₁)(A₂)(A₃) is a C₅-C₃₀ carbocyclic group unsubstituted or substituted with at least one R₁ₐ, or a C₁-C₃₀ heterocyclic group unsubstituted or substituted with at least one R₁ₐ.
Condition 5
in Formula 1, two or more of A₄ to A₆ of the group represented by *-C(A₄)(A₅)(A₆) are linked with C in the group represented by *-C(A₄)(A₅)(A₆) to form a C₅-C₃₀ carbocyclic group unsubstituted or substituted with at least one R₁ₐ, or a C₁-C₃₀ heterocyclic group unsubstituted or substituted with at least one R₁ₐ so that the group represented by *-C(A₄)(A₅)(A₆) is a C₅-C₃₀ carbocyclic group unsubstituted or substituted with at least one R₁ₐ, or a C₁-C₃₀ heterocyclic group unsubstituted or substituted with at least one R₁ₐ.

11. An organic light-emitting device comprising:
a first electrode;
a second electrode; and
an organic layer disposed between the first electrode and the second electrode and comprising an emission layer, the organic layer comprising at least one organometallic compound represented by Formula 1 of any of claims 1-10;
preferably wherein
the first electrode is an anode,
the second electrode is a cathode,
the organic layer further comprises a hole transport region between the first electrode and the emission layer, and an electron transport region between the emission layer and the second electrode,
the hole transport region comprises a hole injection layer, a hole transport layer, an electron blocking layer, a buffer layer or any combination thereof, and
the electron transport region comprises a hole blocking layer, an electron transport layer, an electron injection layer, or any combination thereof.

12. The organic light-emitting device of claim 11, wherein the organometallic compound is in the emission layer,
preferably wherein the emission layer further comprises a host, and an amount of the host in the emission layer is larger than an amount of the organometallic compound in the emission layer.

13. An electronic apparatus comprising the organic light-emitting device of claims 11 or 12.

## Patentansprüche

1. Organometallische Verbindung dargestellt durch Formel 1, 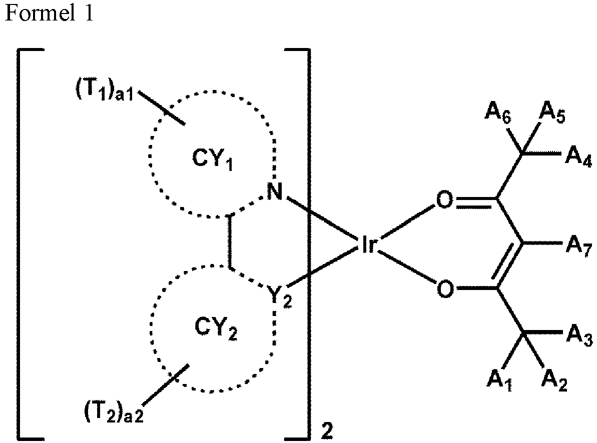 wobei in Formel 1
Y₂ C ist,
Ring CY₁ eine polycyclische C₅-C₃₀-Gruppe ist, in der drei oder mehr monocyclische Gruppen miteinander kondensiert sind, wobei die Gruppe dargestellt durch 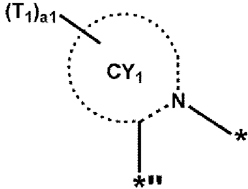 in Formel 1 eine der Gruppen dargestellt durch Formel CY1-1 und CY1-7 bis CY1-9 ist: 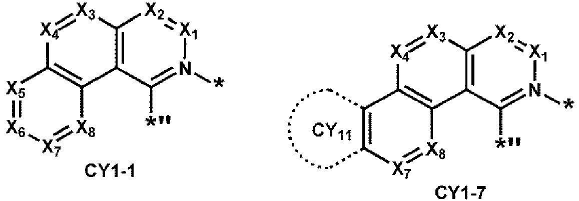 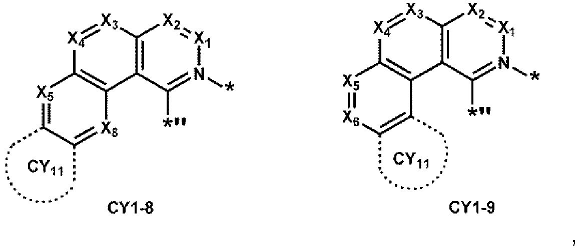
* eine Bindungsstelle an Iridium (Ir) in Formel 1 ist und *" eine Bindungsstelle an den Ring CY₂ in Formel 1 ist,
Ring CY₂ eine carbocyclische C₅-C₃₀-Gruppe oder eine heterocyclische C₁-C₃₀-Gruppe ist,
X₁ bis X₈ jeweils unabhängig C oder N sind, wobei der Fall, in dem alle von X₁ bis X₈ N sind, ausgeschlossen ist,
Ring CY₁₁ eine Cyclopentangruppe, eine Cyclopentengruppe, eine Cyclohexangruppe, eine Cyclohexengruppe, eine Adamantangruppe, eine Norbornangruppe, eine Norbornengruppe, eine Benzolgruppe, eine Pyridingruppe, eine Pyrimidingruppe, eine Pyrazingruppe, eine Pyridazingruppe, eine Naphthalengruppe, eine Chinolingruppe, eine Isochinolingruppe oder eine Chinoxalingruppe ist,
T₁ und A₁ bis A₇ jeweils unabhängig Wasserstoff, Deuterium, -F, -Cl, -Br, -I, -SF₅, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte C₁-C₆₀-Alkylgruppe, eine substituierte oder unsubstituierte C₂-C₆₀-Alkenylgruppe, eine substituierte oder unsubstituierte C₂-C₆₀-Alkinylgruppe, eine substituierte oder unsubstituierte C₁-C₆₀-Alkoxygruppe, eine substituierte oder unsubstituierte C₁-C₆₀-Alkylthiogruppe, eine substituierte oder unsubstituierte C₃-C₁₀-Cycloalkylgruppe, eine substituierte oder unsubstituierte C₁-C₁₀-Heterocycloalkylgruppe, eine substituierte oder unsubstituierte C₃-C₁₀-Cycloalkenylgruppe, eine substituierte oder unsubstituierte C₂-C₁₀-Heterocycloalkenylgruppe, eine substituierte oder unsubstituierte C₆-C₆₀-Arylgruppe, eine substituierte oder unsubstituierte C₆-C₆₀-Aryloxygruppe, eine substituierte oder unsubstituierte C₆-C₆₀-Arylthiogruppe, eine substituierte oder unsubstituierte C₁-C₆₀-Heteroarylgruppe, eine substituierte oder unsubstituierte einwertige nichtaromatische kondensierte polycyclische Gruppe, eine substituierte oder unsubstituierte einwertige nichtaromatische kondensierte heteropolycyclische Gruppe, -N(Q₁)(Q₂), -Si(Q₃)(Q₄)(Q₅), -Ge(Q₃)(Q₄)(Q₅), -B(Q₆)(Q₇), - P(=O)(Q₈)(Q₉) oder -P(Q₈)(Q₉) sind,
T₂ Deuterium, -Cl, -Br, -I, -SF₅, eine Hydroxylgruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte C₁-C₆₀-Alkylgruppe, eine substituierte oder unsubstituierte C₂-C₆₀-Alkenylgruppe, eine substituierte oder unsubstituierte C₂-C₆₀-Alkinylgruppe, eine substituierte oder unsubstituierte C₁-C₆₀-Alkoxygruppe, eine substituierte oder unsubstituierte C₁-C₆₀-Alkylthiogruppe, eine substituierte oder unsubstituierte C₃-C₁₀-Cycloalkylgruppe, eine substituierte oder unsubstituierte C₁-C₁₀-Heterocycloalkylgruppe, eine substituierte oder unsubstituierte C₃-C₁₀-Cycloalkenylgruppe, eine substituierte oder unsubstituierte C₂-C₁₀-Heterocycloalkenylgruppe, eine substituierte oder unsubstituierte C₆-C₆₀-Arylgruppe, eine substituierte oder unsubstituierte C₆-C₆₀-Aryloxygruppe, eine substituierte oder unsubstituierte C₆-C₆₀-Arylthiogruppe, eine substituierte oder unsubstituierte C₁-C₆₀-Heteroarylgruppe, eine substituierte oder unsubstituierte einwertige nichtaromatische kondensierte polycyclische Gruppe, eine substituierte oder unsubstituierte einwertige nichtaromatische kondensierte heteropolycyclische Gruppe, -N(Q₁)(Q₂), - Si(Q₃)(Q₄)(Q₅), -Ge(Q₃)(Q₄)(Q₅), -B(Q₆)(Q₇), -P(=O)(Q₈)(Q₉) oder -P(Q₈)(Q₉) ist,
a1 eine ganze Zahl von 1 bis 20 ist; wenn a1 2 oder größer ist, zwei oder mehr T₁ zueinander identisch oder verschieden sind; und zumindest eines von T₁ in Anzahl von a1 zumindest ein -F umfasst,
zwei oder mehr von T₁ in Anzahl von a1 optional miteinander verknüpft sind, um eine carbocyclische C₅-C₃₀-Gruppe, die unsubstituiert oder substituiert mit zumindest einem R₁ₐ ist, oder eine heterocyclische C₁-C₃₀-Gruppe, die unsubstituiert oder substituiert mit zumindest einem R₁ₐ ist, zu bilden,
zwei oder mehr von T₂ in Anzahl von a2 optional miteinander verknüpft sind, um eine carbocyclische C₅-C₃₀-Gruppe, die unsubstituiert oder substituiert mit zumindest einem R₁ₐ ist, oder eine heterocyclische C₁-C₃₀-Gruppe, die unsubstituiert oder substituiert mit zumindest einem R₁ₐ ist, zu bilden,
T₁ und T₂ optional verknüpft sind, um eine carbocyclische C₅-C₃₀-Gruppe, die unsubstituiert oder substituiert mit zumindest einem R₁ₐ ist, oder eine heterocyclische C₁-C₃₀-Gruppe, die unsubstituiert oder substituiert mit zumindest einem R₁ₐ ist, zu bilden,
zwei oder mehr von A₁ bis A₇ optional miteinander verknüpft sind, um eine carbocyclische C₅-C₃₀-Gruppe, die unsubstituiert oder substituiert mit zumindest einem R₁ₐ ist, oder eine heterocyclische C₁-C₃₀-Gruppe, die unsubstituiert oder substituiert mit zumindest einem R₁ₐ ist, zu bilden,
R₁ₐ das gleiche wie definiert in Verbindung mit A₇ ist,
ein Substituent(en) der substituierten C₁-C₆₀-Alkylgruppe, der substituierten C₂-C₆₀-Alkenylgruppe, der substituierten C₂-C₆₀-Alkinylgruppe, der substituierten C₁-C₆₀-Alkoxygruppe, der substituierten C₁-C₆₀-Alkylthiogruppe, der substituierten C₃-C₁₀-Cycloalkylgruppe, der substituierten C₁-C₁₀-Heterocycloalkylgruppe, der substituierten C₃-C₁₀-Cycloalkenylgruppe, der substituierten C₂-C₁₀-Heterocycloalkenylgruppe, der substituierten C₆-C₆₀-Arylgruppe, der substituierten C₆-C₆₀-Aryloxygruppe, der substituierten C₆-C₆₀-Arylthiogruppe, der substituierten C₁-C₆₀-Heteroarylgruppe, der substituierten einwertigen nichtaromatischen kondensierten polycyclischen Gruppe und der substituierten einwertigen nichtaromatischen kondensierten heteropolycyclischen Gruppe jeweils unabhängig wie folgt sind:
Deuterium, -F, -Cl, -Br, -I, -CD₃, -CD₂H, -CDH₂, -CF₃, -CF₂H, -CFH₂, eine Hydroxylgruppe, eine Cyanogruppe, eine Nitrogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine C₁-C₆₀-Alkylgruppe, eine C₂-C₆₀-Alkenylgruppe, eine C₂-C₆₀-Alkinylgruppe oder eine C₁-C₆₀-Alkoxygruppe;
eine C₁-C₆₀-Alkylgruppe, eine C₂-C₆₀-Alkenylgruppe, eine C₂-C₆₀-Alkinylgruppe oder eine C₁-C₆₀-Alkoxygruppe, jeweils substituiert mit Deuterium, -F, -Cl, -Br, -I, -CD₃, -CD₂H, - CDH₂, -CF₃, -CF₂H, -CFH₂, einer Hydroxylgruppe, einer Cyanogruppe, einer Nitrogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₃-C₁₀-Cycloalkylgruppe, einer C₁-C₁₀-Heterocycloalkylgruppe, einer C₃-C₁₀-Cycloalkenylgruppe, einer C₂-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer einwertigen nichtaromatischen kondensierten polycyclischen Gruppe, einer einwertigen nichtaromatischen kondensierten heteropolycyclischen Gruppe, -N(Q₁₁)(Q₁₂), - Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉), -P(Q₁₈)(Q₁₉) oder einer beliebigen Kombination davon;
eine C₃-C₁₀-Cycloalkylgruppe, eine C₁-C₁₀-Heterocycloalkylgruppe, eine C₃-C₁₀-Cycloalkenylgruppe, eine C₂-C₁₀-Heterocycloalkenylgruppe, eine C₆-C₆₀-Arylgruppe, eine C₆-C₆₀-Aryloxygruppe, eine C₆-C₆₀-Arylthiogruppe, eine C₁-C₆₀-Heteroarylgruppe, eine einwertige nichtaromatische kondensierte polycyclische Gruppe oder eine einwertige nichtaromatische kondensierte heteropolycyclische Gruppe, jeweils unsubstituiert oder substituiert mit Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₆₀-Alkylgruppe, einer C₂-C₆₀-Alkenylgruppe, einer C₂-C₆₀-Alkinylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₂-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer einwertigen nichtaromatischen kondensierten polycyclischen Gruppe, einer einwertigen nichtaromatischen kondensierten heteropolycyclischen Gruppe, -N(Q₂₁)(Q₂₂), -Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), -P(Q₂₈)(Q₂₉) oder einer beliebigen Kombination davon;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), -P(=O)(Q₃₈)(Q₃₉) oder -P(Q₃₈)(Q₃₉); oder
eine beliebige Kombination davon,
Q₁ bis Q₉, Q₁₁ bis Q₁₉, Q₂₁ bis Q₂₉ und Q₃₁ bis Q₃₉ jeweils unabhängig wie folgt sind:
Wasserstoff; Deuterium; -F; -Cl; -Br; -I; eine Hydroxylgruppe; eine Cyanogruppe; eine Nitrogruppe; eine Amidinogruppe; eine Hydrazingruppe; eine Hydrazongruppe; eine Carbonsäuregruppe oder ein Salz davon; eine Sulfonsäuregruppe oder ein Salz davon; eine Phosphorsäuregruppe oder ein Salz davon; eine C₁-C₆₀-Alkylgruppe unsubstituiert oder substituiert mit Deuterium, einer C₁-C₆₀-Alkylgruppe, einer C₆-C₆₀-Arylgruppe oder einer beliebigen Kombination davon; eine C₂-C₆₀-Alkenylgruppe, eine C₂-C₆₀-Alkinylgruppe, eine C₁-C₆₀-Alkoxygruppe; eine C₃-C₁₀-Cycloalkylgruppe; eine C₁-C₁₀-Heterocycloalkylgruppe; eine C₃-C₁₀-Cycloalkenylgruppe; eine C₂-C₁₀-Heterocycloalkenylgruppe, eine C₆-C₆₀-Arylgruppe unsubstituiert oder substituiert mit Deuterium, einer C₁-C₆₀-Alkylgruppe, einer C₆-C₆₀-Arylgruppe oder einer beliebigen Kombination davon; eine C₆-C₆₀-Aryloxygruppe, eine C₆-C₆₀-Arylthiogruppe; eine C₁-C₆₀-Heteroarylgruppe, eine einwertige nichtaromatische kondensierte polycyclische Gruppe; oder eine einwertige nichtaromatisch kondensierte heteropolycyclische Gruppe,
wobei a2 1, 2 oder 3 ist und wenn a2 2 oder größer ist, zwei oder mehr T₂ zueinander identisch oder verschieden sind, und
wobei in Formel 1 der folgende Fall ausgeschlossen ist: 1) A₇ ist Wasserstoff und 2) sowohl die Gruppe dargestellt durch *-C(A₁)(A₂)(A₃) als auch die Gruppe dargestellt durch *-C(A₄)(A₅)(A₆) sind eine Methylgruppe.

2. Organometallische Verbindung nach Anspruch 1, wobei der Ring CY₂ eine Benzolgruppe, eine Naphthalengruppe, eine 1,2,3,4-Tetrahydronaphthalengruppe, eine Thiophengruppe, eine Furangruppe, eine Pyrrolgruppe, eine Cyclopentadiengruppe, eine Silolgruppe, eine Benzothiophengruppe, eine Benzofurangruppe, eine Indolgruppe, eine Indengruppe, eine Benzosilolgruppe, eine Dibenzothiophengruppe, eine Dibenzofurangruppe, eine Carbazolgruppe, eine Fluorengruppe oder eine Dibenzosilolgruppe ist; und/oder
wobei T₁ und A₁ bis A₇ jeweils unabhängig Wasserstoff, Deuterium, -F, eine substituierte oder unsubstituierte C₁-C₂₀-Alkylgruppe, eine substituierte oder unsubstituierte C₃-C₁₀-Cycloalkylgruppe, eine substituierte oder unsubstituierte C₁-C₁₀-Heterocycloalkylgruppe, eine substituierte oder unsubstituierte Phenylgruppe, eine substituierte oder unsubstituierte Biphenylgruppe, eine substituierte oder unsubstituierte Terphenylgruppe, -Si(Q₃)(Q₄)(Q₅) oder - Ge(Q₃)(Q₄)(Q₅) sind;
wobei T₂ Deuterium, eine substituierte oder unsubstituierte C₁-C₂₀-Alkylgruppe, eine substituierte oder unsubstituierte C₃-C₁₀-Cycloalkylgruppe, eine substituierte oder unsubstituierte C₁-C₁₀-Heterocycloalkylgruppe, eine substituierte oder unsubstituierte Phenylgruppe, eine substituierte oder unsubstituierte Biphenylgruppe, eine substituierte oder unsubstituierte Terphenylgruppe, -Si(Q₃)(Q₄)(Q₅) oder -Ge(Q₃)(Q₄)(Q₅) ist;
wobei zumindest eines von T₁ in Anzahl von a1 zumindest ein -F umfasst; und
wobei in Formel 1 der folgende Fall ausgeschlossen ist: 1) A₇ ist Wasserstoff und 2) sowohl die Gruppe dargestellt durch *-C(A₁)(A₂)(A₃) als auch die Gruppe dargestellt durch *-C(A₄)(A₅)(A₆) sind eine Methylgruppe.

3. Organometallische Verbindung nach Anspruch 1 oder 2, wobei T₁ und A₁ bis A₇ jeweils unabhängig wie folgt sind:
Wasserstoff, Deuterium, -F, C₁-C₂₀-Alkylgruppe, eine C₃-C₁₀-Cycloalkylgruppe, eine C₁-C₁₀-Heterocycloalkylgruppe, eine Phenylgruppe, eine Biphenylgruppe oder eine Terphenylgruppe;
eine fluorierte C₁-C₂₀-Alkylgruppe, eine fluorierte C₃-C₁₀-Cycloalkylgruppe, eine fluorierte C₁-C₁₀-Heterocycloalkylgruppe, eine fluorierte Phenylgruppe, eine fluorierte Biphenylgruppe oder eine fluorierte Terphenylgruppe, jeweils unsubstituiert oder substituiert mit Deuterium, C₁-C₂₀-Alkylgruppe, einer C₃-C₁₀-Cycloalkylgruppe, einer C₁-C₁₀-Heterocycloalkylgruppe, einer Phenylgruppe, einer Biphenylgruppe, einer Terphenylgruppe oder einer beliebigen Kombination davon;
eine deuterierte C₁-C₂₀-Alkylgruppe, eine deuterierte C₃-C₁₀-Cycloalkylgruppe, eine deuterierte C₁-C₁₀-Heterocycloalkylgruppe, eine deuterierte Phenylgruppe, eine deuterierte Biphenylgruppe oder eine deuterierte Terphenylgruppe, jeweils unsubstituiert oder substituiert mit -F, C₁-C₂₀-Alkylgruppe, einer C₃-C₁₀-Cycloalkylgruppe, einer C₁-C₁₀-Heterocycloalkylgruppe, einer Phenylgruppe, einer Biphenylgruppe, einer Terphenylgruppe oder einer beliebigen Kombination davon; oder
-Si(Q₃)(Q₄)(Q₅) oder -Ge(Q₃)(Q₄)(Q₅),
wobei zumindest eines von T₁ in Anzahl von a1 zumindest ein -F umfasst; und
wobei in Formel 1 der folgende Fall ausgeschlossen ist: 1) A₇ ist Wasserstoff und 2) sowohl die Gruppe dargestellt durch *-C(A₁)(A₂)(A₃) als auch die Gruppe dargestellt durch *-C(A₄)(A₅)(A₆) sind eine Methylgruppe.

4. Organometallische Verbindung nach einem der Ansprüche 1-3, wobei A₁ bis A₇ jeweils unabhängig wie folgt sind:
Wasserstoff, Deuterium, C₁-C₂₀-Alkylgruppe, eine C₃-C₁₀-Cycloalkylgruppe, eine C₁-C₁₀-Heterocycloalkylgruppe, eine Phenylgruppe, eine Biphenylgruppe oder eine Terphenylgruppe;
eine deuterierte C₁-C₂₀-Alkylgruppe, eine deuterierte C₃-C₁₀-Cycloalkylgruppe, eine deuterierte C₁-C₁₀-Heterocycloalkylgruppe, eine deuterierte Phenylgruppe, eine deuterierte Biphenylgruppe oder eine deuterierte Terphenylgruppe, jeweils unsubstituiert oder substituiert mit einer C₁-C₂₀-Alkylgruppe, einer C₃-C₁₀-Cycloalkylgruppe, einer C₁-C₁₀-Heterocycloalkylgruppe, einer Phenylgruppe, einer Biphenylgruppe, einer Terphenylgruppe oder einer beliebigen Kombination davon; oder
-Si(Q₃)(Q₄)(Q₅) oder -Ge(Q₃)(Q₄)(Q₅);
wobei T₂ wie folgt ist:
Deuterium, C₁-C₂₀-Alkylgruppe, eine C₃-C₁₀-Cycloalkylgruppe, eine C₁-C₁₀-Heterocycloalkylgruppe, eine Phenylgruppe, eine Biphenylgruppe oder eine Terphenylgruppe;
eine deuterierte C₁-C₂₀-Alkylgruppe, eine deuterierte C₃-C₁₀-Cycloalkylgruppe, eine deuterierte C₁-C₁₀-Heterocycloalkylgruppe, eine deuterierte Phenylgruppe, eine deuterierte Biphenylgruppe oder eine deuterierte Terphenylgruppe, jeweils unsubstituiert oder substituiert mit einer C₁-C₂₀-Alkylgruppe, einer C₃-C₁₀-Cycloalkylgruppe, einer C₁-C₁₀-Heterocycloalkylgruppe, einer Phenylgruppe, einer Biphenylgruppe, einer Terphenylgruppe oder einer beliebigen Kombination davon; oder
-Si(Q₃)(Q₄)(Q₅) oder -Ge(Q₃)(Q₄)(Q₅); und
wobei in Formel 1 der folgende Fall ausgeschlossen ist: 1) A₇ ist Wasserstoff und 2) sowohl die Gruppe dargestellt durch *-C(A₁)(A₂)(A₃) als auch die Gruppe dargestellt durch *-C(A₄)(A₅)(A₆) sind eine Methylgruppe.

5. Organometallische Verbindung nach einem der Ansprüche 1-4, wobei eine Gruppe dargestellt durch 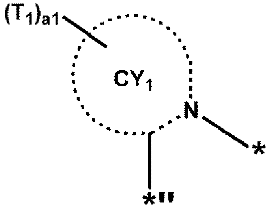 in Formel 1 dargestellt ist durch Formel CY1(1): 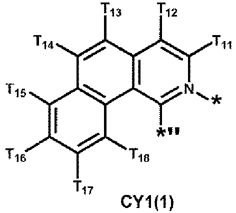 wobei in Formel CY1(1)
T₁₁ bis T₁₈ die gleichen wie definiert in Verbindung mit T₁ in Anspruch 1 sind, vorausgesetzt, dass zumindest eines von T₁₁ bis T₁₈ zumindest eine Fluorgruppe (-F) umfasst,
* eine Bindungsstelle an Ir in Formel 1 angibt, und
*" eine Bindungsstelle an Ring CY₂ in Formel 1 angibt.

6. Organometallische Verbindung nach einem der Ansprüche 1-5, wobei eine Gruppe dargestellt durch 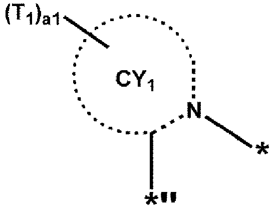 in Formel 1 eine von Gruppen dargestellt durch Formel CY1(1)-1 bis CY1(1)-64 ist: 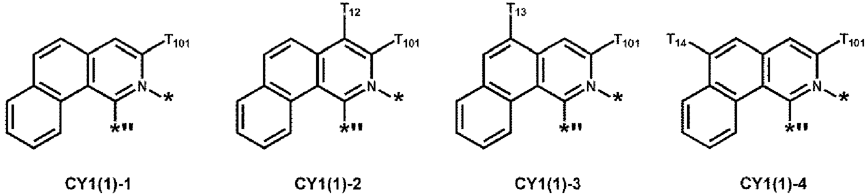 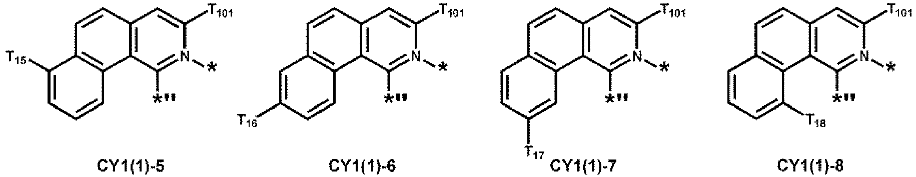 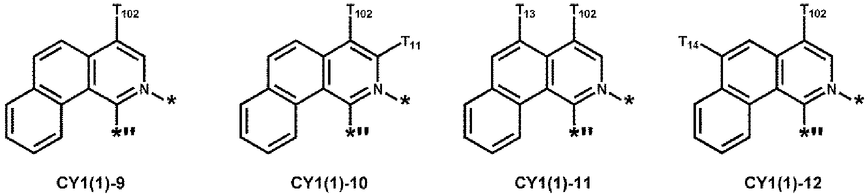 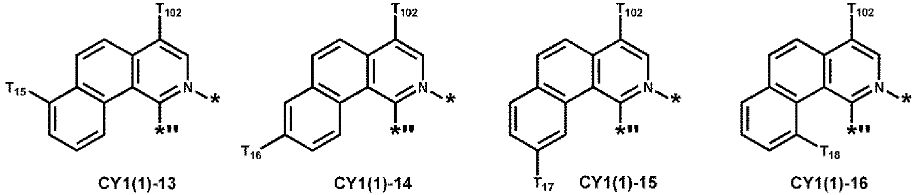 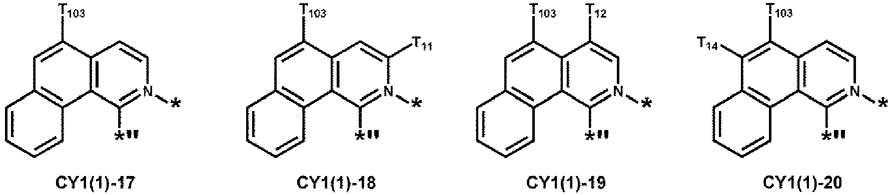 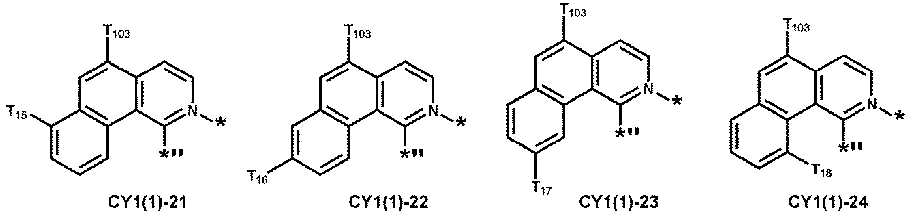 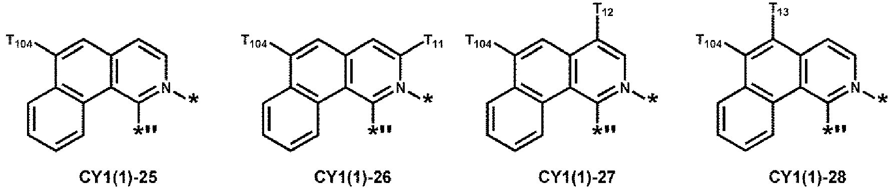 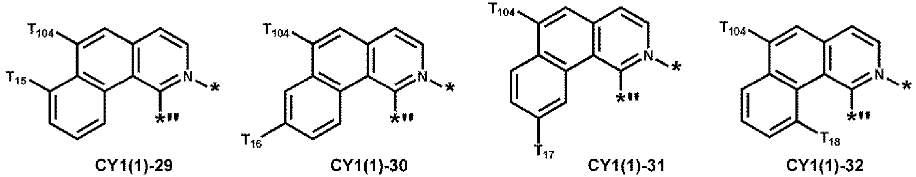 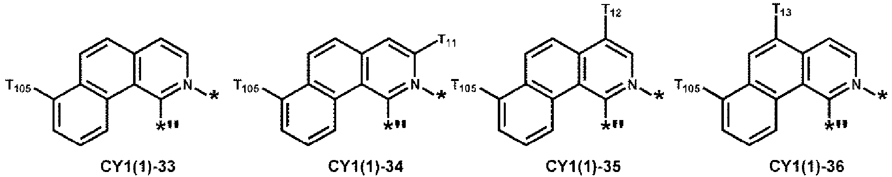 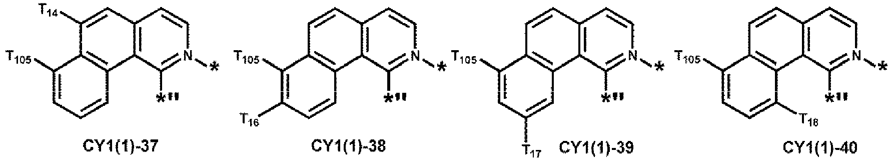 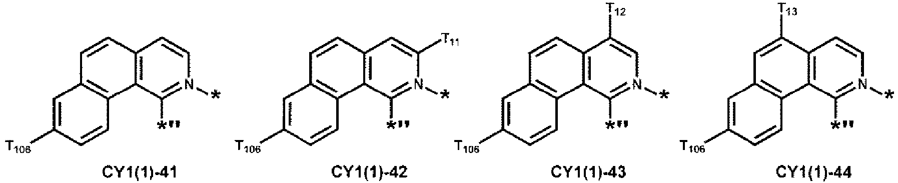 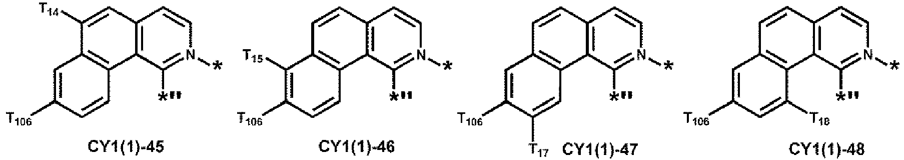 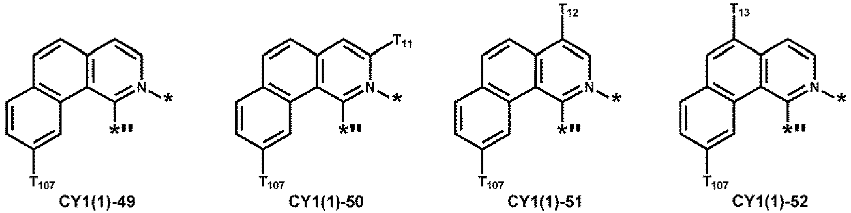 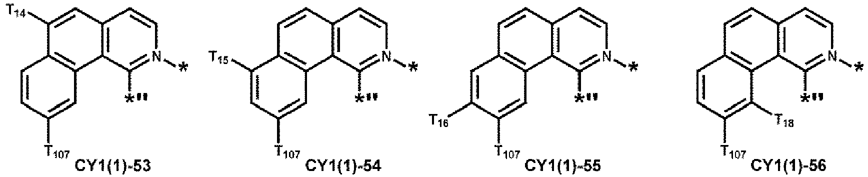 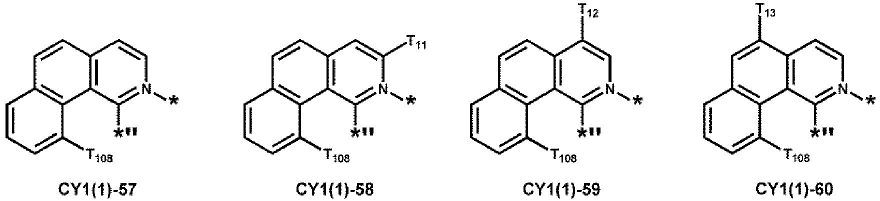 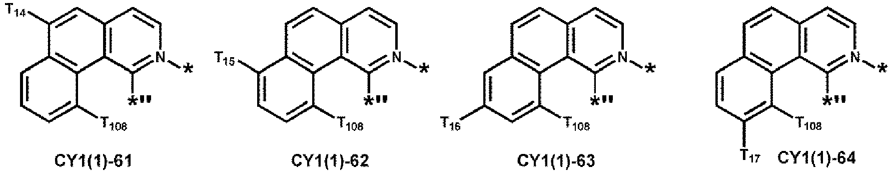 wobei in Formel CY1(1)-1 bis CY1(1)-64
T₁₁ bis T₁₈ die gleichen wie definiert in Verbindung mit T₁ in Anspruch 1 sind, vorausgesetzt, dass jedes von T₁₁ bis T₁₈ nicht Wasserstoff ist,
T₁₀₁ bis T₁₀₈ jeweils zumindest eine Fluorgruppe (-F) umfassen,
* eine Bindungsstelle an Ir in Formel 1 angibt, und
*" eine Bindungsstelle an Ring CY₂ in Formel 1 angibt;
wobei bevorzugt in Formel CY1(1)-1 bis CY1(1)-64,
T₁₁ bis T₁₈ jeweils unabhängig wie folgt sind:
Deuterium, eine C₁-C₂₀-Alkylgruppe, eine C₃-C₁₀-Cycloalkylgruppe, eine C₁-C₁₀-Heterocycloalkylgruppe, eine Phenylgruppe, eine Biphenylgruppe oder eine Terphenylgruppe;
eine deuterierte C₁-C₂₀-Alkylgruppe, eine deuterierte C₃-C₁₀-Cycloalkylgruppe, eine deuterierte C₁-C₁₀-Heterocycloalkylgruppe, eine deuterierte Phenylgruppe, eine deuterierte Biphenylgruppe oder eine deuterierte Terphenylgruppe, jeweils unsubstituiert oder substituiert mit einer C₁-C₂₀-Alkylgruppe, einer C₃-C₁₀-Cycloalkylgruppe, einer C₁-C₁₀-Heterocycloalkylgruppe, einer Phenylgruppe, einer Biphenylgruppe, einer Terphenylgruppe oder einer beliebigen Kombination davon; oder
-Si(Q₃)(Q₄)(Q₅) oder -Ge(Q₃)(Q₄)(Q₅), und
T₁₀₁ bis T₁₀₈ jeweils unabhängig wie folgt sind:
-F; oder
eine fluorierte C₁-C₂₀-Alkylgruppe, eine fluorierte C₃-C₁₀-Cycloalkylgruppe, eine fluorierte C₁-C₁₀-Heterocycloalkylgruppe, eine fluorierte Phenylgruppe, eine fluorierte Biphenylgruppe oder eine fluorierte Terphenylgruppe, jeweils unsubstituiert oder substituiert mit Deuterium, einer C₁-C₂₀-Alkylgruppe, einer C₃-C₁₀-Cycloalkylgruppe, einer C₁-C₁₀-Heterocycloalkylgruppe, einer Phenylgruppe, einer Biphenylgruppe, einer Terphenylgruppe oder einer beliebigen Kombination davon.

7. Organometallische Verbindung nach einem der Ansprüche 1-6, wobei eine Gruppe dargestellt durch 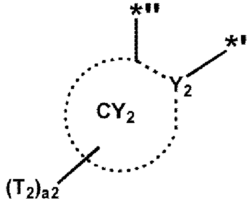 in Formel 1 eine der Gruppen dargestellt durch Formel CY2-1 bis CY2-31 ist: 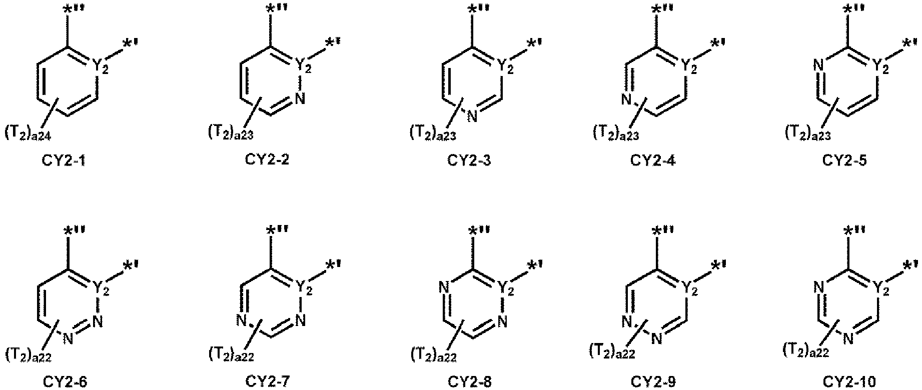 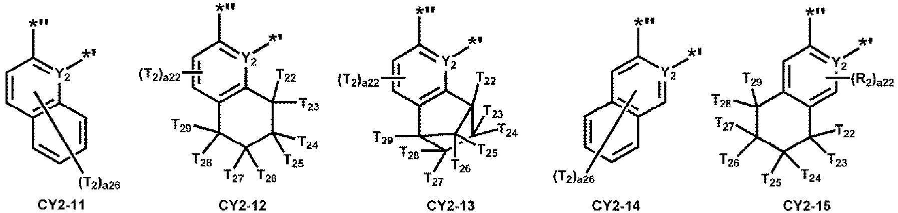 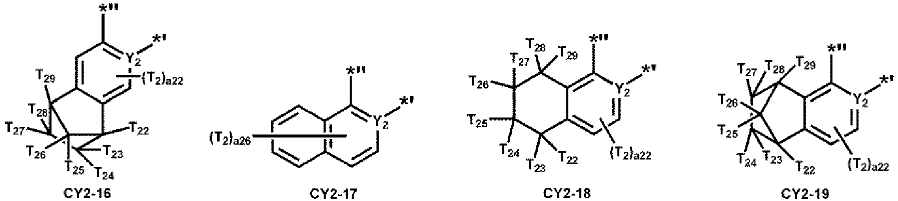 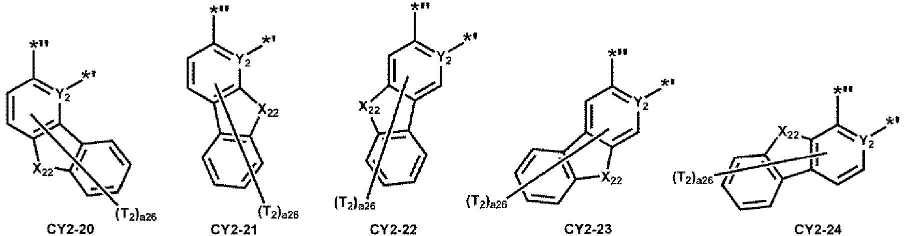 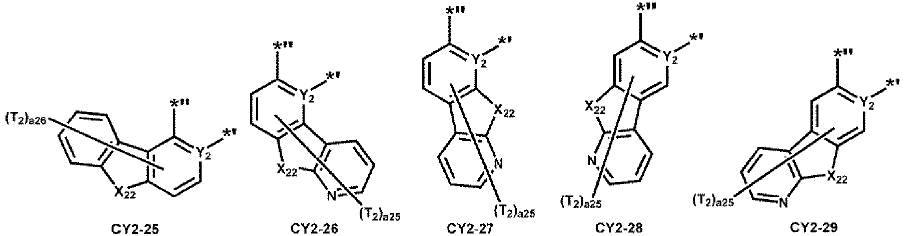 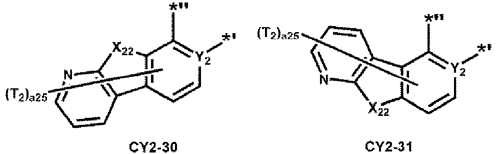 wobei in Formel CY2-1 bis CY2-31
Y₂ und T₂ die gleichen sind wie definiert in Anspruch 1,
X₂₂ C(T₂₈)(T₂₉), N(T₂₈), O, S oder Si(T₂₈)(T₂₉) ist,
T₂₂ bis T₂₉ die gleichen sind wie definiert in Verbindung mit T₂ in Anspruch 1,
a26 eine ganze Zahl von 1, 2 oder 3 ist,
a25 eine ganze Zahl von 1, 2 oder 3 ist,
a24 eine ganze Zahl von 1, 2 oder 3 ist,
a23 eine ganze Zahl von 1, 2 oder 3 ist,
a22 eine ganze Zahl von 1 oder 2 ist,
*" eine Bindungsstelle an Ring CY₁ in Formel 1 angibt, und
*' eine Bindungsstelle an Ir in Formel 1 angibt.

8. Organometallische Verbindung nach einem der Ansprüche 1-7, wobei eine Gruppe dargestellt durch 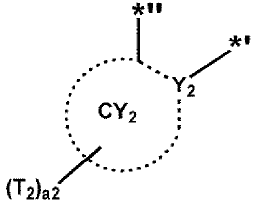 in Formel 1 eine von Gruppen dargestellt durch Formel CY2(2) bis CY2(15), CY2(18) bis CY2(24), CY2(26) bis CY2(32), CY2(34) bis CY3(40), CY3(42) bis CY3(48), CY2(50) bis CY2(52), CY2(55), CY2(56), CY2(64), CY2(65), CY2(67) und CY2(68) ist: 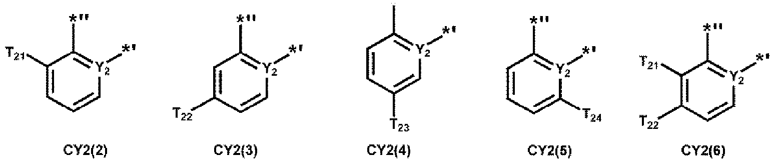 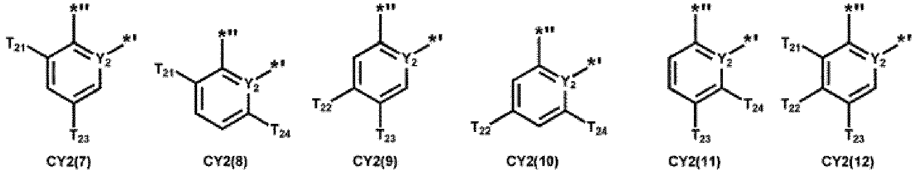 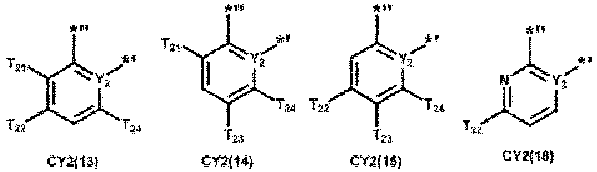 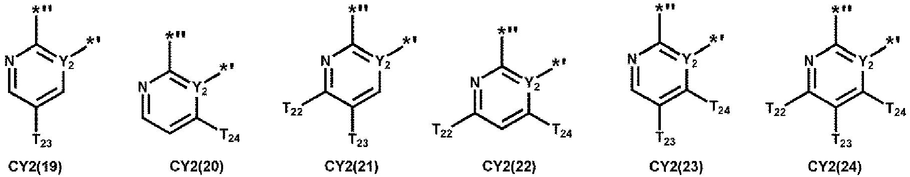 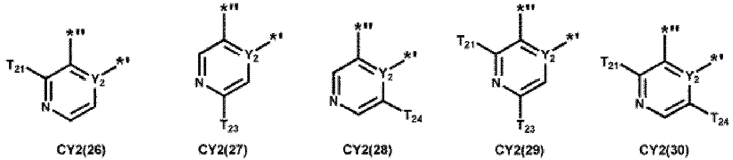 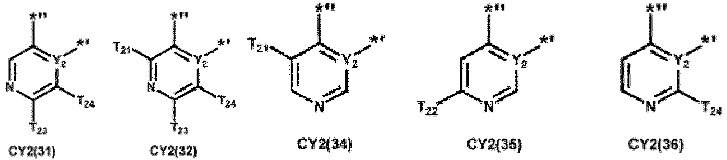 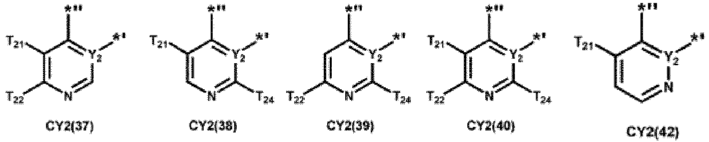 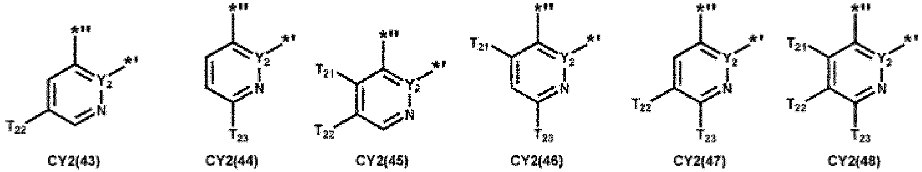 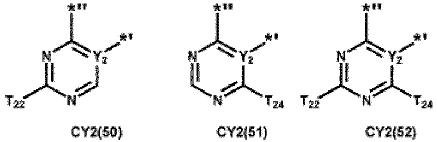 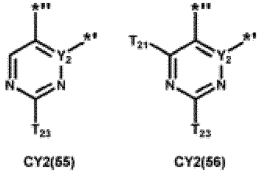 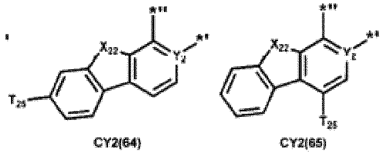 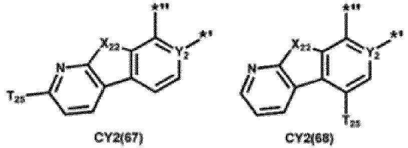
wobei in Formel CY2(2) bis CY2(15), CY2(18) bis CY2(24), CY2(26) bis CY2(32), CY2(34) bis CY3(40), CY3(42) bis CY3(48), CY2(50) bis CY2(52), CY2(55), CY2(56), CY2(64), CY2(65), CY2(67) und CY2(68),
Y₂ das gleiche wie definiert in Anspruch 1 ist,
X₂₂ C(T₂₈)(T₂₉), N(T₂₈), O, S oder Si(T₂₈)(T₂₉) ist,
T₂₁ bis T₂₅, T₂₈ und T₂₉ die gleichen wie definiert in Verbindung mit T₂ in Anspruch 1 sind,
*" eine Bindungsstelle an Ring CY₁ in Formel 1 angibt, und
*' eine Bindungsstelle an Ir in Formel 1 angibt.

9. Organometallische Verbindung nach einem der Ansprüche 1-8, wobei die organometallische Verbindung zumindest eine von Bedingung 1 bis Bedingung 3 erfüllt:
Bedingung 1
A₁ bis A₆ in Formel 1 sind jeweils unabhängig eine substituierte oder unsubstituierte C₁-C₆₀-Alkylgruppe, eine substituierte oder unsubstituierte C₃-C₁₀-Cycloalkylgruppe, eine substituierte oder unsubstituierte C₂-C₁₀-Heterocycloalkylgruppe, eine substituierte oder unsubstituierte C₆-C₆₀-Arylgruppe, eine substituierte oder unsubstituierte C₂-C₆₀-Heteroarylgruppe, eine substituierte oder unsubstituierte einwertige nichtaromatische kondensierte polycyclische Gruppe oder eine substituierte oder unsubstituierte einwertige nichtaromatische kondensierte heteropolycyclische Gruppe;
Bedingung 2
zumindest eines von A₁ bis A₆ in Formel 1 ist jeweils unabhängig eine substituierte oder unsubstituierte C₂-C₆₀-Alkylgruppe, eine substituierte oder unsubstituierte C₃-C₁₀-Cycloalkylgruppe, eine substituierte oder unsubstituierte C₂-C₁₀-Heterocycloalkylgruppe, eine substituierte oder unsubstituierte C₆-C₆₀-Arylgruppe, eine substituierte oder unsubstituierte C₂-C₆₀-Heteroarylgruppe, eine substituierte oder unsubstituierte einwertige nichtaromatische kondensierte polycyclische Gruppe oder eine substituierte oder unsubstituierte einwertige nichtaromatische kondensierte heteropolycyclische Gruppe; und
Bedingung 3
A₇ in Formel 1 ist Deuterium, eine substituierte oder unsubstituierte C₁-C₆₀-Alkylgruppe, eine substituierte oder unsubstituierte C₃-C₁₀-Cycloalkylgruppe, eine substituierte oder unsubstituierte C₂-C₁₀-Heterocycloalkylgruppe, eine substituierte oder unsubstituierte C₆-C₆₀-Arylgruppe, eine substituierte oder unsubstituierte C₂-C₆₀-Heteroarylgruppe, eine substituierte oder unsubstituierte einwertige nichtaromatische kondensierte polycyclische Gruppe oder eine substituierte oder unsubstituierte einwertige nichtaromatische kondensierte heteropolycyclische Gruppe.

10. Organometallische Verbindung nach einem der Ansprüche 1-9, wobei die organometallische Verbindung zumindest eine von Bedingung 4 und Bedingung 5 erfüllt:
Bedingung 4
in Formel 1 sind zwei oder mehr von A₁ bis A₃ der Gruppe dargestellt durch *-C(A₁)(A₂)(A₃) verknüpft mit C in der Gruppe dargestellt durch *-C(A₁)(A₂)(A₃), um eine carbocyclische C₅-C₃₀-Gruppe unsubstituiert oder substituiert mit zumindest einem R₁ₐ oder eine heterocyclische C₁-C₃₀-Gruppe unsubstituiert oder substituiert mit zumindest einem R₁ₐ zu bilden, sodass die Gruppe dargestellt durch *-C(A₁)(A₂)(A₃) eine carbocyclische C₅-C₃₀-Gruppe unsubstituiert oder substituiert mit zumindest einem R₁ₐ oder eine heterocyclische C₁-C₃₀-Gruppe unsubstituiert oder substituiert mit zumindest einem R₁ₐ ist
Bedingung 5
in Formel 1 sind zwei oder mehr von A₄ bis A₆ der Gruppe dargestellt durch *-C(A₄)(A₅)(A₆) verknüpft mit C in der Gruppe dargestellt durch *-C(A₄)(A₅)(A₆), um eine carbocyclische C₅-C₃₀-Gruppe unsubstituiert oder substituiert mit zumindest einem R₁ₐ oder eine heterocyclische C₁-C₃₀-Gruppe unsubstituiert oder substituiert mit zumindest einem R₁ₐ zu bilden, sodass die Gruppe dargestellt durch *-C(A₄)(A₅)(A₆) eine carbocyclische C₅-C₃₀-Gruppe unsubstituiert oder substituiert mit zumindest einem R₁ₐ oder eine heterocyclische C₁-C₃₀-Gruppe unsubstituiert oder substituiert mit zumindest einem R₁ₐ ist.

11. Organische lichtemittierende Vorrichtung, umfassend:
eine erste Elektrode;
eine zweite Elektrode; und
eine organische Schicht angeordnet zwischen der ersten Elektrode und der zweiten Elektrode und umfassend eine Emissionsschicht, wobei die organische Schicht zumindest eine organometallische Verbindung dargestellt durch Formel 1 nach einem der Ansprüche 1-10 umfasst;
wobei bevorzugt
die erste Elektrode eine Anode ist,
die zweite Elektrode eine Kathode ist,
die organische Schicht ferner eine Lochtransportregion zwischen der ersten Elektrode und der Emissionsschicht und eine Elektronentransportregion zwischen der Emissionsschicht und der zweiten Elektrode umfasst,
die Lochtransportregion eine Locheinspritzschicht, eine Lochtransportschicht, eine Elektronenblockierschicht, eine Pufferschicht oder eine beliebige Kombination davon umfasst, und
die Elektronentransportregion eine Lochblockierschicht, eine Elektronentransportschicht, eine Elektroneneinspritzschicht oder eine beliebige Kombination davon umfasst.

12. Organische lichtemittierende Vorrichtung nach Anspruch 11, wobei die organometallische Verbindung in der Emissionsschicht ist;
wobei bevorzugt die Emissionsschicht ferner einen Wirt umfasst und eine Menge des Wirts in der Emissionsschicht größer als eine Menge der organometallischen Verbindung in der Emissionsschicht ist.

13. Elektronische Vorrichtung, umfassend die organische lichtemittierende Vorrichtung nach Anspruch 11 oder 12.

## Revendications

1. Composé organométallique représenté par la formule 1, 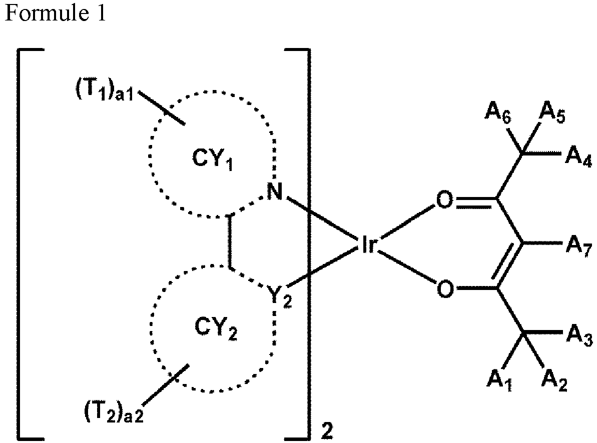 dans lequel, dans la formule 1,
Y₂ est C,
le cycle CY₁ est un groupe polycyclique en C₅ à C₃₀ où trois groupes monocycliques ou plus sont condensés les uns aux autres, dans lequel le groupe représenté par 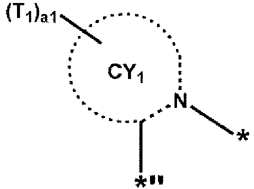 dans la formule 1 est l'un des groupes représentés par les formules CY1-1 et CY1-7 à CY1-9 : 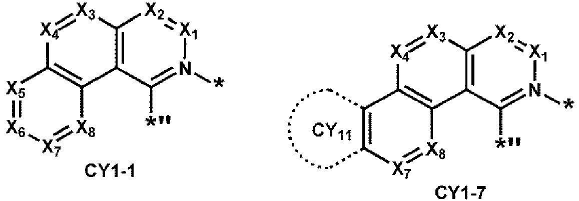 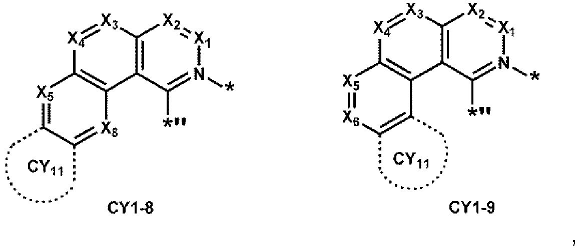
* est un site de liaison à l'iridium (Ir) dans la formule 1, et *" est un site de liaison au cycle CY₂ dans la formule 1,
le cycle CY₂ est un groupe carbocyclique en C₅ à C₃₀ ou un groupe hétérocyclique en C₁ à C₃₀,
X₁ à X₈ sont chacun indépendamment C ou N, dans lequel le cas où tous les X₁ à X₈ sont N est exclu,
le cycle CY₁₁ est un groupe cyclopentane, un groupe cyclopentène, un groupe cyclohexane, un groupe cyclohexène, un groupe adamantane, un groupe norbornane, un groupe norbornène, un groupe benzène, un groupe pyridine, un groupe pyrimidine, un groupe pyrazine, un groupe pyridazine, un groupe naphtalène, un groupe quinoléine, un groupe isoquinoléine ou un groupe quinoxaline,
T₁ et A₁ à A₇ sont chacun indépendamment un hydrogène, un deutérium, -F, -Cl, -Br, -I, - SF₅, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀ substitué ou non substitué, un groupe alcényle en C₂ à C₆₀ substitué ou non substitué, un groupe alcynyle en C₂ à C₆₀ substitué ou non substitué, un groupe alcoxy en C₁ à C₆₀ substitué ou non substitué, un groupe alkylthio en C₁ à C₆₀ substitué ou non substitué, un groupe cycloalkyle en C₃ à C₁₀ substitué ou non substitué, un groupe hétérocycloalkyle en C₁ à C₁₀ substitué ou non substitué, un groupe cycloalcényle en C₃ à C₁₀ substitué ou non substitué, un groupe hétérocycloalcényle en C₂ à C₁₀ substitué ou non substitué, un groupe aryle en C₆ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe polycyclique condensé non aromatique monovalent substitué ou non substitué, un groupe hétéropolycyclique condensé non aromatique monovalent substitué ou non substitué, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), ou - P(Q₈)(Q₉),
T₂ est un deutérium, -Cl, -Br, -I, -SF₅, un groupe hydroxyle, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀ substitué ou non substitué, un groupe alcényle en C₂ à C₆₀ substitué ou non substitué, un groupe alcynyle en C₂ à C₆₀ substitué ou non substitué, un groupe alcoxy en C₁ à C₆₀ substitué ou non substitué, un groupe alkylthio en C₁ à C₆₀ substitué ou non substitué, un groupe cycloalkyle en C₃ à C₁₀ substitué ou non substitué, un groupe hétérocycloalkyle en C₁ à C₁₀ substitué ou non substitué, un groupe cycloalcényle en C₃ à C₁₀ substitué ou non substitué, un groupe hétérocycloalcényle en C₂ à C₁₀ substitué ou non substitué, un groupe aryle en C₆ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe polycyclique condensé non aromatique monovalent substitué ou non substitué, un groupe hétéropolycyclique condensé non aromatique monovalent substitué ou non substitué, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), ou - P(Q₈)(Q₉),
a1 est un nombre entier de 1 à 20 ; lorsque a1 est égal ou supérieur à 2, deux T₁(s) ou plus sont identiques ou différents les uns des autres ; et au moins l'un des T₁(s) en nombre de a1 comprend au moins un -F,
deux des T₁(s) ou plus en nombre de a1 sont éventuellement liés l'un à l'autre pour former un groupe carbocyclique en C₅ à C₃₀ non substitué ou substitué avec au moins un R₁ₐ, ou un groupe hétérocyclique en C₁ à C₃₀ non substitué ou substitué avec au moins un R₁ₐ,
deux des T₂(s) ou plus en nombre de a2 sont éventuellement liés l'un à l'autre pour former un groupe carbocyclique en C₅ à C₃₀ non substitué ou substitué avec au moins un R₁ₐ, ou un groupe hétérocyclique en C₁ à C₃₀ non substitué ou substitué avec au moins un R₁ₐ,
T₁ et T₂ sont éventuellement liés l'un à l'autre pour former un groupe carbocyclique en C₅ à C₃₀ non substitué ou substitué avec au moins un R₁ₐ, ou un groupe hétérocyclique en C₁ à C₃₀ non substitué ou substitué avec au moins un R₁ₐ,
deux de A₁ à A₇ ou plus sont éventuellement liés l'un à l'autre pour former un groupe carbocyclique en C₅ à C₃₀ non substitué ou substitué avec au moins un R₁ₐ, ou un groupe hétérocyclique en C₁ à C₃₀ non substitué ou substitué avec au moins un R1a,
R₁ₐ est le même que celui défini en relation avec A₇,
un ou plusieurs substituant(s) du groupe alkyle en C₁ à C₆₀ substitué, du groupe alcényle en C₂ à C₆₀ substitué, du groupe alcynyle en C₂ à C₆₀ substitué, du groupe alcoxy en C₁ à C₆₀ substitué, du groupe alkylthio en C₁ à C₆₀ substitué, du groupe cycloalkyle en C₃ à C₁₀ substitué, du groupe hétérocycloalkyle en C₁ à C₁₀ substitué, du groupe cycloalcényle en C₃ à C₁₀ substitué, du groupe hétérocycloalcényle en C₂ à C₁₀ substitué, du groupe aryle en C₆ à C₆₀ substitué, du groupe aryloxy en C₆ à C₆₀ substitué, du groupe arylthio en C₆ à C₆₀ substitué, du groupe hétéroaryle en C₁ à C₆₀ substitué, du groupe polycyclique condensé non aromatique monovalent substitué et du groupe hétéropolycyclique condensé non aromatique monovalent substitué sont chacun indépendamment :
un deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀, un groupe alcényle en C₂ à C₆₀, un groupe alcynyle en C₂ à C₆₀, ou un groupe alcoxy en C₁ à C₆₀ ;
un groupe alkyle en C₁ à C₆₀, un groupe alcényle en C₂ à C₆₀, un groupe alcynyle en C₂ à C₆₀, ou un groupe alcoxy en C₁ à C₆₀, chacun substitué avec un deutérium, -F, -Cl, -Br, -I, -CD₃, - CD₂H, -CDH₂, -CF₃, -CF₂H, -CFH₂, un groupe hydroxyle, un groupe cyano, un groupe nitro, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₂ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, un groupe hétéropolycyclique condensé non aromatique monovalent, -N(Q₁₁)(Q₁₂), -Si(Q₁₃)(Q₁₄)(Q₁₅), - Ge(Q₁₃)(Q₁₄)(Q₁₅), -B(Q₁₆)(Q₁₇), -P(=O)(Q₁₈)(Q₁₉), -P(Q₁₈)(Q₁₉), ou toute combinaison de ceux-ci ;
un groupe cycloalkyle en C₃ à C₁₀, un groupe hétérocycloalkyle en C₁ à C₁₀, un groupe cycloalcényle en C₃ à C₁₀, un groupe hétérocycloalcényle en C₂ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, ou un groupe hétéropolycyclique condensé non aromatique monovalent, chacun non substitué ou substitué avec du deutérium, -F, - Cl, -Br, -I, -CD₃, -CD₂H, -CDH₂, -CF₃, -CF₂H, -CFH₂, un groupe hydroxyle, un groupe cyano, un groupe nitro, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀, un groupe alcényle en C₂ à C₆₀, un groupe alcynyle en C₂ à C₆₀, un groupe alcoxy en C₁ à C₆₀, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₂ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, un groupe hétéropolycyclique condensé non aromatique monovalent, -N(Q₂₁)(Q₂₂), -Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), - P(Q₂₈)(Q₂₉), ou toute combinaison de ceux-ci ;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), -P(=O)(Q₃₈)(Q₃₉), ou -P(Q₃₈)(Q₃₉) ; ou
toute combinaison de ceux-ci,
Q₁ à Q₉, Q₁₁ à Q₁₉, Q₂₁ à Q₂₉ et Q₃₁ à Q₃₉ sont chacun indépendamment :
un hydrogène ; un deutérium ; -F ; -Cl ; -Br ; -I ; un groupe hydroxyle ; un groupe cyano ; un groupe nitro ; un groupe amidino ; un groupe hydrazine ; un groupe hydrazone ; un groupe acide carboxylique ou un sel de celui-ci ; un groupe acide sulfonique ou un sel de celui-ci ; un groupe acide phosphorique ou un sel de celui-ci ; un groupe alkyle en C₁ à C₆₀ non substitué ou substitué avec un deutérium, un groupe alkyle en C₁ à C₆₀, un groupe aryle en C₆ à C₆₀, ou toute combinaison de ceux-ci ; un groupe alcényle en C₂ à C₆₀ ; un groupe alcynyle en C₂ à C₆₀ ; un groupe alcoxy en C₁ à C₆₀ ; un groupe cycloalkyle en C₃ à C₁₀ ; un groupe hétérocycloalkyle en C₁ à C₁₀ ; un groupe cycloalcényle en C₃ à C₁₀, un groupe hétérocycloalcényle en C₂ à C₁₀, un groupe aryle en C₆ à C₆₀ non substitué ou substitué avec un deutérium, un groupe alkyle en C₁ à C₆₀, un groupe aryle en C₆ à C₆₀, ou toute combinaison de ceux-ci ; un groupe aryloxy en C₆ à C₆₀ ; un groupe arylthio en C₆ à C₆₀ ; un groupe hétéroaryle en C₁ à C₆₀ ; un groupe polycyclique condensé non aromatique monovalent ; ou un groupe hétéropolycyclique condensé non aromatique monovalent,
dans lequel a2 est égal à 1, 2 ou 3 et lorsque a2 est égal ou supérieur à 2, deux T₂(s) ou plus sont identiques ou différents les uns des autres, et
dans lequel, dans la formule 1, le cas suivant est exclu : 1) A₇ est un hydrogène, et 2) à la fois le groupe représenté par *-C(A₁)(A₂)(A₃) et le groupe représenté par *-C(A₄)(A₅)(A₆) sont un groupe méthyle.

2. Composé organométallique de la revendication 1, dans lequel le cycle CY₂ est un groupe benzène, un groupe naphtalène, un groupe 1,2,3,4-tétrahydronaphtalène, un groupe thiophène, un groupe furane, un groupe pyrrole, un groupe cyclopentadiène, un groupe silole, un groupe benzothiophène, un groupe benzofurane, un groupe indole, un groupe indène, un groupe benzosilole, un groupe dibenzothiophène, un groupe dibenzofurane, un groupe carbazole, un groupe fluorène ou un groupe dibenzosilole ; et/ou
dans lequel T₁ et A₁ à A₇ sont chacun indépendamment un hydrogène, un deutérium, -F, un groupe alkyle en C₁ à C₂₀ substitué ou non substitué, un groupe cycloalkyle en C₃ à C₁₀ substitué ou non substitué, un groupe hétérocycloalkyle en C₁ à C₁₀ substitué ou non substitué, un groupe phényle substitué ou non substitué, un groupe biphényle substitué ou non substitué, un groupe terphényle substitué ou non substitué, -Si(Q₃)(Q₄)(Q₅), ou -Ge(Q₃)(Q₄)(Q₅) ;
dans lequel T₂ est un deutérium, un groupe alkyle en C₁ à C₂₀ substitué ou non substitué, un groupe cycloalkyle en C₃ à C₁₀ substitué ou non substitué, un groupe hétérocycloalkyle en C₁ à C₁₀ substitué ou non substitué, un groupe phényle substitué ou non substitué, un groupe biphényle substitué ou non substitué, un groupe terphényle substitué ou non substitué, - Si(Q₃)(Q₄)(Q₅), ou -Ge(Q₃)(Q₄)(Q₅) ;
dans lequel au moins l'un des T₁(s) en nombre de a1 comprend au moins un -F ; et
dans lequel, dans la formule 1, le cas suivant est exclu : 1) A₇ est un hydrogène, et 2) à la fois le groupe représenté par *-C(A₁)(A₂)(A₃) et le groupe représenté par *-C(A₄)(A₅)(A₆) sont un groupe méthyle.

3. Composé organométallique de l'une des revendications 1 ou 2, dans lequel T₁ et A₁ à A₇ sont chacun indépendamment :
un hydrogène, un deutérium, -F, un groupe alkyle en C₁ à C₂₀, un groupe cycloalkyle en C₃ à C₁₀, un groupe hétérocycloalkyle en C₁ à C₁₀, un groupe phényle, un groupe biphényle ou un groupe terphényle ;
un groupe alkyle en C₁ à C₂₀ fluoré, un groupe cycloalkyle en C₃ à C₁₀ fluoré, un groupe hétérocycloalkyle en C₁ à C₁₀ fluoré, un groupe phényle fluoré, un groupe biphényle fluoré ou un groupe terphényle fluoré, chacun non substitué ou substitué avec un deutérium, un groupe alkyle en C₁ à C₂₀, un groupe cycloalkyle en C₃ à C₁₀, un groupe hétérocycloalkyle en C₁ à C₁₀, un groupe phényle, un groupe biphényle, un groupe terphényle ou toute combinaison de ceux-ci ;
un groupe alkyle en C₁ à C₂₀ deutéré, un groupe cycloalkyle en C₃ à C₁₀ deutéré, un groupe hétérocycloalkyle en C₁ à C₁₀ deutéré, un groupe phényle deutéré, un groupe biphényle deutéré ou un groupe terphényle deutéré, chacun non substitué ou substitué avec -F, un groupe alkyle en C₁ à C₂₀, un groupe cycloalkyle en C₃ à C₁₀, un groupe hétérocycloalkyle en C₁ à C₁₀, un groupe phényle, un groupe biphényle, un groupe terphényle ou toute combinaison de ceux-ci ; ou
-Si(Q₃)(Q₄)(Q₅), ou -Ge(Q₃)(Q₄)(Q₅) ;
dans lequel au moins l'un des T₁(s) en nombre de a1 comprend au moins un -F ; et
dans lequel, dans la formule 1, le cas suivant est exclu : 1) A₇ est un hydrogène, et 2) à la fois le groupe représenté par *-C(A₁)(A₂)(A₃) et le groupe représenté par *-C(A₄)(A₅)(A₆) sont un groupe méthyle.

4. Composé organométallique de l'une quelconque des revendications 1 à 3, dans lequel A₁ à A₇ sont chacun indépendamment :
un hydrogène, un deutérium, un groupe alkyle en C₁ à C₂₀, un groupe cycloalkyle en C₃ à C₁₀, un groupe hétérocycloalkyle en C₁ à C₁₀, un groupe phényle, un groupe biphényle ou un groupe terphényle ;
un groupe alkyle en C₁ à C₂₀ deutéré, un groupe cycloalkyle en C₃ à C₁₀ deutéré, un groupe hétérocycloalkyle en C₁ à C₁₀ deutéré, un groupe phényle deutéré, un groupe biphényle deutéré ou un groupe terphényle deutéré, chacun non substitué ou substitué avec un groupe alkyle en C₁ à C₂₀, un groupe cycloalkyle en C₃ à C₁₀, un groupe hétérocycloalkyle en C₁ à C₁₀, un groupe phényle, un groupe biphényle, un groupe terphényle ou toute combinaison de ceux-ci ; ou
-Si(Q₃)(Q₄)(Q₅), ou -Ge(Q₃)(Q₄)(Q₅) ;
dans lequel T₂ est :
un deutérium, un groupe alkyle en C₁ à C₂₀, un groupe cycloalkyle en C₃ à C₁₀, un groupe hétérocycloalkyle en C₁ à C₁₀, un groupe phényle, un groupe biphényle ou un groupe terphényle ;
un groupe alkyle en C₁ à C₂₀ deutéré, un groupe cycloalkyle en C₃ à C₁₀ deutéré, un groupe hétérocycloalkyle en C₁ à C₁₀ deutéré, un groupe phényle deutéré, un groupe biphényle deutéré ou un groupe terphényle deutéré, chacun non substitué ou substitué avec un groupe alkyle en C₁ à C₂₀, un groupe cycloalkyle en C₃ à C₁₀, un groupe hétérocycloalkyle en C₁ à C₁₀, un groupe phényle, un groupe biphényle, un groupe terphényle ou toute combinaison de ceux-ci ; ou
-Si(Q₃)(Q₄)(Q₅), ou -Ge(Q₃)(Q₄)(Q₅) ; et
dans lequel, dans la formule 1, le cas suivant est exclu : 1) A₇ est un hydrogène, et 2) à la fois le groupe représenté par *-C(A₁)(A₂)(A₃) et le groupe représenté par *-C(A₄)(A₅)(A₆) sont un groupe méthyle.

5. Composé organométallique de l'une quelconque des revendications 1 à 4, dans lequel un groupe représenté par 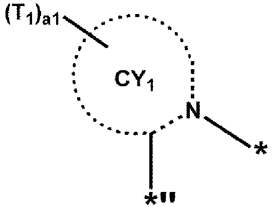 dans la formule 1 est représenté par la formule CY1(1) : 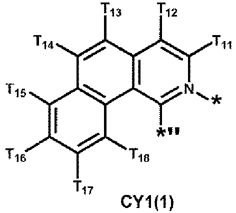 dans lequel, dans la formule CY1(1),
T₁₁ à T₁₈ sont les mêmes que ceux définis en relation avec T₁ dans la revendication 1, à condition qu'au moins l'un de T₁₁ à T₁₈ comprenne au moins un groupe fluoro (-F),
* indique un site de liaison à Ir dans la formule 1, et
*" indique un site de liaison au cycle CY₂ dans la formule 1.

6. Composé organométallique de l'une quelconque des revendications 1 à 5, dans lequel un groupe représenté par 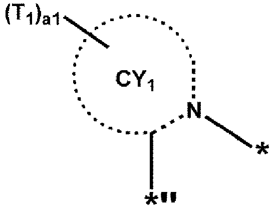 dans la formule 1 est l'un de groupes représentés par les formules CY1(1)-1 à CY1(1)-64 : 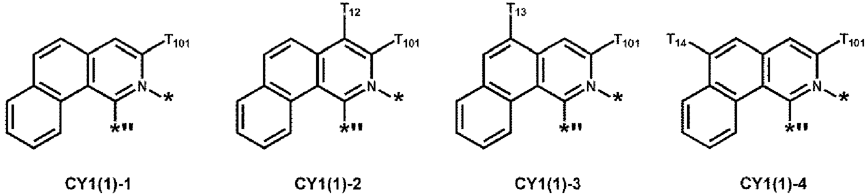 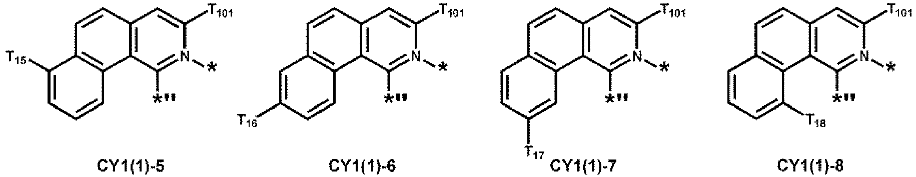 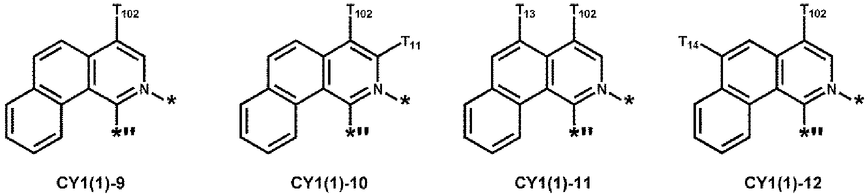 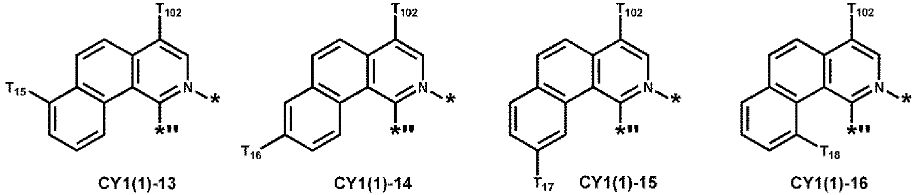 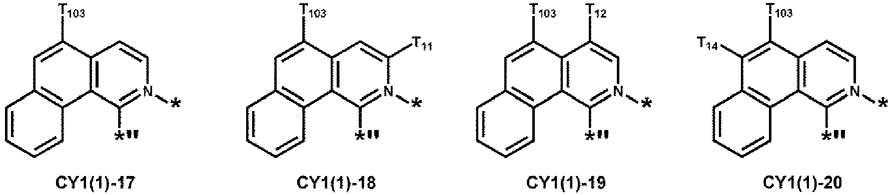 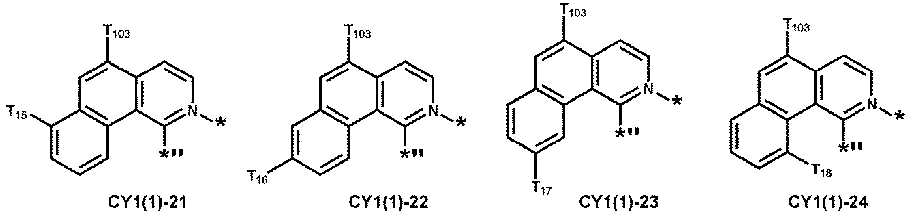 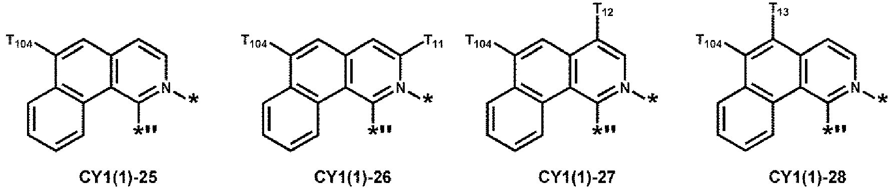 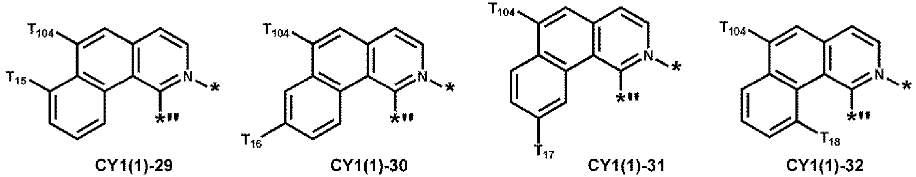 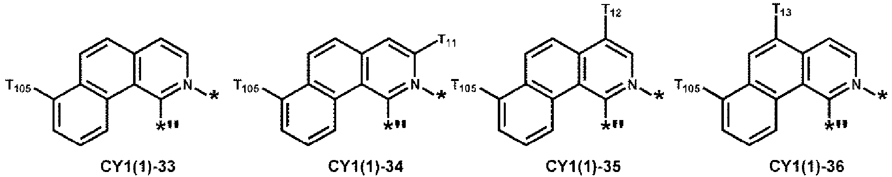 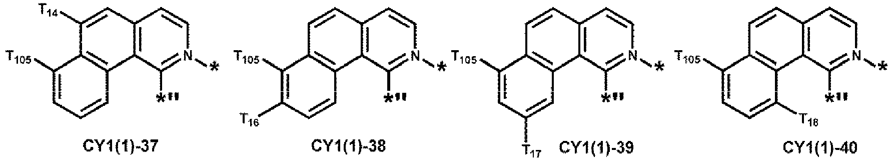 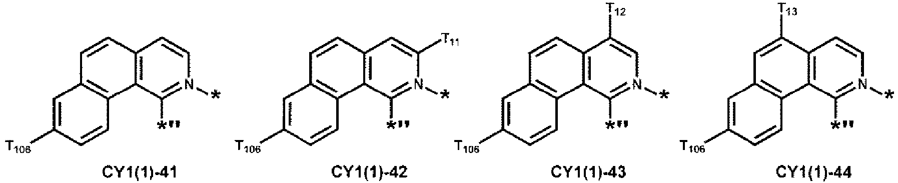 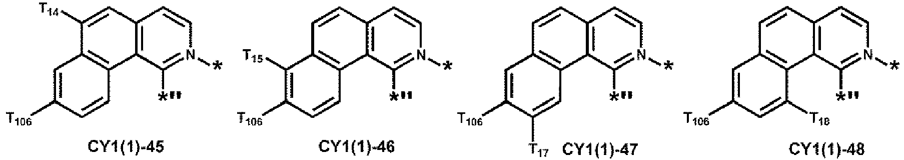 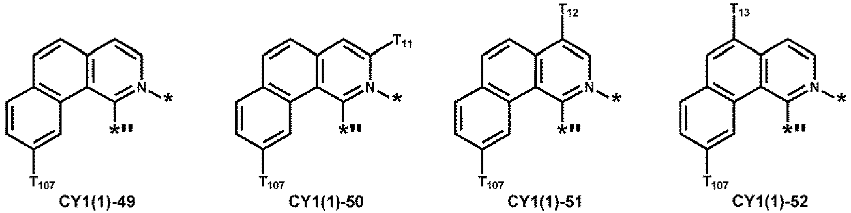 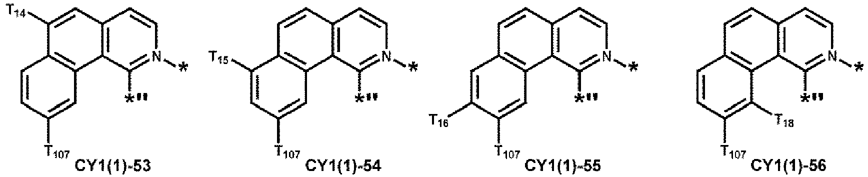 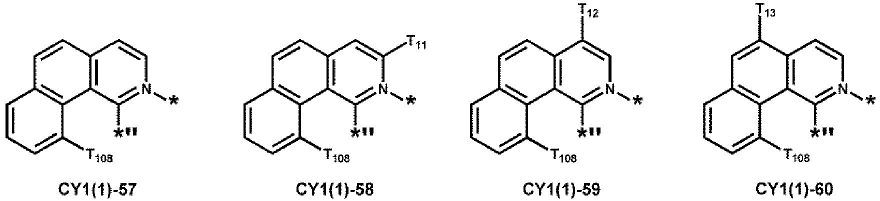 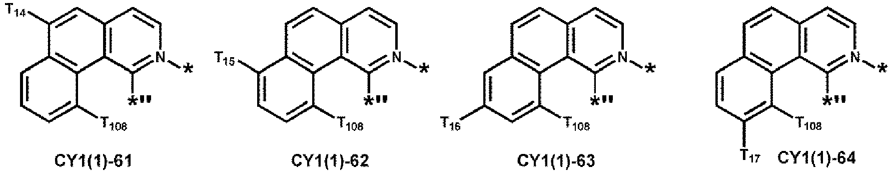 dans lequel, dans les formules CY1(1)-1 à CY1(1)-64,
T₁₁ à T₁₈ sont les mêmes que ceux définis en relation avec T₁ dans la revendication 1, à condition que chacun de T₁₁ à T₁₈ ne soit pas un hydrogène,
T₁₀₁ à T₁₀₈ comprennent chacun au moins un groupe fluoro (-F),
* indique un site de liaison à Ir dans la formule 1, et,
*" indique un site de liaison au cycle CY₂ dans la formule 1 ;
de préférence dans lequel, dans les formules CY1(1)-1 à CY1(1)-64,
T₁₁ à T₁₈ sont chacun indépendamment :
un deutérium, un groupe alkyle en C₁ à C₂₀, un groupe cycloalkyle en C₃ à C₁₀, un groupe hétérocycloalkyle en C₁ à C₁₀, un groupe phényle, un groupe biphényle ou un groupe terphényle ;
un groupe alkyle en C₁ à C₂₀ deutéré, un groupe cycloalkyle en C₃ à C₁₀ deutéré, un groupe hétérocycloalkyle en C₁ à C₁₀ deutéré, un groupe phényle deutéré, un groupe biphényle deutéré ou un groupe terphényle deutéré, chacun non substitué ou substitué avec un groupe alkyle en C₁ à C₂₀, un groupe cycloalkyle en C₃ à C₁₀, un groupe hétérocycloalkyle en C₁ à C₁₀, un groupe phényle, un groupe biphényle, un groupe terphényle ou toute combinaison de ceux-ci ; ou
-Si(Q₃)(Q₄)(Q₅), ou -Ge(Q₃)(Q₄)(Q₅), et
T₁₀₁ à T₁₀₈ sont chacun indépendamment :
-F ; ou
un groupe alkyle en C₁ à C₂₀ fluoré, un groupe cycloalkyle en C₃ à C₁₀ fluoré, un groupe hétérocycloalkyle en C₁ à C₁₀ fluoré, un groupe phényle fluoré, un groupe biphényle fluoré ou un groupe terphényle fluoré, chacun non substitué ou substitué avec un deutérium, un groupe alkyle en C₁ à C₂₀, un groupe cycloalkyle en C₃ à C₁₀, un groupe hétérocycloalkyle en C₁ à C₁₀, un groupe phényle, un groupe biphényle, un groupe terphényle ou toute combinaison de ceux-ci.

7. Composé organométallique de l'une quelconque des revendications 1 à 6, dans lequel un groupe représenté par 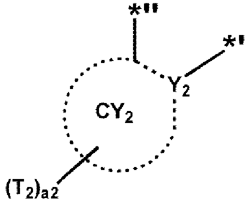 dans la formule 1 est l'un de groupes représentés par les formules CY2-1 à CY2-31 : 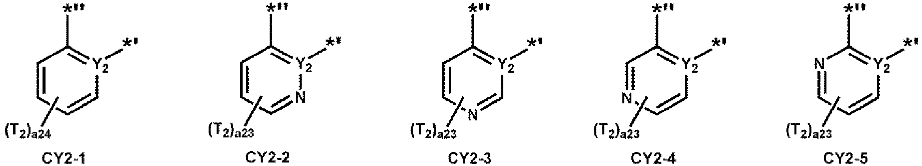 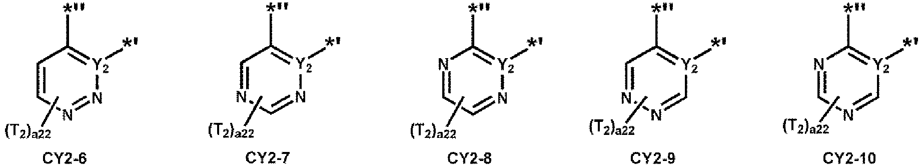 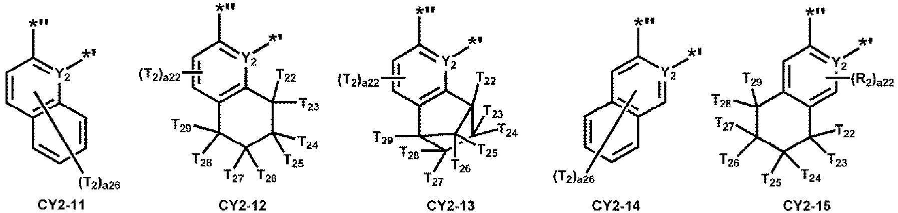 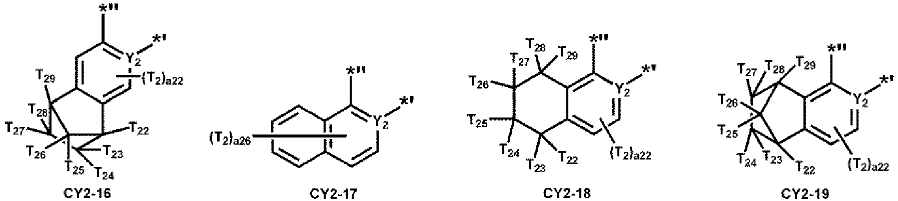 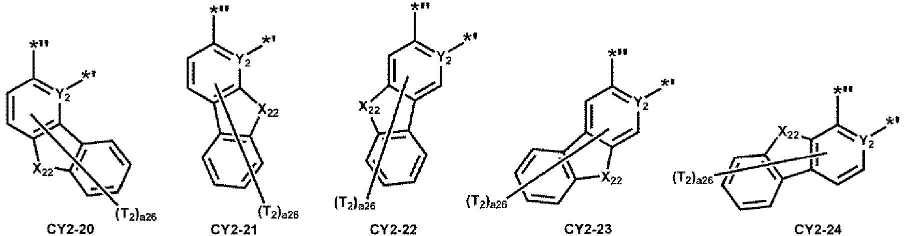 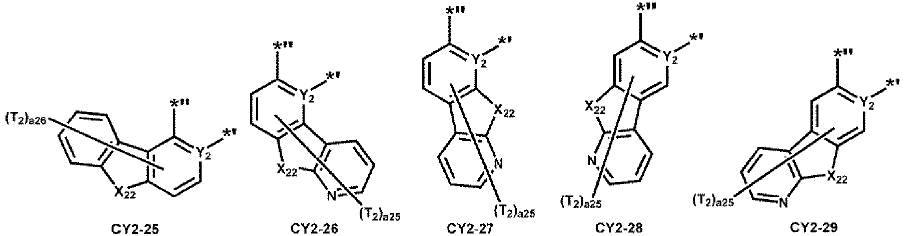 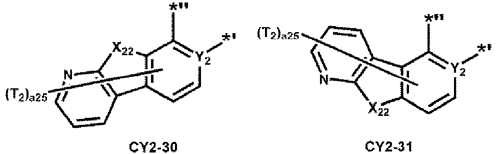 dans lequel, dans les formules CY2-1 à CY2-31,
Y₂ et T₂ sont les mêmes que ceux définis dans la revendication 1,
X₂₂ est C(T₂₈)(T₂₉), N(T₂₈), O, S ou Si(T₂₈)(T₂₉),
T₂₂ à T₂₉ sont les mêmes que ceux définis en relation avec T₂ dans la revendication 1,
a26 est un nombre entier de 1, 2, ou 3,
a25 est un nombre entier de 1, 2, ou 3,
a24 est un nombre entier de 1, 2 ou 3,
a23 est un nombre entier de 1, 2 ou 3,
a22 est un nombre entier de 1 ou 2,
*" indique un site de liaison au cycle CY₁ dans la formule 1, et
*' indique un site de liaison à Ir dans la formule 1.

8. Composé organométallique de l'une quelconque des revendications 1 à 7, dans lequel un groupe représenté par 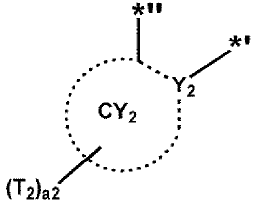 dans la formule 1 est l'un des groupes représentés par les formules CY2(2) à CY2(15), CY2(18) à CY2(24), CY2(26) à CY2(32), CY2(34) à CY3(40), CY3(42) à CY3(48), CY2(50) à CY2(52), CY2(55), CY2(56), CY2(64), CY2(65), CY2(67) et CY2(68) : 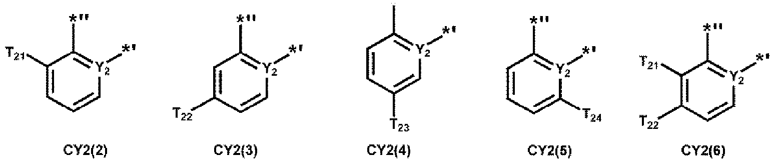 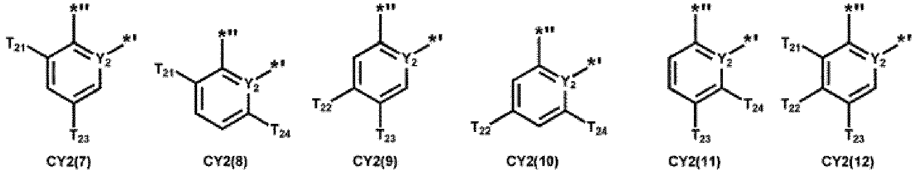 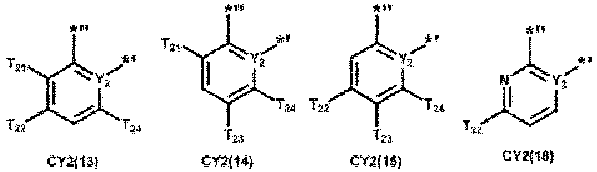 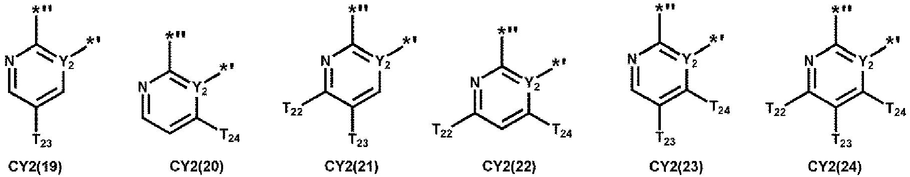 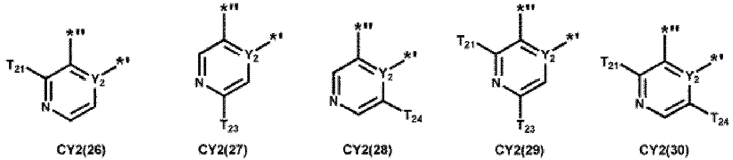 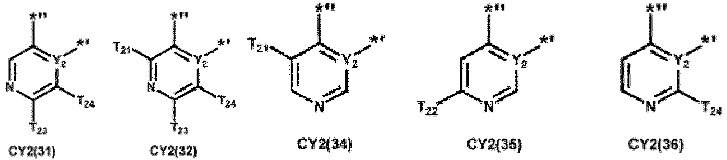 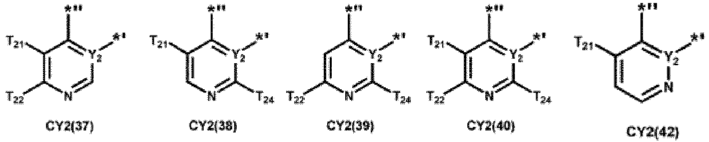 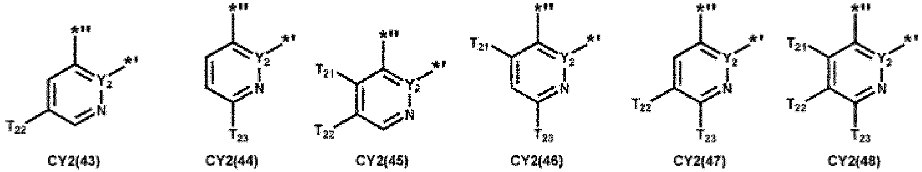 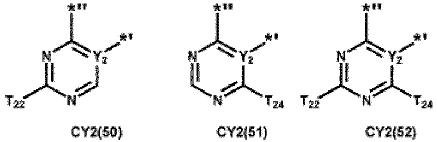 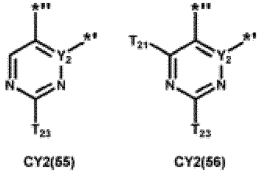 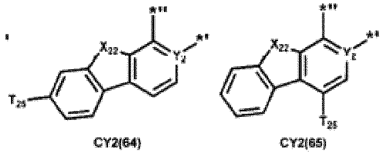 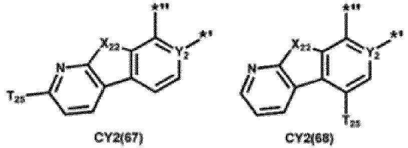
dans lequel, dans les formules CY2(2) à CY2(15), CY2(18) à CY2(24), CY2(26) à CY2(32), CY2(34) à CY3(40), CY3(42) à CY3(48), CY2(50) à CY2(52), CY2(55), CY2(56), CY2(64), CY2(65), CY2(67) et CY2(68),
Y₂ est le même que défini dans la revendication 1,
X₂₂ est C(T₂₈)(T₂₉), N(T₂₈), O, S, ou Si(T₂₈)(T₂₉),
T₂₁ à T₂₅, T₂₈, et T₂₉ sont les mêmes que ceux définis en relation avec T₂ dans la revendication 1,
*" indique un site de liaison au cycle CY₁ dans la formule 1, et
*' indique un site de liaison à Ir dans la formule 1.

9. Composé organométallique de l'une quelconque des revendications 1 à 8, dans lequel le composé organométallique satisfait au moins l'une de la condition 1 à la condition 3 :
Condition 1
A₁ à A₆ dans la formule 1 sont chacun indépendamment un groupe alkyle en Cₗ-C₆₀ substitué ou non substitué, un groupe cycloalkyle en C₃-C₁₀ substitué ou non substitué, un groupe hétérocycloalkyle en C₂-C₁₀ substitué ou non substitué, un groupe aryle en C₆-C₆₀ substitué ou non substitué, un groupe hétéroaryle en C₂-C₆₀ substitué ou non substitué, un groupe polycyclique condensé non aromatique monovalent substitué ou non substitué ou un groupe hétéropolycyclique condensé non aromatique monovalent substitué ou non substitué ;
Condition 2
au moins l'un de A₁ à A₆ dans la formule 1 sont chacun indépendamment un groupe alkyle en C₂-C₆₀ substitué ou non substitué, un groupe cycloalkyle en C₃-C₁₀ substitué ou non substitué, un groupe hétérocycloalkyle en C₂-C₁₀ substitué ou non substitué, un groupe aryle en C₆-C₆₀ substitué ou non substitué, un groupe hétéroaryle en C₂-C₆₀ substitué ou non substitué, un groupe polycyclique condensé non aromatique monovalent substitué ou non substitué ou un groupe hétéropolycyclique condensé non aromatique monovalent substitué ou non substitué ; et
Condition 3
A₇ dans la formule 1 est un deutérium, un groupe alkyle en C₁-C₆₀ substitué ou non substitué, un groupe cycloalkyle en C₃-C₁₀ substitué ou non substitué, un groupe hétérocycloalkyle en C₂-C₁₀ substitué ou non substitué, un groupe aryle en C₆-C₆₀ substitué ou non substitué, un groupe hétéroaryle en C₂-C₆₀ substitué ou non substitué, un groupe polycyclique condensé non aromatique monovalent substitué ou non substitué ou un groupe hétéropolycyclique condensé non aromatique monovalent substitué ou non substitué.

10. Composé organométallique de l'une quelconque des revendications 1 à 9, dans lequel le composé organométallique satisfait au moins l'une de la condition 4 et de la condition 5 :
Condition 4
dans la formule 1, deux de A₁ à A₃ ou plus du groupe représenté par *-C(A₁)(A₂)(A₃) sont liés à C dans le groupe représenté par *-C(A₁)(A₂)(A₃) pour former un groupe carbocyclique en C₅ à C₃₀ non substitué ou substitué avec au moins un R₁ₐ, ou un groupe hétérocyclique en C₁ à C₃₀ non substitué ou substitué avec au moins un R₁ₐ de sorte que le groupe représenté par *-C(A₁)(A₂)(A₃) est un groupe carbocyclique en C₅ à C₃₀ non substitué ou substitué avec au moins un R₁ₐ, ou un groupe hétérocyclique en C₁ à C₃₀ non substitué ou substitué avec au moins un R₁ₐ
Condition 5
dans la formule 1, deux de A₄ à A₆ ou plus du groupe représenté par *-C(A₄)(A₅)(A₆) sont liés à C dans le groupe représenté par *-C(A₄)(A₅)(A₆) pour former un groupe carbocyclique en C₅ à C₃₀ non substitué ou substitué avec au moins un R₁ₐ, ou un groupe hétérocyclique en C₁ à C₃₀ non substitué ou substitué avec au moins un R₁ₐ de sorte que le groupe représenté par *-C(A₄)(A₅)(A₆) est un groupe carbocyclique en C₅ à C₃₀ non substitué ou substitué avec au moins un R₁ₐ, ou un groupe hétérocyclique en C₁ à C₃₀ non substitué ou substitué avec au moins un R₁ₐ.

11. Dispositif électroluminescent organique, comprenant :
une première électrode ;
une seconde électrode ; et
une couche organique disposée entre la première électrode et la seconde électrode et comprenant une couche d'émission, la couche organique comprenant au moins un composé organométallique représenté par la formule 1 de l'une quelconque des revendications 1 à 10 ;
de préférence dans lequel
la première électrode est une anode,
la seconde électrode est une cathode,
la couche organique comprend en outre une région de transport de trous disposée entre la première électrode et la couche d'émission, et une région de transport d'électrons disposée entre la couche d'émission et la seconde électrode,
la région de transport de trous comprend une couche d'injection de trous, une couche de transport de trous, une couche de blocage d'électrons, une couche tampon ou une combinaison de celles-ci, et
la région de transport d'électrons comprend une couche de blocage de trous, une couche de transport d'électrons, une couche d'injection d'électrons ou toute combinaison de celles-ci.

12. Dispositif électroluminescent organique de la revendication 11, dans lequel le composé organométallique se situe dans la couche d'émission ;
de préférence dans lequel la couche d'émission comprend en outre un hôte, et une quantité de l'hôte dans la couche d'émission est supérieure à une quantité du composé organométallique dans la couche d'émission.

13. Appareil électronique comprenant le dispositif électroluminescent organique de l'une des revendications 11 ou 12.
